# EUROPEAN PATENT SPECIFICATION

(11) **EP 0 739 343 B1**
(45) Date of publication and mention of the grant of the patent: **29.03.2000**
(21) Application number: 95908496.3
(22) Date of filing: 10.01.1995
(51) Int. Cl.: C07D 405/12, C07D 405/14, A61K 31/335

(54) **WATER SOLUBLE ONIUM SALT OF TAXO-DITERPENOID**
WASSERLÖSLICHES ONIUM SALZ EINES TAXO-DITERPENOIDS
SEL ONIUM SOLUBLE DANS L'EAU DE TAXO-DITERPENOIDE

(30) Priority: 11.01.1994 US 180034
(43) Date of publication of application: 30.10.1996
(73) Proprietor: THE SCRIPPS RESEARCH INSTITUTE, La Jolla, CA 92037 (US)
(72) Inventor: NICOLAOU, K., C., La Jolla, CA 92037 (US); WRASIDLO, Wolfgang, La Jolla, CA 92037 (US); GUY, Rodney, K., San Diego, CA 92117 (US); PITSINOS, Emmanuel, GR-152 32 Halandri (GR)
(74) Representative: Hedley, Nicholas James Matthew
(86) International application number: US9500478
(87) International publication number: WO9518804

(56) References cited:
- EP-A- 0 473 326
- Angewandte Chemie (1979), 91, pp. 798-812

## Description

### Technical Field:

The invention relates to taxol prodrugs. More particularly, the invention relates to taxol and taxol memetics derivatized with onium salts of aza-arenes to form water soluble prodrugs which are activated upon contact with serum protein.

### Background:

Taxol, an antineoplastic agent originally isolated from *Taxus brevifolia,* is approved for usage in the treatment of ovarian cancer and is expected to see usage in breast, lung, and skin cancers as well. However, since Taxol possesses an extremely low water solubility, i.e., less than 1.5 x 10⁻⁶ M, it has been necessary to formulate Taxol in a mixture of Cremaphor^{TM}, a polyoxyethylated castor oil, and ethanol in order to achieve a therapeutic concentration. This formulation can induce a variety of significant side effects including hypersensitivity reactions.

While premedication and slow administration of the drug can circumvent these problems in the clinic, the entire protocol is quite cumbersome and requires extensive close monitoring of patients. Although taxol's dramatic efficacy has driven clinical usage forward despite these problems, a water soluble form of taxol could completely obviate the need for this troublesome protocol.

One approach to bypassing these formulation difficulties, previously attempted by several groups including our own, is the introduction of solubilizing functionality that normal metabolic pathways could remove *in vivo.* Compounds of this type, termed prodrugs, consist, in the case of taxol, primarily of ester derivatives at the 2' and 7 positions (see for example EP-A-0 423 326). Currently none of these protaxols have given success in the clinic. In each case, the prodrug is rapidly cleared from circulation by the kidneys.

Taxol is only one of a class of taxo-diterpenoids having bioactivity. Another preferred taxo-diterpenoid having clinically significant activity is Taxotere^{TM}. C-2 substituted taxol analogs are also known to have bioactivity. Unfortunately, all known bioactive taxo-diterpenoids have a low aqueous solubility.

What is needed is a prodrug which is activated by contact with serum and which is retained for a clinically significant period after administration.

### Summary:

The invention is an onium salt of a taxo-diterpenoid-Cⁿ,2-O-aza-arene. The onium salt of aza-arene includes a delocalized charge which imparts aqueous solubility. The invention herein teaches that onium salts of taxo-diterpenoid-Cⁿ,2-O-aza-arenes are soluble and stable in aqueous media; that, with the addition of serum protein, 2-O-aza-arene is displaced and a soluble protein:taxo-diterpenoid intermediate is formed; and that the protein:taxo-diterpenoid intermediate dissociates over time to provide a bioactive taxo-diterpenoid. Preferred taxo-diterpenoids include taxol, C-2 substituted analogs of taxol, and Taxotere™. Taxo-diterpenoid-Cⁿ,2-O-aza-arene may be produced in a one step synthesis by reacting onium salts of 2-halogenated aza-arenes with reactive hydroxyls on the taxo-diterpenoid. Reactive hydroxyls on taxol and Taxotere™ are located at C^{2'} and C⁷. A preferred onium salt of 2-halogenated aza-arene is 2-fluoro-1-methylpyridinium tosylate. Other employable onium salts of 2-halogenated aza-arenes are disclosed by T.Mukaiyama, *Angewandte Chemie* **1979**, *18*(18), 707-808, incorporated herein by reference.

More particularly, the invention is directed to water soluble taxo-diterpenoids represented by the following formula: 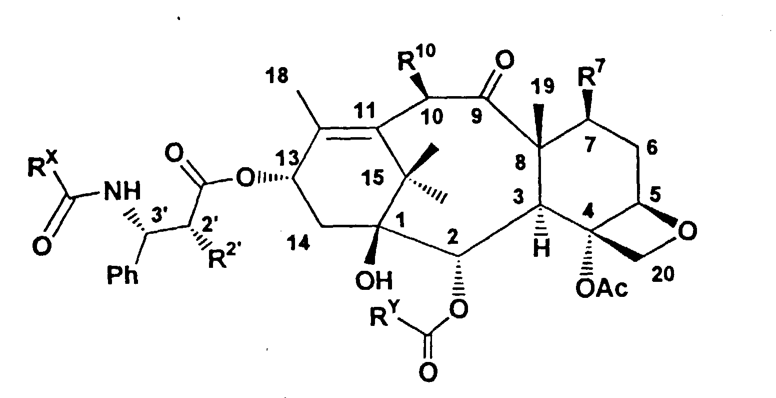 wherein R^{x} is Ph or *t*BuO; R¹⁰ is OAc or OH; R^{y} is a C-2 substituent defined below; and R^{2'} and R⁷ are each selected from the group consisting of OH and an onium salt of a 2-O-aza-arene, with the proviso that at least one of R^{2'} and R⁷ is the onium salt of the 2-O-aza-arene. The onium salt of the 2-O-aza-arene can be represented by either of the following formulas for onium salt I or onium salt II: 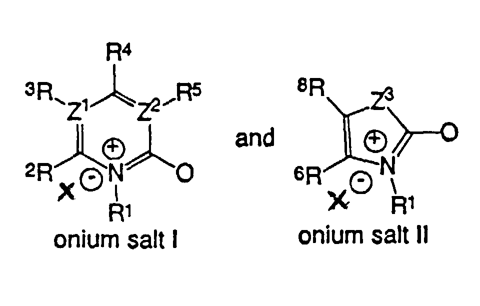 wherein Z¹, Z² Z³, R^{y}, x^{⊖} and R¹ to R⁸ are as defined in accompanying claim 1.

### Brief Description of the Drawings:

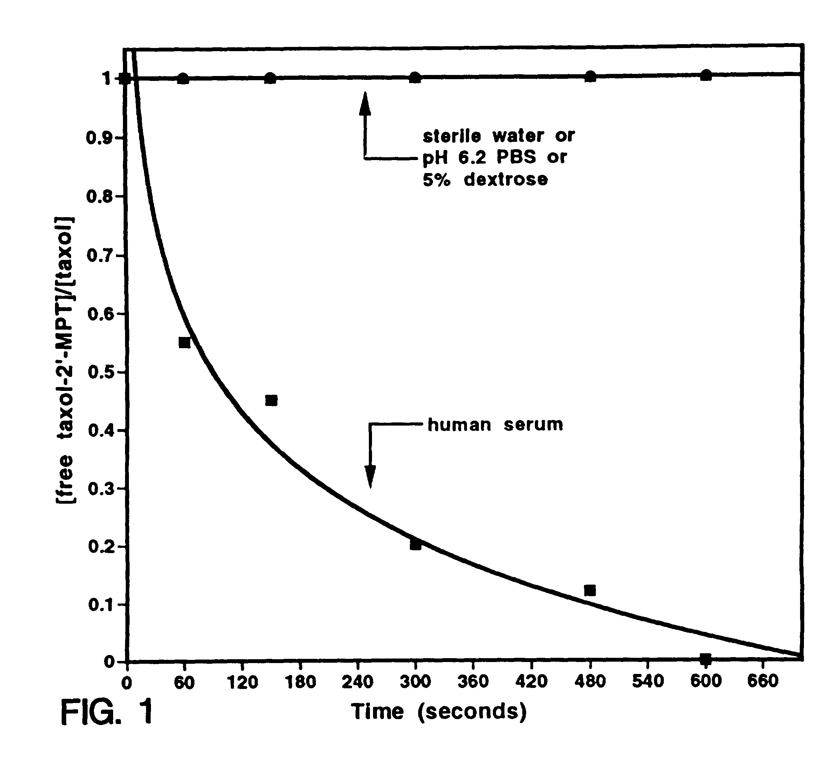

Figure **1** illustrates the kinetics of taxol release from taxol-2'-MPT (**2**) in various aqueous solutions at 25°C (horizontal line). Although stable in water and aqueous buffer solutions, methylene chloride extraction of plasma treated with compound **2** showed complete conversion of 2 into taxol (**1**) within 10 minutes (curve, 20% of total recovered).

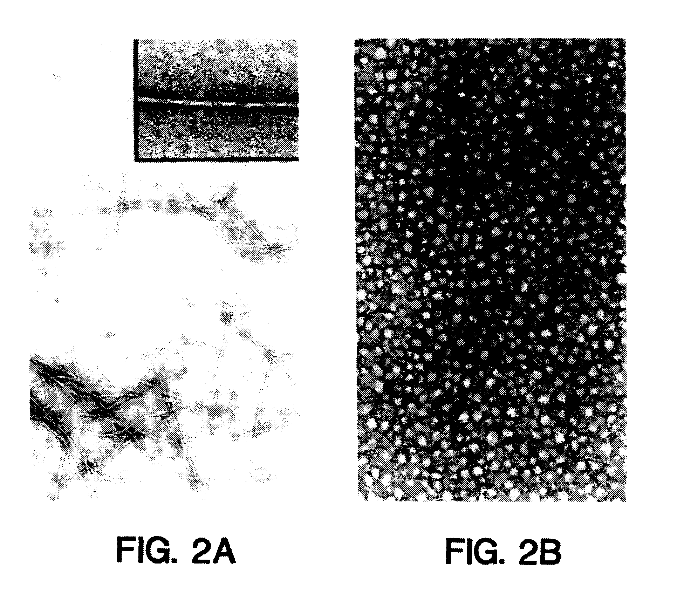

Figure 2 **A** illustrates a transmission electron micrograph (TEM) of self-assembled helical fibrous nanostructures of taxol-2'-MPT, i.e., compound **2** in buffered solutions (2,1 mM in 100 mM PBS) above the critical aggregation concentration (CAC) of this compound using a negative phosphotungstate stain and a magnification of x25000. The inset shows a portion of one of the fibrils further magnified to illustrate the helical nature of the structure.

Figure 2 **B** illustrates a transmission electron micrograph (TEM) of self-assembled spherical nanostructures of taxol-2'-MPT (compound **2**) in unbuffered solutions (2,1 mM in H₂O) above the critical aggregation concentration (CAC) of this compound using a negative uranyl acetate stain and a magnification of x45000.

Figure 3 illustrates a tubulin polymerization-depolymerization measurements with negative control (triangles), positive taxol control (diamonds), and taxol-2'-MPT, i.e., compound **2** (dots). Calcium chloride promoted depolymerization is suppressed by taxol but not by taxol-2'-MPT.

Figure 4 illustrates the relative cytotoxicities of taxol-2'-MPT (compound **2**) and taxol against a variety of cell lines.

Figure 5 illustrates the efficacy of taxol-2'-MPT in lung tumor xenograft nude mouse model: 5% dextrose (triangles), taxol (diamonds), and taxol-2'-MPT (dots).

### Detailed Description:

The synthesis, physical properties, and pharmaceutical profiles of water soluble onium salts of taxo-diterpenoid-Cⁿ,2-O-aza-arenes are described.

### Synthesis of Taxol-2'-MPT:

Taxol-2'-MPT (methylpyridinium tosylate), compound 2, was synthesized according to the method of T.Mukaiyama, Angewandte Chemie **1979**, 18(18), 707-808, incorporated herein by reference. Taxol (10 mg, 0.012 mM), from NaPro Biochemicals, Boulder CO, USA, was dried by azeotropic distillation with toluene (2x1.0 mL) and then dissolved in methylene chloride (0.4 mL) and treated sequentially under an atmosphere of dry argon, with freshly distilled triethylamine (5 microL, 0.04 mM, 3 equivalents) and 2-fluoro-1-methylpyridinium tosylate (5 mg, 0.018 mM, 1.5 equivalents) Aldrich Chemicals, and allowed to stir at ambient temperature for 30 minutes. The clear colorless solution rapidly turned to a clear pale yellow. The course of the reaction was monitored through thin layer chromatography (TLC) (E. Merck RP-18 silica, 65 tetrahydrofuran : 35 water, UV/phosphomolybdic acid) and after thirty minutes of stirring at ambient temperature, judged complete as no taxol remained and only one compound was apparent by TLC (Rf 0.8). Purification via reverse phase high pressure liquid chromatography (HPLC) (C₁₈ column, 1mM NH₄OAc pH 6.5 buffer / methanol gradient, 1.5 mL / min. UV) to give, after removal of solvent in vaccuo, pure taxol-2'-MPT (**2**) (12 mg, 93% yield) as a white amorphous solid. All spectroscopic data (¹H NMR and HRMS) were in accord with the structure assigned to **2**. ¹H NMR (CDCl₃, 125 MHz) δ: 1.055 (s, 3 H, C17-**H**), 1.083 (s, 3 H, C19-**H**), 1.724 (s, 3 H, C19-H), 1.858 (m, 1 H, C6-6**H**), 1.913 (s, 3 H, C**H**₃-Ph), 2.193 (s, 3 H, C10-OC(O)C**H**₃), 2.514 (m, 1 H, C6-a**H**), 3.663 (d, 1 H, *J* = 7.0 Hz, C3-H), 4.110 (d, 1 H, *J* = 8.5, C20-6**H**, A of AB), 4.133 (s, 3 H, N-CH₃), 4.230 (d, 1 H, *J* = 8.5, C20-a**H**, B of AB), 4.315 (dd, 1 H, *J* = 8.7, 10.7, C7-H), 4.901 (dd, 1 H, *J* = 1.0, 7.7, C5-H), 5.501 (d, 1 H, C2-**H**, J = 7.0), 5.702 (bt, 1 H, C2'-**H**, *J* = 8.0), 5.951 (dd, 1 H, C13-**H**, *J* = 1.0, 8.0), 6.120 (bd, 1 H, C3'-**H**, *J* = 10.0), 6.181 (s, 1 H, C10-**H**) 7.702 (t, 1 H, N-**H**, *J* = 7.5), 7.33 - 7.45 (m, 8 H, Ar-**H**), 7.56 - 7.62 (m, 4 H, Ar-**H**), 7.56 - 7.62 (m, 4 H, Ar-**H**), 7.68 - 7.75 (m, 4 H, Ar-**H**), 8.00 - 8.05 (m, 1 H Ar-**H**), 8.23 - 8.28 (m, 1 H, Pyr-**H**), 8.41 (m, 1 H, Pyr-**H**). IR (neat, KCl plate) cm⁻¹: 3640 - 3120 (bm), 3030-2870 (bm), 2320 (m), 1720 (s), 1630 (m), 1560 (m), 1500 (m), 1360 (s), 1160 (m), 1070 (m), 700 (m). UV/Vis (CHCl₃) nm: 254, 280. FAB HRMS: calc for C₅₃H₅₇O₁₄N₂: 945.3810; found: 945.3810

The molecular structures of taxol, compound 1, and of taxol-2'-MPT, compound 2, are illustated in Scheme 1A. The synthesis of taxol-2'-MPT is illustrated in Scheme 1B.

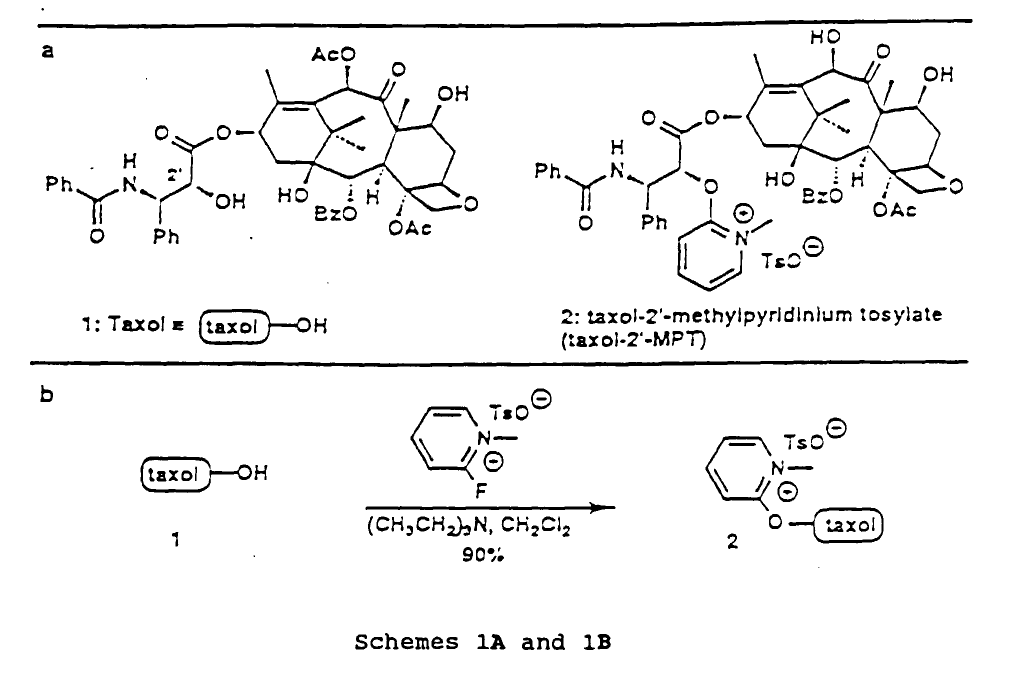

### Synthesis of Taxol-7-MPT:

The synthesis of taxol-7-MPT differed only slightly from the synthesis of Taxol-2'-MPT. Taxol (10 mg, 0.012 mM), from NaPro Biochemicals, Boulder CO, USA, was dissolved in methylene chloride (2.0 mL) and treated sequentially with triethylamine (67 microL, 0.48 mM, 40 equivalents) and 2-fluoro-1-methylpyridinium tosylate (34 mg, 0.12 mM, 10 equivalents) Aldrich Chemicals, and allowed to stir at ambient temperature for 5 minutes. Purification via reverse phase high pressure liquid chromatography (HPLC) gave pure taxol-2'-MPT (**2**) (12 mg, 93% yield) as a white amorphous solid. The Rf of taxol-7-MPT is about 0.3 minutes less than the Rf of taxol-2'-MPT. The yield was 11 mg or 85%. Spectroscopic data (¹H NMR and HRMS) were as expected.

### Synthesis of Taxol-bis-2',7-MPT:

The synthesis of taxol-bis-2',7-MPT differed from the synthesis of Taxol-7-MPT only with respect to reaction time. Taxol (10 mg, 0.012 mM), from NaPro Biochemicals, Boulder CO, USA, was dissolved in methylene chloride (2.0 mL) and treated sequentially with triethylamine (67 microL, 0.48 mM, 40 equivalents) and 2-fluoro-1- methylpyridinium tosylate (34 mg, 0.12 mM, 10 equivalents) Aldrich Chemicals, and allowed to stir at ambient temperature for 18 hours. Purification via reverse phase high pressure liquid chromatography (HPLC) gave pure taxol-2'-MPT (**2**) (12 mg, 93% yield) as a white amorphous solid. The Rf of taxol-bis-2',7-MPT is about 0.3 minutes less than the Rf of taxol-2'-MPT. The yield was 13 mg or 85%. Spectroscopic data (¹H NMR and HRMS) were as expected.

Alternative synthetic schemes based upon the method of T. Mukaiyama (Anagwandte Chemie **1979**, 18(18), 707-808) using a variety of onium salts of 2-halogenated aza-arenes for derivatizing either the 2' or the 7 positions of taxol are illustrated in the following scheme:

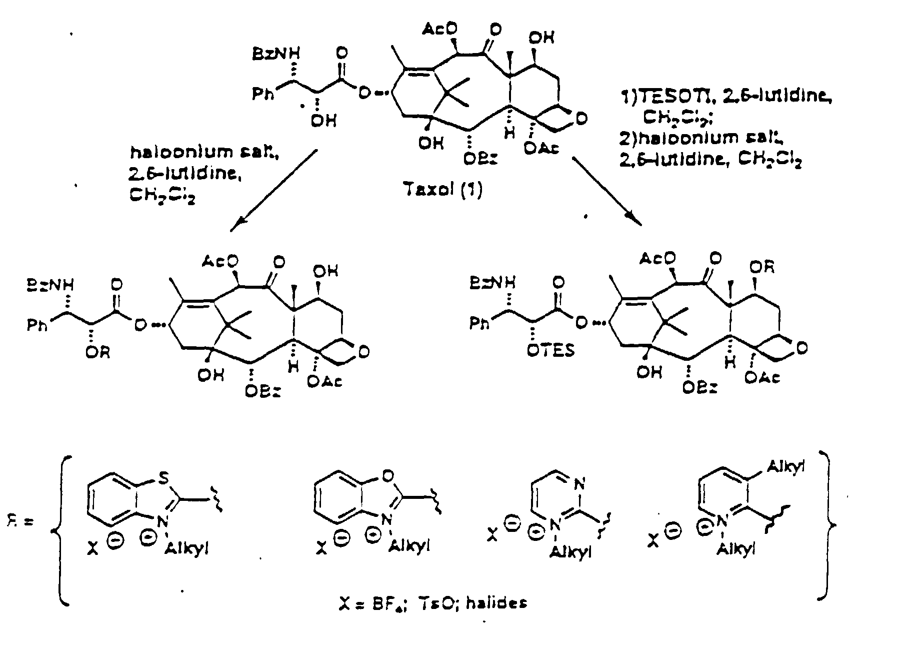

### Stability measurements and Kinetics of Taxol release:

Due to a difference in retention time using our standard HPLC conditions (see Fig. 1) and differing ultraviolet absorption maxima (1₂₈₀/1₂₅₄ = 1.6 for 2 and 0.3 for **1**), the stability of **2** was easily assayed by HPLC (Fig. 1). In all of the ensuing studies, the only degradation products detected were taxol and the pyridinone that results from hydrolysis of pyridinium salts (Fig. lc.). Taxol-2'-MPT appears completely stable in the solid state in a temperature range of ⁻80 °C to 25 °C regardless of the presence of an inert atmosphere. In water, 5% dextrose, and 1.5% saline **2** is stable for several days but begins to exhibit slow degradation after 4 days. In phosphate buffered saline (PBS) or ammonium acetate - phosphate buffer systems of pH 6.0 to 7.3, **2** is stable at 25 °C for over 21 days. Taxol-2'-MPT (**2**) is, however, unstable in 5% HCl (pH 1.1) and brine. Most significantly, **2** breaks down rapidly when incubated at 37°C with human plasma. This result suggests the presence of factors within plasma that initiate the degradation of **2** to taxol. Since taxol has been shown to bind to albumin to the extent of ca. 85% in sera, it is suspected that basic lysine residues on this protein may initiate breakdown.

The kinetics of taxol release from taxol-2'-MPT (2) in various aqueous solutions at 25°C is shown in Figure 1. In sterile water, pH 6.2 phosphate buffered saline, or 5% dextrose, no taxol release is observed over a period of 11 minutes, as shown by the horizontal line. Although stable in water and aqueous buffer solutions, methylene chloride extraction of plasma treated with compound 2 showed complete conversion of **2** into taxol (1) within 10 minutes, as shown by the curved line. Under these conditions 20% of total is recovered. More particularly, Taxol-2'-MPT (**2**) was dissolved in the aqueous system with the aid of sonication for five minutes. Aliquots were then removed at the times shown and partitioned into methylene chloride to quench the reaction. Samples were then analyzed using a Waters Maxima HPLC instrument equipped with an autoinjector (3.9x300 mm C₁₈ column equipped with a precolumn. The flow rate was 1.5 mL/minute. The eluent gradient A-B extended over 30 minutes. "A" was 80% 80mM ammonium acetate, pH 6.0. "B" was 100% methanol. An ultraviolet diode array detector was employed. The ratio of compound **2** (Rf 16.2 min.) to taxol (compound **1**, Rf 16.8 min.) remaining was determined from the relative areas of the peaks after normalization with previously determined calibration curves.

### Solubility Measurements:

The solubility and partition coefficient data for **2** and taxol were determined using an HPLC method.

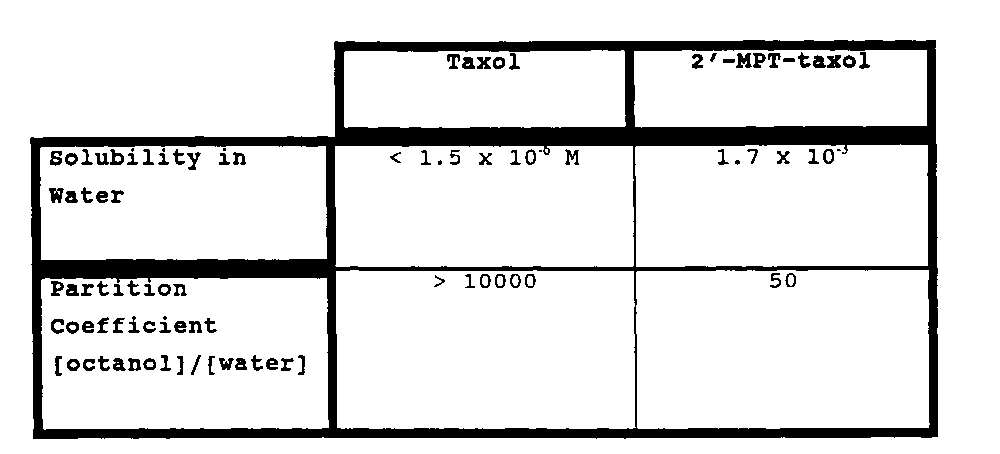

Solubility was found by forming a solution in water with the aid of sonication for five minutes, centrifugation of the samples, and injection of the supernatant. The values reported were normalized using calibration curves constructed for both compounds by preparing known concentrations in the range 1 x 10⁻⁶ to 1 x 10⁻³ M in methylene chloride and subjecting to HPLC analysis under the same conditions. One should note that the solubility of taxol is at the detection limit of the instrument and thus represents an upper limit. The solubility of **2** was found at a concentration at which the solution was clear (see below) and thus represents a lower limit. Compound **2** exhibited similar solubilities for various buffer systems in the pH range 6.2 to 7.4. Partition coefficients were determined by dissolving the compound in the organic phase, shaking the resulting solution with water for ten minutes, and analyzing each phase by HPLC as above. No degradation of **2** was noted during these studies. These data clearly show that **2** is significantly more soluble in water than the parent taxol. The solubility demonstrated in a range of aqueous systems is higher than the clinically relevant dosages (3 to 30 mM).

### Self-Assembling Structures:

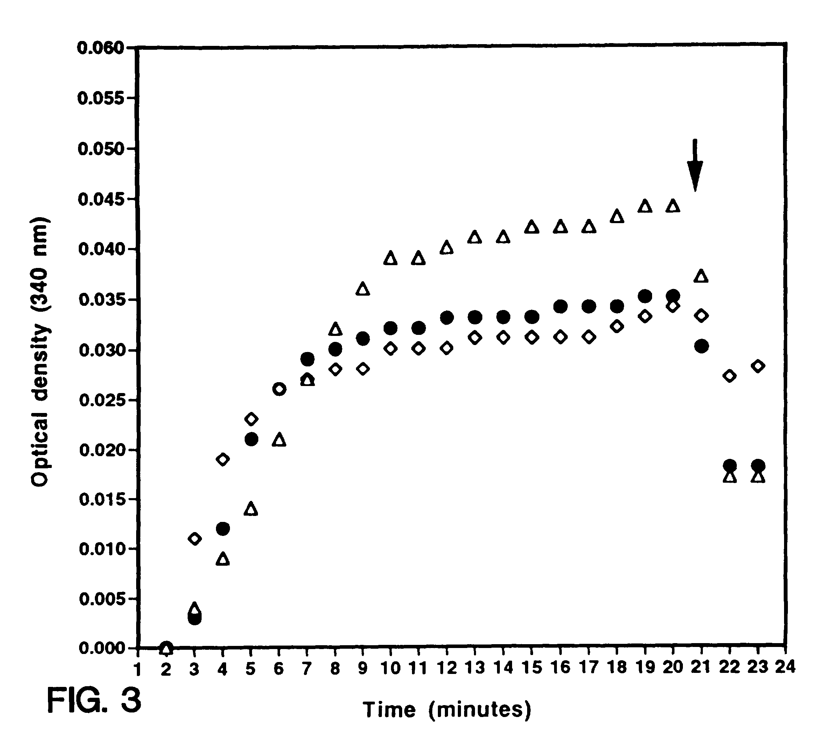

While the water solutions of **2** were optically clear at all concentrations examined, buffered solutions of concentrations greater than 1 x 10⁻³ M exhibited a haze to the naked eye and diffuse scattering of monochromatic light. Ultraviolet spectroscopic absorption measurements at 340 nm (Fig. 3) showed an exponential increase in optical density (OD) above a critical concentration of 4 x 10⁻⁴ M, a result characteristic of macromolecular structure in solution. Transmission electron microscopy (TEM) confirmed the presence of supramolecular structures in these solutions. Uniform aggregates of fibrillar structure (Fig. 2a) with helical conformations were observed. These structures exhibited varying (up to 800 Å) lengths but consistent diameter of ca. 80 Å with a helical twist of about 7. Additionally, freshly sonicated solutions of **2** showed the presence of spherical structures (Fig. 2b) with diameters of about 50 Å. It is likely that the long term stability of these solutions is due, at least in part, to stabilization provided by this structured environment.

### Microtubule polvmerization-depolymerization measurements:

Microtubule polymerization-depolymerization measurements (Fig. 3) with taxol-2'-MPT (**2**) were very similar to GTP-saline controls and drastically different from taxol. Compound **2** does not appear to bind to tubulin in the manner of taxol. In the buffered aqueous environment of this assay, **2** is not converted to taxol and thus does not affect the tubulin-microtubule equilibria. Taxol, recovered from human plasma treated with **2**, exhibited the expected microtubule stabilization, indicating that **2** does act as a prodrug for taxol.

Tubulin polymerization-depolymerization measurements are illustrate in Figure 3. Negative controls are shown with triangles; positive taxol controls are shown with diamonds; and taxol-2'-MPT, i.e., compound **2** is shown with dots. The measurements indicate that calcium chloride promoted depolymerization is suppressed by taxol but not by taxol-2'-MPT.

More particularly, measurement were performed in 96 well plates at 37°C following the protocol of R. Merlock and W. Wrasidlo *(Analytical Biochemistry* **1993**, in press). Calcium chloride addition is indicated by the arrow. In each case, 1.0 mM GTP was used to promote the initial polymerization of tubulin. Negative control employed tubulin (1.0 mg/mL) alone, CaCl₂ (0,25 mM) added after 20 minutes. Positive taxol control employed tubulin (1.0 mg/mL) with taxol (10⁻⁶M) and CaCl₂ (0.25mM) added after 20 minutes. The experimental taxol-2'-MPT employed tubulin (1.0 mg/mL) with taxol-2'-MPT (10⁻⁶) and CaCl₂ (0.25 mM) added after 20 minutes. Turbidity was measured as optical density at 340 nm using a microplate reader (Molecular Devices Thermomax).

### Toxicity Measurements:

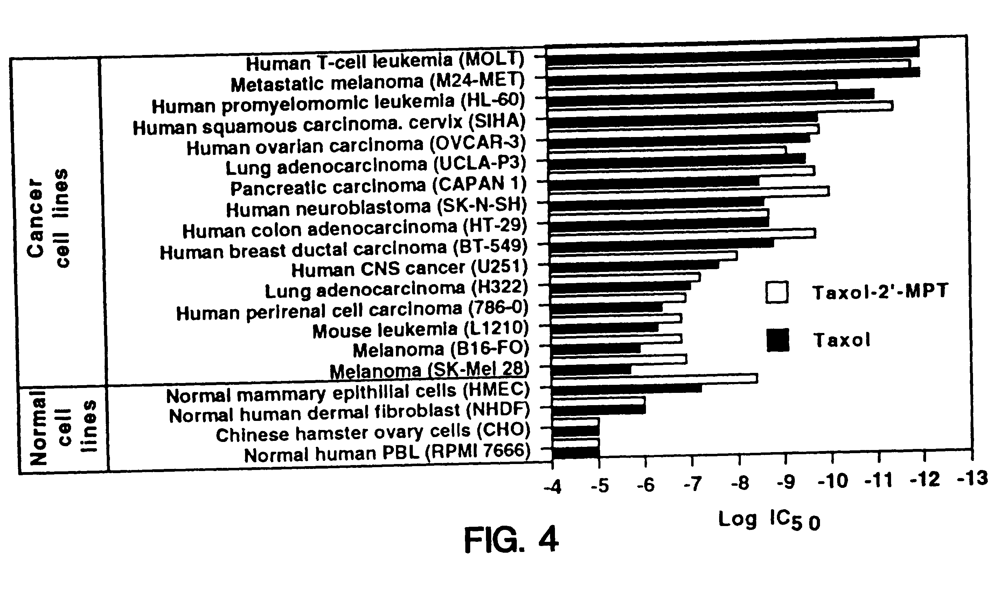

Compound **2** was tested for its cytotoxicity against a cell line panel including leukemia, ovarian, lung, and breast carcinoma cells (Fig. 4). The differential cytotoxicity profiles for **2** and taxol were similar, although some differences were noted. Both compounds exhibited IC₅₀ values ranging from 10⁻⁵ to 10⁻¹² M with means close to one nanomolar. Normal cells had cytotoxicity levels three to four orders of magnitude below mean values. Extremely high cytotoxicity levels were recorded for human leukemia, metastatic melanoma and cervical carcinoma. As expected for a prodrug of taxol in the cellular environment, **2** shows the same remarkable tumor cell selectivity and cell line specificity as taxol.

The relative cytotoxicities of taxol-2'-MPT (compound **2**) and taxol against a variety of cell lines are illustrated in Figure 4. More particularly, cells were plated on 96 well plates with the following controls: no cells and toxic control (1 x 10⁻³ M SDS). The drug was added to the first set of wells and diluted via standard dilution method from the stock. Plates were incubated at 37°C, 5% CO₂ in sterile air in an humidified incubator for 72 hours. An aliquot of 50 µL of a solution of 2,3-bis(methoxy-4-nitro-5-sulfophenyl)-5-[(phenylamino)carbonyl]-1H-tetrazolium hydroxide (XTT), 1 mg mL⁻¹, in phosphate buffered saline (PBS, 100mM) was added to the wells. In the presence of viable cells, this colorless clear media is enzymatically altered to give a pink coloration. The plates were read at 450 nm using a plate reader. Percentage cytotoxicity was calculated using the formula: %C = 1 - (OD toxin)(OD growth control)⁻¹(100).

### Efficacy of taxol-2'-MPT in lung tumor xenoaraft:

The encouraging *in vitro* data obtained with taxol-2'-MPT (**2**) prompted us to study its *in vivo* action using nude mice inflicted with human lung carcinoma xenografts (Fig. 1). The samples of **2** used for this study were formulated in sterile PBS without Cremaphor™, indicating the suitability of this compound for simple bolus administration.
Preliminary data shows that the control of tumor growth exhibited by **2** is at least comparable to that of taxol and significantly (0.001 p-value, multiple linear regression model) different from controls. These results provide a reasonable indication that **2** is converted rapidly to taxol in *vivo* and should thus exhibit pharmacology similar to taxol. Indeed, in metabolic study using tritiated **2**, only 5% of the compound was excreted through the kidneys, a result that is completely in accord with the behavior of taxol.

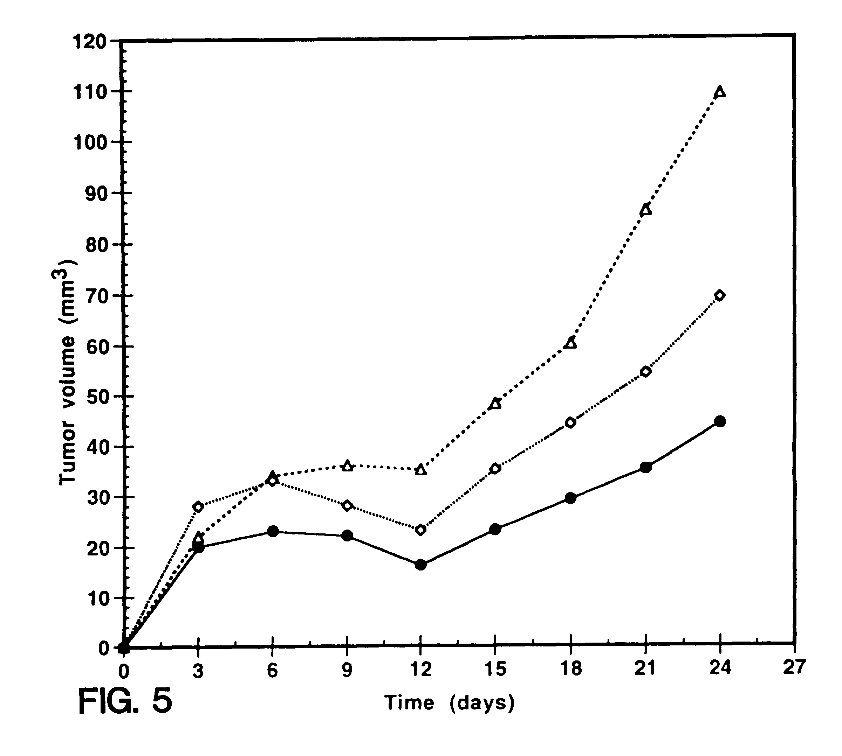

The efficacy of taxol-2'-MPT in lung tumor xenograft nude mouse model is illustrated in Figure 5. The tumor model was generated from an ATCC A549 non-small cell lung adenocarcinoma cell line that was maintained under the standard cell proliferation conditions (37°C, 5% carbon dioxide in sterile air). Hemocytometer counted cells suspended in Hanks medium (Gibco, Grand Island NY) were implanted S.C. (10⁶ cells in 0.4 mL per tumor volume determined using the equation (length)(width)²/2. The test compounds (1.0 microM) were injected I.P. on day 1,3, and 7 using the following media: control. 5% dextrose in water (D5W), triangles; taxol, suspended in Cremaphor/D5W (5/95, 18.0 mg/kg of animal weight), diamonds; and taxol-2'-MPT, dissolved in D5W (23.9 mg/kg of animal wight), dots. The procedures used for the maintenance of tumors and the experimental details were according to protocols set forth by the Developmental Therapeutics Program, National Cancer Institute, viz., *National Cancer Institute Cancer Chemotherapy Reports,* **3** (1972).

### Mechanisms of taxol release:

The mechanism of acid catalyzed taxol (**1**) release from taxol-2'-MPT is illustrated in Scheme 2.

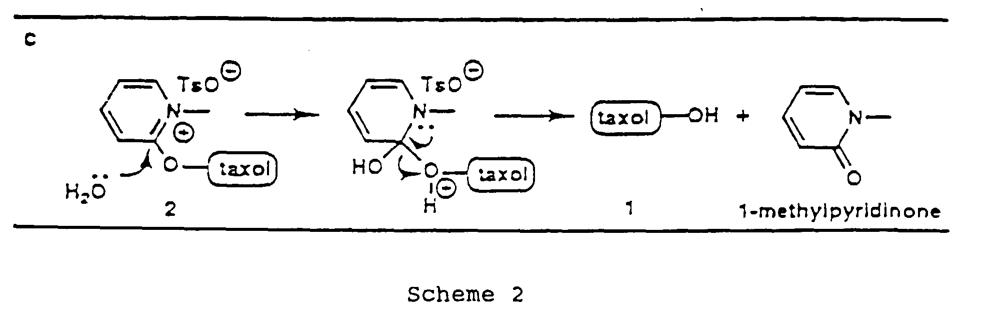

However, the release of taxol from taxol-2'-MPT can also be catalyzed by serum protein and by proteins having nucleophilic groups. When contacted with serum protein, taxol-2'-MPT is observed to displace its MPT group and form a protein:taxol intermediate. Dialysis of the protein:taxol intermediate indicates a dissociation period of hours or days. Displacement of the MPT group by serum proteins seems to be specific for such serum proteins. Tested non-serum proteins seemed to lack this activity. In particular, immunoglobulins and serum albumen seem to be particularly effective displacing the MPT group and forming protein:taxol intermediates. The precise nature of the bonding between the protein and taxol has not been characterized. Scheme 2 illustrates alternative pathways for MPT release.

### Discussion

Taxol-2'-MPT has proven to be a remarkably stable compound in most aqueous media. It is probable that this stability is conferred upon **2** by the facile formation of supramolecular aggregates, a process that is probably driven by the amphiphillic nature of the compound. The stability, water solubility, and lack of cytotoxicity of taxol-2'-MPT makes this class of compound an ideal a prodrug for taxol and memetics of taxol. While essentially completely stable in aqueous media at physiological pH and ion strength, the compound rapidly discharges taxol in sera. This profile is ideal for a clinically useful prodrug to taxol. It is possible that these properties should allow the formulation of taxol-2'-MPT (**2**) without the use of Cremaphor™ or ethanol.

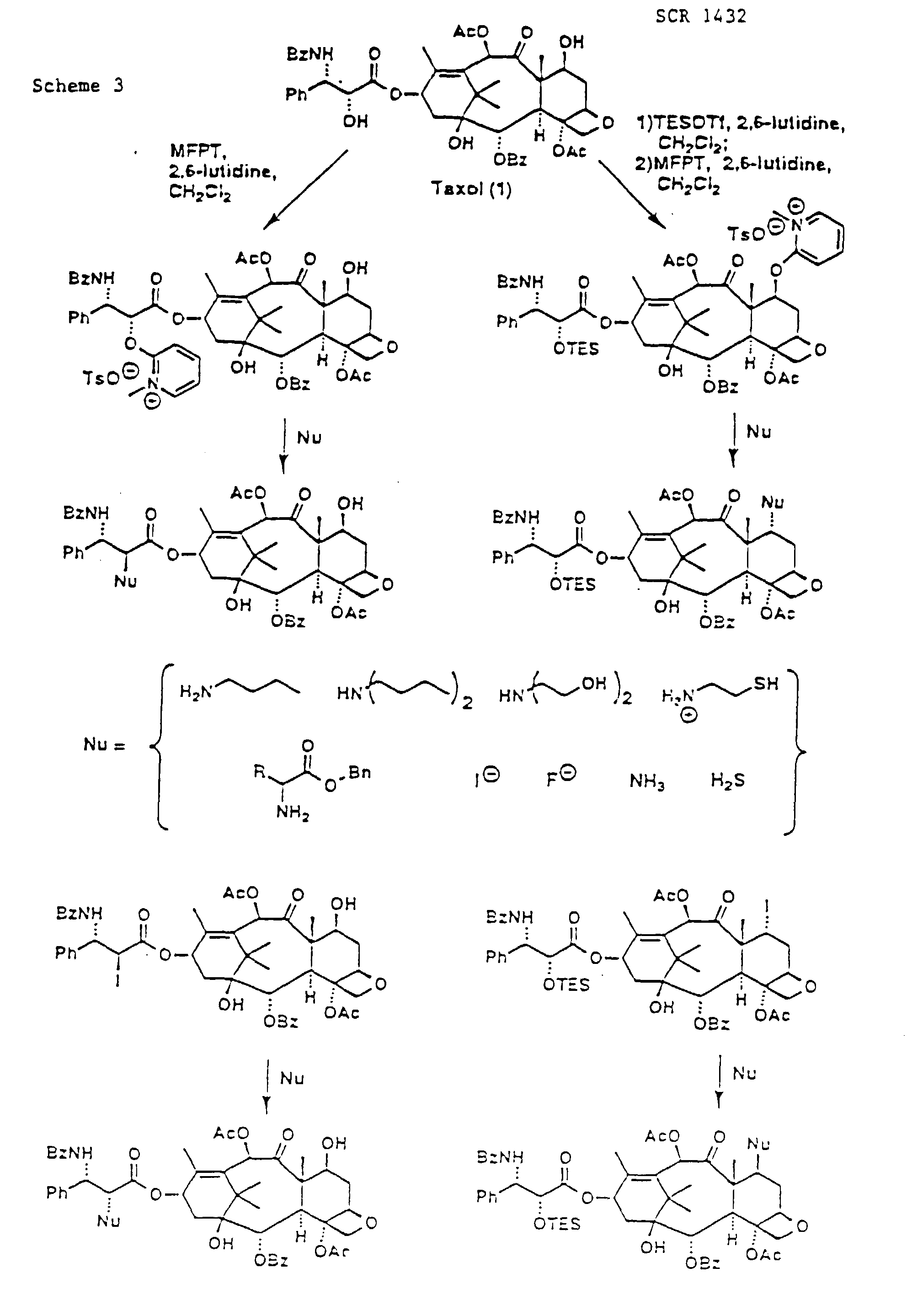

### Synthetic Methods

### Preparation of 7-TES deacetylbaccatin III (4)

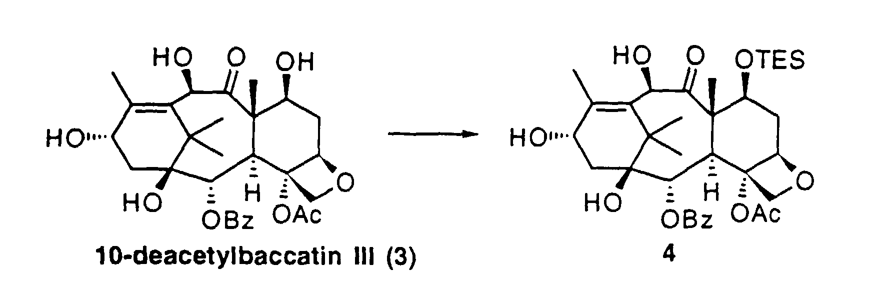

**7-TES deacetylbaccatin III (4).** To a solution of 10-deacetylbaccatin III (**3**, 3.0 g, 5.51 mmol, Indena Corpation, Italy) in pyridine (250 mL) was added chlorotriethylsilane (18.5 mL, 110 mmol) dropwise. The resulting solution was stirred at 25 °C for 17 hours. After dilution with diethylether (750 mL), the solution was washed with aqueous CuSO₄ (3 x 200 mL) and brine (200 mL). The organic layer was dried (MgSO₄), concentrated, and purified by flash chromatography (silica, 35 →50% ethylacetate in petroleum ether) to give alcohol 4 (3.39 g, 91%) as a white solid.

**Physical Data for 7-TES deacetylbaccatin III (4).** R_{f} = 0.32 (silica, 50% ethylacetate in hexanes); IR (thin film) νₘₐₓ 3464, 2954, 2282, 1710, 1453, 1362, 1271, 1242, 1105, 994 cm⁻¹ ; ¹H NMR (500 MHz, CDCl₃) δ 8.06 (dd, *J* = 8.0, 0.9 Hz, 2 H, Bz), 7.57 (t, *J*= 7.9 Hz, 1 H, Bz), 7.44 (t, *J*= 7.9 Hz, 2 H, Bz), 5.56 (d, *J*= 7.0 Hz, 1 H, 2-H), 5.14 (d, *J*= 1.9 Hz, 1 H, 10-H), 4.92 (d, *J*= 9.5 Hz, 1 H, 5-H), 4.84-4.78 (m, 1 H, 13-H), 4.37 (dd, *J*= 10.6, 7.0 Hz, 1 H, 7-H), 4.27 (d, *J*= 8.5 Hz, 1 H, 20-H), 4.25 (d, *J* = 1.9 Hz, 1 H, 10-OH), 4.12 (d, *J*= 8.5 Hz, 1 H, 20-H), 3.91 (d, *J*= 7.0 Hz, 1 H, 3-H), 2.48-2.40 (m, 1 H, 6-H), 2.25 (s, 3 H, Me), 2.25-2.17 (m, 2 H, 14-CH₂), 2.04 (s, 3 H, Me), 1.90-1.82 (m, 1 H, 6-H), 1.70 (s, 3 H, Me), 1.03 (s, 6 H, Me, Me), 0.90 (t, *J*= 8 Hz, 9 H, Si(CH₂CH₃)₃), 0.58-0.42 (band, 6 H, Si(CH₂CH₃)₃); ¹³C NMR (125 MHz, CDCl₃) δ 210.3, 170.8, 167.0, 141.8, 135.1, 133.6, 130.1, 129.4, 128.6, 84.2, 80.7, 78.8, 76.5, 74.8, 74.6, 72.9, 67.9, 57.9, 47.0, 42.7, 38.6, 37.2, 26.8, 22.6, 19.5, 15.2, 9.9, 6.7, 5.1; FAB HRMS (NBA / CsI) m / e 791.2251, M + Cs⁺ calcd for C₃₅H₅₀O₁₀Si 791.2228.

### Preparation of enone 5

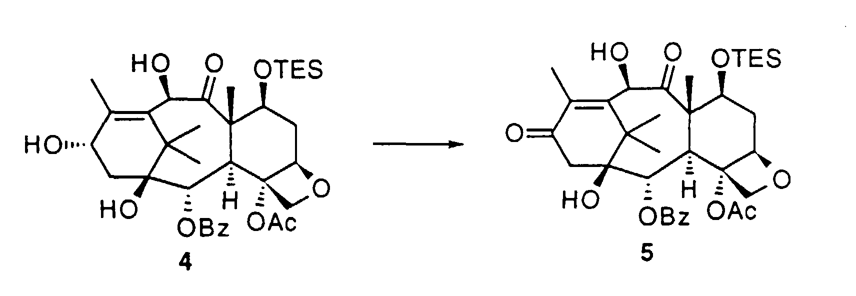

**Enone 5**. To a solution of 7-TES deacetylbaccatin III (**4**, 1.5 g, 2.28 mmol) and 4-methylmorpholine N-oxide (NMO, 240 mg, 2.05 mmol) in CH₂Cl₂ (5 mL) was added 4 A molecular sieves (200 mg) and the suspension was stirred at 25 °C for 10 minutesutes. A catalytic amount of tetrapropylammonium perruthenate from Aldrich Chemical Company Inc. (TPAP, 40 mg, 0.11 mmol) was added by portions and the reaction mixture was stirred at 25 °C for 0.5 hours. Small amounts of 4-methylmorpholine N-oxide and TPAP were added alternatively at 0.5 hour intervals until the starting material was consumed to the extent of ca. 95% by TLC. The reaction mixture was filtered through silica gel, eluted with CH₂Cl₂ (100 mL), and concentrated to give enone **5** (1.44 g, 96%) as a white solid.

**Physical Data for Enone 5**. R_{f} = 0.5 (silica, 50% ethylacetate in hexanes); IR (thin film) νₘₐₓ 3446, 2957, 2882, 1726, 1672, 1456, 1367, 1243, 1106 cm⁻¹ ; ¹H NMR (500 MHz, CDCl₃) δ 8.05 (dd, *J* = 8.0, 1.0 Hz, 2 H, Bz), 7.61 (t, *J* = 7.5 Hz, 1 H, Bz), 7.45 (t, *J* = 7.5 Hz, 2 H, Bz), 5.63 (d, *J*= 7.5 Hz, 1 H, 2-H), 5.30 (d, *J* = 2.0 Hz, 1 H, 10-H), 4.90 (d, *J*= 8.0 Hz, 1 H, 5-H), 4.36 (dd, *J* = 10.5, 7.0 Hz, 1 H, 7-H), 4.31 (d, *J*= 8.5 Hz, 1 H, 20-H), 4.30 (d, *J* = 2.0 Hz, 1 H, 10-OH), 4.11 (d, *J* = 8.5 Hz, 1 H, 20-H), 3.93 (d, *J*= 7.5 Hz, 1 H, 3-H), 2.92 (d, *J*= 19.5 Hz, 1 H, 14-H), 2.62 (d, *J* = 19.5 Hz, 1 H, 14-H), 2.50-2.42 (m, 1 H, 6-H), 2.17 (s, 3 H, Me), 2.08 (s, 3 H, Me), 1.90-1.82 (m, 1 H, 6-H), 1.77 (s, 1 H, 1-OH), 1.70 (s, 3 H, Me), 1.21 (s, 3 H, Me), 1.14 (s, 3 H, Me), 0.90 (t, *J* = 8.0 Hz, 9 H, Si(CH₂CH₃)₃), 0.60-0.42 (band, 6 H, Si(CH₂CH₃)₃); ¹³C NMR (125 MHz, CDCl₃) δ 208.2, 198.1, 170.2, 166.8, 156.6, 139.1, 134.0, 130.0, 128.8, 128.8, 84.0, 80.4, 78.5, 76.2, 75.7, 72.9, 72.8, 58.8, 45.9, 43.4, 42.5, 37.2, 33.0, 21.7, 17.5, 13.6, 9.6, 6.7, 5.1; FAB HRMS (NBA / NaI) *m / e* 657.3070, M + Na⁺ calcd for C₃₅H₄₈O₁₀Si 657.3095.

### Preparation of triol 6

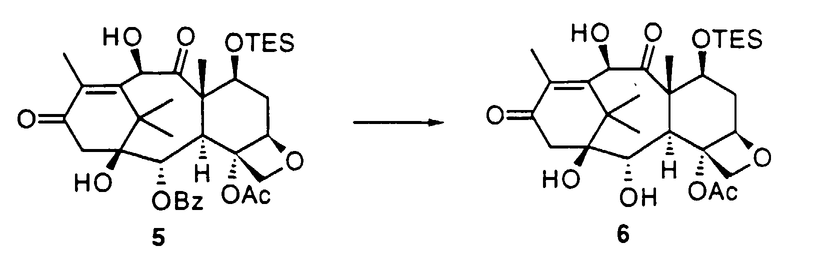

**Triol 6.** To a solution of enone **5** (1.44 g, 2.19 mmol) in MeOH (300 mL) at 0 °C was slowly added an aqueous solution of K₂CO₃ (3.0 g in 32 mL of H₂O). The solution was stirred at 0 °C for 2.5 hours. The reaction was then quenched with aqueous NH₄Cl (150 mL) and the resulting mixture was extracted with CH₂Cl₂ (2 x 200 mL). The organic layer was dried (Na₂SO₄), concentrated, and purified by flash chromatography (silica, 35 → 50% ethylacetate in petroleum ether) to give enone 5 (270 mg, 19%) and triol 6 (912 mg, 93% based on 81% conversion).

**Physical Data for Triol 6**. R_{f} = 0.24 (silica, 50% ethylacetate in hexanes); IR (thin film) νₘₐₓ 3414, 2957, 2881, 1727, 1664, 1370 cm⁻¹ ; ¹H NMR (500 MHz, CDCl₃) δ 5.23 (d, *J*= 9.5 Hz, 1 H, 10-H), 4.89 (d, *J*= 9.5 Hz, 1 H, 5-H), 4.63 (d, *J*= 9.5 Hz, 1 H, 20-H), 4.56 (d, *J*= 9.5 Hz, 1 H, 20-H), 4.32 (dd, *J*= 11.0, 7.0 Hz, 1 H, 7-H), 4.28 (d, *J* = 2.5 Hz, 1 H, 10-OH), 3.89 (dd, *J* = 6.5, 4.0 Hz, 1 H, 2-H), 3.57 (d, *J*= 6.5 Hz, 1 H, 3-H), 2.78 (d, *J*= 19.5 Hz, 1 H, 14-H), 2.58 (d, 4.0 Hz, 1 H, 2-OH), 2.52 (d, *J*= 19.5 Hz, 1 H, 14-H), 2.49-2.42 (m, 1 H, 6-H), 2.03 (s, 3 H, Me), 1.92-1.84 (m, 1 H, 6-H), 1.68 (s, 3 H, Me), 1.21 (s, 3 H, Me), 1.04 (s, 3 H, Me), 0.90 (t, *J*= 8.0 Hz, 9 H, Si(CH₂CH₃)₃), 0.60-0.40 (band, 6 H, Si(CH₂CH₃)₃); ¹³C NMR (125 MHz, CDCl₃) δ 208.9, 198.5, 170.1, 156.7, 138.8, 83.8, 81.2, 77.6, 75.7, 72.8, 72.5, 58.8, 45.8, 43.1, 42.8, 37.3, 32.7, 21.6, 17.5, 13.6, 9.7, 6.7, 5.1; FAB HRMS (NBA / NaI) *m/e* 575.2648, M + Na⁺ calcd for C₂₈H₄₄O₉Si 575.2652.

### Preparation of Carbonate 7

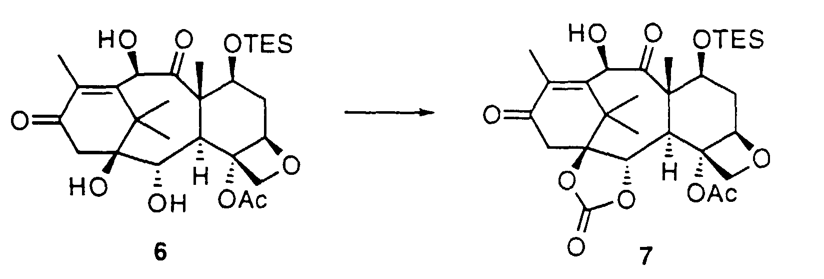

**Carbonate 7.** A solution of triol **6** (60.0 mg, 0.109 mmol) in THF (2 mL) was treated with carbonyldiimidazole (110.0 mg, 0.678 mmol) and stirred at 40 °C for 0.5 hour The reaction mixture was concentrated and redisolved in THF (5 mL). TLC analysis confirmed total consumption of starting material. 1N aqueous HCl (5 mL) was added and the resulting solution was allowed to stir for 15 minutes at 25 °C. diethylether (25 mL) was added, the organic layer was separated, washed with aqueous NaHCO₃ (10 mL) and brine (10 mL), dried (MgSO₄), and concentrated to give carbonate 7 (58 mg, 93%) as a white foam.

**Physical Data for Carbonate 7**. R_{f} = 0.50 (silica, 35% ethylacetate in hexanes); IR (thin film) νₘₐₓ 3438, 2957, 2882, 1820, 1731, 1685, 1370, 1236 cm⁻¹ ; ¹H NMR (500 MHz, CDCl₃) δ 5.27 (d, *J*= 2.5 Hz, 1 H, 10-H), 4.89 (d, *J*= 9.0 Hz, 1 H, 5-H), 4.60 (d, *J* = 9.0 Hz, 1 H, 20-H), 4.45 (d, *J*= 9.0 Hz, 1 H, 20-H), 4.43 (d, *J*= 6.0 Hz, 1 H, 2-H), 4.33 (dd, *J*= 10.0, 7.5 Hz, 1 H, 7-H), 4.28 (d, *J* = 2.5 Hz, 1 H, 10-OH), 3.54 (d, *J* = 6.0 Hz, 1 H, 3-H), 2.88 (d, *J*= 20.0 Hz, 1 H, 14-H), 2.75 (d, *J* = 20.0 Hz, 1 H, 14-H), 2.54-2.47 (m, 1 H, 6-H), 2.08 (s, 3 H, Me), 2.06 (s, 3 H, Me), 1.92-1.84 (m, 1 H, 6-H), 1.77 (s, 3 H, Me), 1.31 (s, 3 H, Me), 1.15 (s, 3 H, Me), 0.88 (t, *J*= 8.5 Hz, 9 H, Si(CH₂CH₃)₃), 0.55-0.45 (band, 6 H, Si(CH₂CH₃)₃); ¹³C NMR (125 MHz, CDCl₃) δ 208.4, 195.5, 170.5, 154.0, 152.0, 141.2, 88.4, 83.9, 79.8, 79.0, 76.7, 75.7, 71.9, 60.3, 43.0, 41.6, 39.8, 37.7, 31.6, 21.5, 17.8, 14.4, 9.7, 6.6, 5.0; FAB HRMS (NBA) *m/e* 579.2652, M + H⁺ calcd for C₂₉H₄₂O₁₀Si 579.2626.

### Preparation of nButyl-C-2 ester derivative (Alcohol 8)

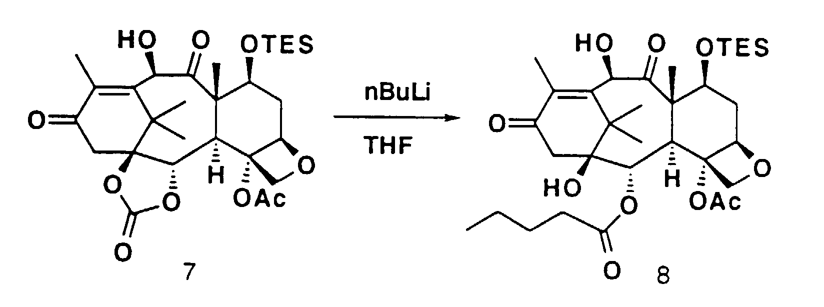

**Alcohol 8**. A solution of carbonate **7** (10 mg, 0.0173 mmol) in tetrahydrofuran (1 mL) at -78 °C was treated with n-Butyllithium from Aldrich Chemical Company, Inc. (0.087 mL of a 1.6 M solution in hexanes, 0.139 mmol) and stirred for 1.0 hour The reaction mixture was poured into a mixture of diethylether (10 mL) and aqueous NH₄Cl (5 mL). The organic layer was separated and the aqueous layer was extracted with diethylether (2 x 5 mL). The combined organic layer was washed with a saturated solution of brine (5 mL), dried (MgSO₄), concentrated, and purified by flash chromatography (silica, 35 → 50% ethylacetate in hexanes) to give 8 (7.9 mg, 72%) as an amorphous solid.

**Physical Data for Alcohol 8**. R_{f} = 0.36 (silica, 35% ethylacetate in petroleum ether); IR (film) νₘₐₓ 3437, 2962, 2865, 1726, 1671, 1367, 1239, 1105 cm⁻¹ ; ¹H NMR (500 MHz, CDCl₃) δ 5.36 (d, *J*= 6.5 Hz, 1H, 2-H), 5.26 (d, *J*= 2.5 Hz, 1H, 10-H), 4.89 (br d, *J*= 8.0 Hz, 1H, 5-H), 4.47 (d, *J*= 8.0 Hz, 1H, 20-H), 4.32 (dd, *J*= 10.5, 6.5 Hz, 1H, 7-H), 4.26 (d, *J*= 2.5 Hz, 1H, 10-OH), 4.15 (d, *J* = 8.0 Hz, 1H, 20-H), 3.81 (d, *J* = 6.5 Hz, 1H, 3-H), 2.73 (d, *J* = 20.0 Hz, 1H, 14-H), 2.57 (d, *J* = 20.0 Hz, 1H, 14-H), 2.49-2.41 (m, 1H, 6-H), 2.38-2.23 (m, 2H, OCCH₂(CH₂)₂CH₃), 2.06 (s, 3H, Me), 2.04 (s, 3H, Me), 1.90-1.82 (m, 1H, 6-H), 1.67 (s, 1H, OH), 1.64 (s, 3H, Me), 1.68-1.52 (m, 2H, OCCH₂CH₂CH₂CH₃), 1.41-1.30 (m, 2H, OC(CH₂)₂CH₂CH₃), 1.19 (s, 3H, Me), 1.07 (s, 3 H, Me), 0.94-0.86 (band, 12H, CH₃ of Bu, OSi(CH₂CH₃)₃), 0.58-0.45 (band, 6H, OSi(CH₂CH₃)₃); FAB HRMS (NBA) *m/e* 637.3421, M + H⁺ calcd for C₃₃H₅₂O₁₀Si 637.3408.

### Preparation of vinyl-C-2 ester derivative (Alcohols 9 and 10)

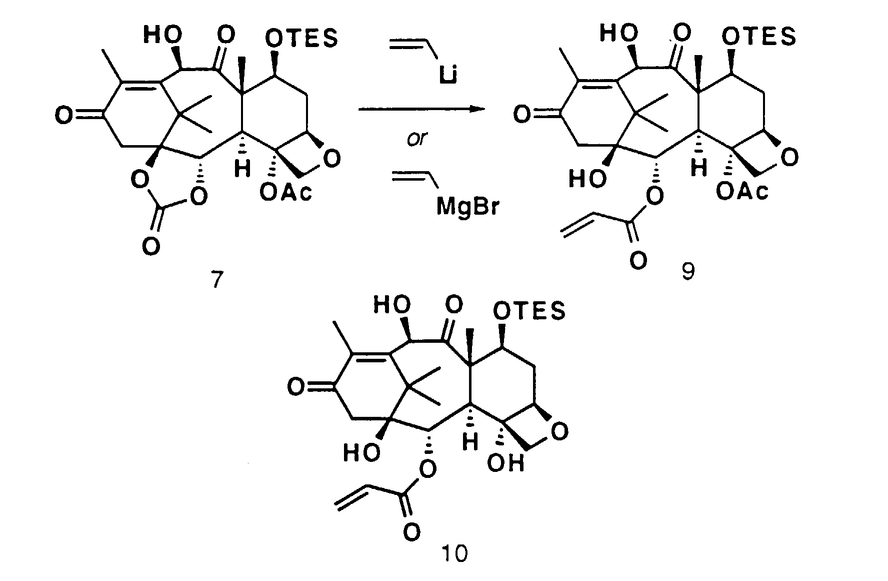

**Alcohols 9 and 10**. A solution of carbonate **7** (111.3 mg, 0.192 mmol) in tetrahydrofuran (2 mL) at -78 °C was treated with vinyllithium (3.7 mL of a 0.52 M solution in diethylether, 1.92 mmol, prepared from tetravinyltin and nButyllithium: methodology from Wakefield, B.J. *Organolithium Methods,* Academic Press: London, 1988, p. 46) and stirred for 2.25 hour The reaction mixture was poured into a mixture of CH₂Cl₂ (20 mL) and aqueous NH₄Cl (10 mL), the organic layer was separated, and the aqueous layer was extracted with CH₂Cl₂ (3 x 10 mL). The combined organic layer was washed with brine (15 mL), dried (MgSO₄), concentrated, and purified by flash chromatography (silica, 30 → 50% ethylacetate in petroleum ether) to give 9 (60.0 mg, 52%), and 10 (25.7 mg, 24% ) as white foams.

**Physical Data for Alcohol 9.** R_{f} = 0.52 (silica, 50% ethylacetate in hexanes); IR (film) νₘₐₓ 3442, 2956, 2882, 1727, 1672, 1407, 1368, 1243, 1182, 1110, 1050, 986, 826, 736 cm⁻¹ ; ¹H NMR (500 MHz, CDCl₃) δ 6.51 (dd, *J*= 17.0, 1.0 Hz, 1H, vinyl H), 6.13 (dd, *J*= 17.0, 10.5 Hz, 1H, vinyl H), 6.00 (dd, *J* = 10.5, 1.0 Hz, 1H, vinyl H), 5.45 (br d, *J*= 6.5 Hz, 1H, 2-H), 5.30 (d, *J*= 2.5 Hz, 1H, 10-H), 4.91 (br d,*J*= 9.5 Hz, 1H, 5-H), 4.44 (d, *J*= 8.5 Hz, 1H, 20-H), 4.35 (dd, *J*= 10.5, 6.5 Hz, 1H, 7-H), 4.30 (d, *J*= 2.5 Hz, 1H, 10-OH), 4.14 (d, *J*= 8.5 Hz, 1H, 20-H), 3.88 (d, *J*= 6.5 Hz, 1H, 3-H), 2.79 (d, *J*= 20.0 Hz, 1H, 14-H), 2.61 (d, *J*= 20.0 Hz, 1H, 14-H), 2.48 (ddd, *J* = 14.5, 9.5, 6.5 Hz, 1H, 6-H), 2.09 (s, 3H, Me), 2.08 (s, 3H, Me), 1.89 (ddd, *J*= 14.5, 10.5, 2.0 Hz, 1H, 6-H), 1.72 (s, 1H, OH), 1.68 (s, 3H, Me), 1.22 (s, 3H, Me), 1.12 (s, 3H, Me), 0.92 (t, *J*= 8.0 Hz, 9H, OSi(CH₂CH₃)₃), 0.62-0.46 (band, 6H, OSi(CH₂CH₃)₃); FAB HRMS (NBA / CsI) *m/e* 739.1925, M + Cs⁺ calcd for C₃₁H₄₆O₁₀Si 739.1915.

**Physical Data for Alcohol 10.** R_{f} = 0.24 (silica, 50% ethylacetate in hexanes); IR (film) νₘₐₓ 3439, 2955, 2881, 1711, 1671, 1409, 1365, 1188, 1115, 980, 833, 735 cm⁻¹; ¹H NMR (500 MHz, CDCl₃) δ 6.48 (br d, *J* = 17.0 Hz, 1H, vinyl H), 6.10 (dd, *J*= 17.0, 10.5 Hz, 1H, vinyl H), 5.97 (br d, *J*= 10.5 Hz, 1H, vinyl H), 5.47 (br d, *J*= 6.0 Hz, 1H, 2-H), 5.25 (d, *J*= 2.5 Hz, 1H, 10-H), 4.75 (dd, *J*= 9.5, 3.5 Hz, 1H, 5-H), 4.38 (d, *J* = 8.5 Hz, 1H, 20-H), 4.30 (d, *J*= 2.5 Hz, 1H, 10-OH), 4.24 (d, *J*= 8.5 Hz, 1H, 20-H), 3.90 (dd, *J* = 11.5, 6.0 Hz, 1H, 7-H), 3.28 (d, *J* = 19.5 Hz, 1H, 14-H), 3.24 (d, *J*= 6.0 Hz, 1H, 3-H), 3.06 (br s, 1H, OH), 2.58 (d, *J*= 19.5 Hz, 1H, 14-H), 2.38 (ddd, *J*= 14.5, 9.5, 6.0 Hz, 1H, 6-H), 2.07 (s, 3H, Me), 1.98 (ddd, *J*= 14.5, 11.5, 3.5 Hz, 1H, 6-H), 1.87 (s, 1H, OH), 1.61 (s, 3H, Me), 1.23 (s, 3H, Me), 1.13 (s, 3H, Me), 0.90 (t, *J*= 8.0 Hz, 9H, OSi(CH₂CH₃)₃), 0.59-0.45 (band, 6H, OSi(CH₂CH₃)₃); FAB HRMS (NBA / CsI) *m/e* 697.1802, M + Cs⁺ calcd for C₂₉H₄₄O₉Si 697.1809.

### Preparation of 2-Furyl-C-2 ester derivative (Alcohol 11)

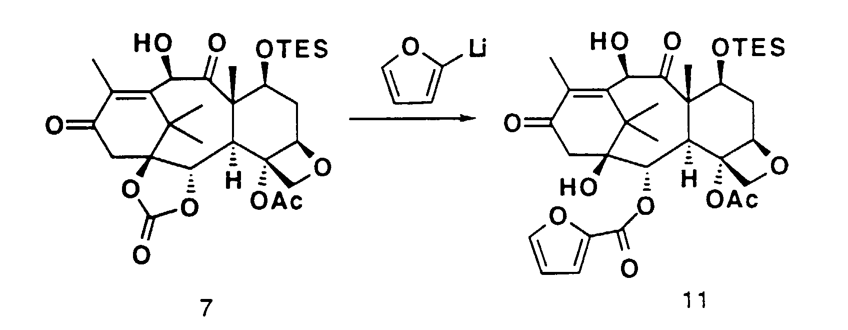

**Alcohol 11**. A solution of carbonate **7** (46 mg, 0.0795 mmol) in tetrahydrofuran (3 mL) at -78 °C was treated with 2-furyllithium (4 mL of a 0.47 M suspension in diethylether, 1.88 mmol, prepared from furan (Aldrich Chemical Company, Inc.) and n-Butyllithium (Aldrich Chemical Company, Inc.); methodology from Ramanathan, V.; Levine, R. *J*. *Org. Chem.* **1962**, 27, 1216) and stirred for 10 minutesutes The reaction mixture was poured into a mixture of CH₂Cl₂ (15 mL) and aqueous NH₄Cl (20 mL). The organic layer was separated and the aqueous layer was extracted with CH₂Cl₂ (2 x 10 mL). The combined organic layer was washed with brine (10 mL), dried (MgSO₄) and concentrated to give **11** which was taken into the next step without further purification.

**Physical Data for Alcohol 11**. R_{f} = 0.38 (silica, 20% ethylacetate in petroleum ether); IR (film) νₘₐₓ 3442, 2956, 2882, 1727, 1672, 1468, 1300, 1240, 1110, 1007, 733 cm⁻¹ ; ¹H NMR (500 MHz, CDCl₃) δ 7.66-7.64 (m, 1H, furan), 7.24 (br d, *J*= 3.5 Hz, 1H, furan), 6.58 (dd, *J*= 3.5, 1.5 Hz, 1H, furan), 5.55 (d, *J* = 6.5 Hz, 1H, 2-H), 5.31 (d, *J*= 2.0 Hz, 1H, 10-H), 4.92 (br d, *J*= 9.0 Hz, 1H, 5-H), 4.43 (d, *J* = 8.5 Hz, 1H, 20-H), 4.37 (dd, *J* = 10.5, 6.5 Hz, 1H, 7-H), 4.32 (d, *J*= 2.0 Hz, 1H, 10-OH), 4.18 (d, *J* = 8.5 Hz, 1H, 20-H), 3.93 (d, *J* = 6.5 Hz, 1H, 3-H), 2.88 (d, *J* = 20.0 Hz, 1H, 14-H), 2.63 (d, *J*= 20.0 Hz, 1H, 14-H), 2.55-2.37 (m, 1H, 6-H), 2.15 (s, 3H, Me), 2.09 (s, 3H, Me), 1.93-1.87 (m, 1H, 6-H), 1.81 (s, 1H, OH), 1.71 (s, 3H, Me), 1.23 (s, 3H, Me), 1.15 (s, 3H, Me), 0.93 (t, *J*= 8.0 Hz, 9H, OSi(CH₂CH₃)₃), 0.62-0.42 (band, 6H, OSi(CH₂CH₃)₃); FAB HRMS (NBA / NaI) *m/e* 669.2717, M + Na⁺ calcd for C₃₃H₄₆O₁₁Si 669.2707.

### Preparation of 2-thiophenyl-C-2 ester derivative (Alcohol 12)

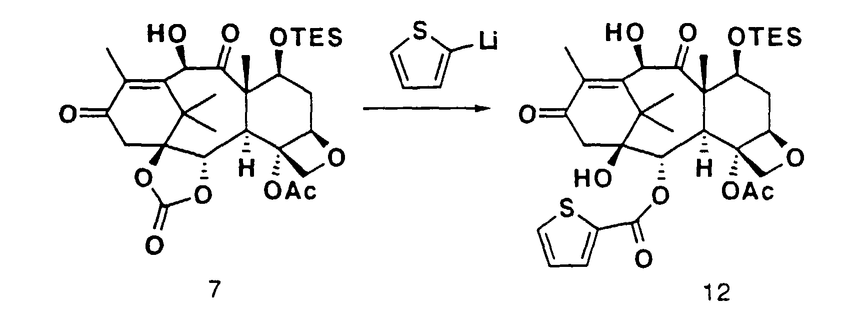

**Alcohol 12**. A solution of carbonate **7** (50.0 mg, 0.0864 mmol) in tetrahydrofuran (5 mL) at -78 °C was treated with 2-thienyllithium from Aldrich Chemical Company, inc. (1.30 mL of a 1.0 M solution in tetrahydrofuran, 1.30 mmol) and stirred for 0.5 hour The reaction mixture was poured into a mixture of diethylether (10 mL) and aqueous NH₄Cl (5 mL). The organic layer was separated and the aqueous layer was extracted with diethylether (2 x 10 mL). The combined organic layer was washed with brine (10 mL), dried (MgSO₄), concentrated, and purified by flash chromatography (silica, 10 → 35% ethylacetate in hexanes) to give **7** (16.5 mg, 33%), **12** (36.8 mg, 96% based on 67% conversion) as an amorphous solid.

**Physical Data for Alcohol 12.** R_{f} = 0.56 (silica, 50% ethylacetate in hexanes); IR (film) νₘₐₓ 3403, 2945, 2881, 1717, 1669, 1520, 1413, 1360, 1248, 1078; ¹H NMR (500 MHz, CDCl₃) δ 7.84 (dd, *J* = 3.5, 1.0 Hz, 1H, thiophene), 7.64 (d, *J*= 1.0, 5.0 Hz, 1H, thiophene), 7.14 (dd, *J*= 5.0, 3.5 Hz, 1H, thiophene), 5.53 (br d, *J* = 6.5 Hz, 1H, 2-H), 5.29 (d, *J* = 2.5 Hz, 1H, 10-H), 4.90 (br d, *J* = 7.5 Hz, 1H, 5-H), 4.44 (d, *J* = 8.5 Hz, 1H, 20-H), 4.35 (dd, *J*= 10.5 Hz, 6.5 Hz, 1H, 7-H), 4.29 (d, *J*= 2.5 Hz, 1H, 10-OH), 4.19 (d, *J*= 8.5 Hz, 1H, 20-H), 3.90 (d, *J*= 6.5 Hz, 1H, 3-H), 2.89 (d, *J*= 19.5 Hz, 1H, 14-H), 2.62 (d, *J*= 19.5 Hz, 1H, 14-H), 2.49-2.43 (m, 1H, 6-H), 2.15 (s, 3H, Me), 2.07 (s, 3H, Me), 1.92-1.84 (m, 1H, 6-H), 1.73 (s, 1H, OH), 1.71 (s, 3H, Me), 1.21 (s, 3H, Me), 1.13 (s, 3H, Me), 0.91 (t, *J* = 8.0 Hz, 9H, OSi(CH₂CH₃)₃), 0.56-0.49 (band, 6H, OSi(CH₂CH₃)₃); FAB HRMS (NBA) *m/e* 663.2655, M + H⁺ calcd for C₃₃H₄₆O₁₀SSi 663.2659.

### Preparation of 3-thiophenyl-C-2 ester derivatives (Alcohol 13 and 14)

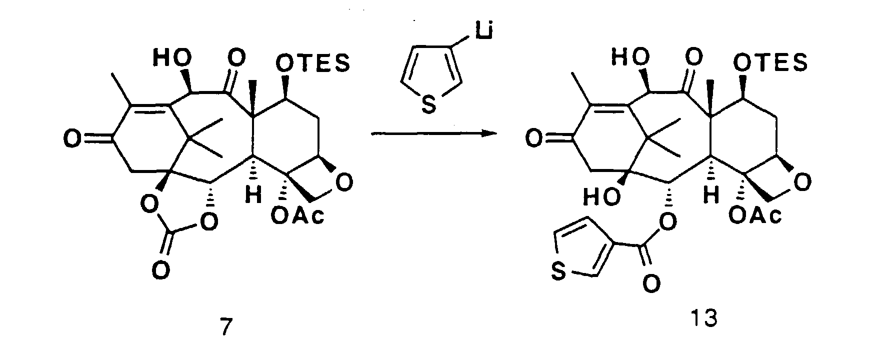

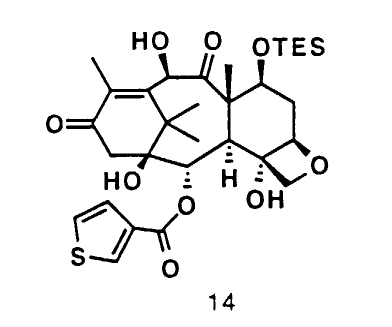

**Alcohols 13 and 14**. A solution of carbonate **7** (107.9 mg, 0.186 mmol) in tetrahydrofuran (6.2 mL) at -78 °C was treated with 3-thienyllithium (2.76 mL of a 0.41 M solution in diethylether : tetrahydrofuran : hexanes (4.5 : 1 : 2), 1.13 mmol, prepared from 3-bromothiophene and *n*-Butyllithium; methodology from Camici, L.; Ricci, A.; Taddei, M. *Tetrahedron Lett*. **1986**, *27*, 5155) and stirred for 1.5 hour The reaction mixture was poured into a mixture of CH₂Cl₂ (15 mL) and aqueous NH₄Cl (20 mL), the organic layer was separated, and the aqueous layer was extracted with CH₂Cl₂ (2 x 10 mL). The combined organic layer was washed with brine (10 mL), dried (MgSO₄), concentrated, and purified by flash chromatography (silica, 20 → 30% ethylacetate in hexanes) to give **7** (16.9 mg, 16%), **13** (87.0 mg, 83% based on 84% conversion), and hydrolyzed C4 acetate **14** (C4-hydrolyzed side product, 9.7 mg, 10% based on 84% conversion) as amorphous solids.

**Physical Data for Alcohol 13**. R_{f} = 0.74 (silica, 50% ethylacetate in hexanes), 0.41 (silica, 10% ethylacetate in benzene, 3 elutions); IR (thin film) νₘₐₓ 3442, 3110, 2956, 2882, 1725, 1672, 1410, 1368, 1244, 1198, 1101, 988, 825, 744 cm⁻¹ ; ¹H NMR (500 MHz, CDCl₃) δ 8.18 (dd, *J* = 3.0, 1.2 Hz, 1H, thiophene), 7.54 (dd, *J* = 5.0, 1.2 Hz, 1H, thiophene), 7.37 (dd, *J* = 5.0, 3.0 Hz, 1H, thiophene), 5.56 (dd, *J* = 6.5, 1.0 Hz, 1H, 2-H), 5.31 (d, *J* = 2.5 Hz, 1H, 10-H), 4.92 (dd, *J* = 7.5, 2.0 Hz, 1H, 5-H), 4.40-4.34 (m, 2H, 20-H, 7-H), 4.31 (d, *J* = 2.5 Hz, 1H, 10-OH), 4.15 (d, *J* = 8.5 Hz, 1H, 20-H), 3.93 (d, *J* = 6.5 Hz, 1H, 3-H), 2.88 (d, *J* = 20 Hz, 1H, 14-H), 2.63 (dd, *J* = 20.0, 1.0 Hz, 1H, 14-H), 2.47 (ddd, *J*= 14.5, 9.5, 6.5 Hz, 1H, 6-H), 2.18 (s, 3H, Me), 2.10 (s, 3H, Me), 1.89 (ddd, *J*= 14.5, 10.5, 2.0 Hz, 1H, 6-H), 1.81 (br s, 1H, OH), 1.72 (s, 3H, Me), 1.23 (s, 3H, Me), 1.15 (s, 3H, Me), 0.93 (t, *J*= 8.0 Hz, 9H, OSi(CH₂CH₃)₃), 0.62-0.48 (band, 6H, Si(CH₂CH₃)₃); FAB HRMS (NBA / CsI) *m/e* 795.1640, M + Cs⁺ calcd for C₃₃H₄₆O₁₀SSi 795.1635.

**Physical Data for Alcohol 14**: R_{f} = 0.54 (silica, 50% ethylacetate in hexanes); IR (thin film) νₘₐₓ 3437, 3108, 2955, 2880, 1709, 1674, 1605, 1520, 1410, 1360, 1258, 1194, 1103, 1004, 829, 744 cm⁻¹ ; ¹H NMR (500 MHz, CDCl₃) δ 8.15 (dd, *J*= 3.0, 1.0 Hz, 1H, thiophene), 7.49 (dd, *J*= 5.0, 1.0 Hz, 1H, thiophene), 7.35 (dd, *J* = 5.0, 3.0 Hz, 1H, thiophene), 5.59 (d, *J* = 6.0 Hz, 1H, 2-H), 5.27 (d, *J*= 2.5 Hz, 1H, 10-H), 4.73 (dd, *J*= 9.5, 3.5 Hz, 1H, 5-H), 4.40 (d, *J* = 8.5 Hz, 1H, 20-H), 4.32 (d, *J* = 2.5 Hz, 1H, 10-OH), 4.15 (d, *J* = 8.5 Hz, 1H, 20-H), 3.92 (dd, *J* = 11.5, 6.0 Hz, 1H, 7-H), 3.44 (d, *J*= 19.5 Hz, 1H, 14-H), 3.30 (d, *J*= 6.0 Hz, 1H, 3-H), 2.91 (br s, 1H, OH), 2.61 (d, *J* = 19.5 Hz, 1H, 14-H), 2.38 (ddd, *J*= 14.5, 9.5, 6.0 Hz, 1H, 6-H), 2.09 (s, 3H, Me), 1.99 (ddd, *J* = 14.5, 11.5, 3.5 Hz, 1H, 6-H), 1.81 (br s, 1H, OH), 1.65 (s, 3H, Me), 1.24 (s, 3H, Me), 1.16 (s, 3H, Me), 0.91 (t, *J*= 8.0 Hz, 9H, OSi(CH₂CH₃)₃), 0.60-0.46 (band, 6H, OSi(CH₂CH₃)₃); FAB HRMS (NBA / CsI) *m/e* 753.1530, M + Cs⁺ calcd for C₃₁H₄₄O₉SSi 753.1530.

### Preparation of 2-pyridinyl-C-2 ester derivatives (Alcohol 15, 16 and triol 6)

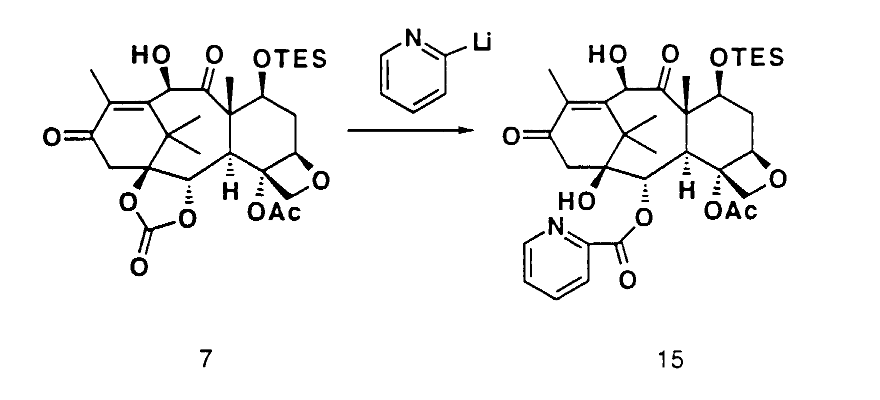

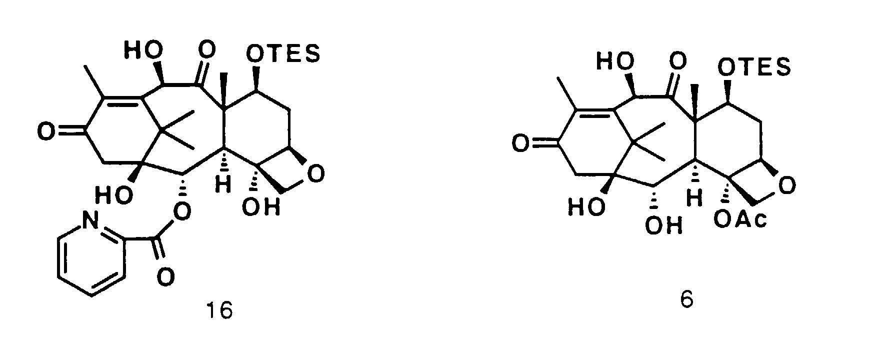

**Alcohol 15 and 16, and triol 6**. A solution of carbonate **7** (62.6 mg, 0.108 mmol) in tetrahydrofuran (5.4 mL) at -78 °C was treated with 2-lithiopyridine (1.15 mL of a 0.44 M solution in diethylether-pentane 1 : 1, 0.506 mmol, prepared from 2-bromopyridine and *t*-Butyllithium; methodology from Malmberg, H.; Nilsson, M. *Tetrahedron,* **1986**, *42*, 3981) and stirred for 1.3 hour The reaction mixture was poured into a mixture of ethylacetate (10 mL) and aqueous NH₄Cl (5 mL), the organic layer was separated, and the aqueous layer was extracted with ethylacetate (2 x 10 mL). The combined organic layer was washed with brine (5 mL), dried (MgSO₄), concentrated, and purified by flash chromatography (silica, 70 → 100% ethylacetate in petroleum ether) to give 6 (16.3 mg, 27%), 15 (28.0 mg, 39%), and 16 (8.4 mg, 13%) as amorphous solids.

**Physical Data for Alcohol 15**. R_{f} = 0.60 (silica, ethylacetate); ¹H NMR (500 MHz, CDCl₃) δ 8.77 (ddd, *J*= 4.5, 1.7, 1.0 Hz, 1H, pyridine), 8.05 (br d, *J*= 7.5 Hz, 1H, pyridine), 7.89 (ddd, *J*= 7.5, 7.5, 1.7 Hz, 1H, pyridine), 7.53 (ddd, *J*= 7.5, 4.5, 1.0 Hz, 1H, pyridine), 5.61 (dd, *J*= 6.5, 1.0 Hz, 1H, 2-H), 5.33 (d, *J* = 2.5 Hz, 1H, 10-H), 4.92 (dd,*J* = 9.5, 2.0 Hz, 1H, 5-H), 4.39 (dd, *J*= 10.5, 6.5 Hz, 1H, 7-H), 4.36 (d, *J* = 9.0 Hz, 1H, 20-H), 4.33 (d, *J* = 2.5 Hz, 1H, 10-OH), 4.28 (d, *J* = 9.0 Hz, 1H, 20-H), 3.96 (d, *J*= 6.5 Hz, 1H, 3-H), 2.98 (d, *J*= 20.0 Hz, 1H, 14-H), 2.71 (dd, *J*= 20.0, 1.0 Hz, 1H, 14-H), 2.50 (s, 1H, OH), 2.48 (ddd, *J*= 14.5, 9.5, 6.5 Hz, 1H, 6-H), 2.15 (s, 3H, Me), 2.11 (s, 3H, Me), 1.90 (ddd, *J*= 14.5, 10.5, 2.0 Hz, 1H, 6-H), 1.76 (s, 3H, Me), 1.24 (s, 3H, Me), 1.16 (s, 3H, Me), 0.93 (t, *J* = 8.0 Hz, 9H, OSi(CH₂CH₃)₃), 0.63-0.47 (band, 6H, OSi(CH₂CH₃)₃); FAB HRMS (NBA / CsI) *m/e* 790.2060, M + Cs⁺ calcd for C₃₄H₄₇O₁₀NSi 790.2024.

**Physical Data for Alcohol 16**. R_{f} = 0.45 (silica, ethylacetate); IR (film) νₘₐₓ 3435, 2954, 2879, 1732, 1674, 1589, 1362, 1305, 1241, 1116, 998, 829, 741 cm⁻¹; ¹H NMR (500 MHz, CDCl₃) δ 8.73 (br d, *J* = 4.5 Hz, 1H, pyridine), 8.15 (br d, *J*= 7.5 Hz, 1H, pyridine), 7.90 (ddd, *J*= 7.5, 7.5, 1.7 Hz, 1H, pyridine), 7.56 (ddd, *J*= 7.5, 4.5, 1.0 Hz, 1H, pyridine), 5.53 (dd, *J*= 7.5, 1.0, 1H, 2-H), 5.30 (d, *J*= 2.5 Hz, 1H, 10-H), 4.84 (dd, *J*= 9.5, 3.0 Hz, 1H, 5-H), 4.81 (br s, 1H, OH), 4.31 (d, *J*= 2.5 Hz, 1H, 10-OH), 4.25 (s, 2H, 20-CH₂), 3.97 (dd, *J* = 11.5, 6.5 Hz, 1H, 7-H), 3.31 (d, *J*= 19.5 Hz, 1H, 14-H), 3.23 (d, *J*= 7.5 Hz, 1H, 3-H), 2.57 (br d, *J*= 19.5 Hz, 1H, 14-H), 2.43 (ddd, *J*= 14.5, 9.5, 6.5 Hz, 1H, 6-H), 2.11 (s, 3H, Me), 1.95 (ddd, *J* = 14.5, 11.5, 3.0 Hz, 1H, 6-H), 1.92 (br s, 1H, OH), 1.70 (s, 3H, Me), 1.24 (s, 3H, Me), 1.17 (s, 3H, Me), 0.91 (t, *J* = 8.0 Hz, 9H, OSi(CH₂CH₃)₃), 0.60-0.46 (band, 6H, OSi(CH₂CH₃)₃).

**Physical Data for Triol 6.** R_{f} = 0.24 (silica, 50% ethylacetate in hexanes); IR (thin film) νₘₐₓ 3414, 2957, 2881, 1727, 1664, 1370 cm⁻¹; ¹H NMR (500 MHz, CDCl₃) 8 5.23 (d, *J* = 9.5 Hz, 1 H, 10-H), 4.89 (d, *J*= 9.5 Hz, 1 H, 5-H), 4.63 (d,*J* = 9.5 Hz, 1 H, 20-H), 4.56 (d, *J* = 9.5 Hz, 1 H, 20-H), 4.32 (dd, *J* = 11.0, 7.0 Hz, 1 H, 7-H), 4.28 (d, *J* = 2.5 Hz, 1 H, 10-OH), 3.89 (dd, *J* = 6.5, 4.0 Hz, 1 H, 2-H), 3.57 (d, *J* = 6.5 Hz, 1 H, 3-H), 2.78 (d, *J* = 19.5 Hz, 1 H, 14-H), 2.58 (d, 4.0 Hz, 1 H, 2-OH), 2.52 (d, *J*= 19.5 Hz, 1 H, 14-H), 2.49-2.42 (m, 1 H, 6-H), 2.03 (s, 3 H, Me), 1.92-1.84 (m, 1 H, 6-H), 1.68 (s, 3 H, Me), 1.21 (s, 3 H, Me), 1.04 (s, 3 H, Me), 0.90 (t, *J*= 8.0 Hz, 9 H, Si(CH₂CH₃)₃), 0.60-0.40 (band, 6 H, Si(CH₂CH₃)₃); ¹³C NNIR (125 MHz, CDCl₃) δ 208.9, 198.5, 170.1, 156.7, 138.8, 83.8, 81.2, 77.6, 75.7, 72.8, 72.5, 58.8, 45.8, 43.1, 42.8, 37.3, 32.7, 21.6, 17.5, 13.6, 9.7, 6.7, 5.1; FAB HRMS (NBA / NaI) *m/e* 575.2648, M + Na⁺ calcd for C₂₈H₄₄O₉Si 575.2652.

### Preparation of 3-pyridinyl-C-2 ester derivative (Alcohol 17)

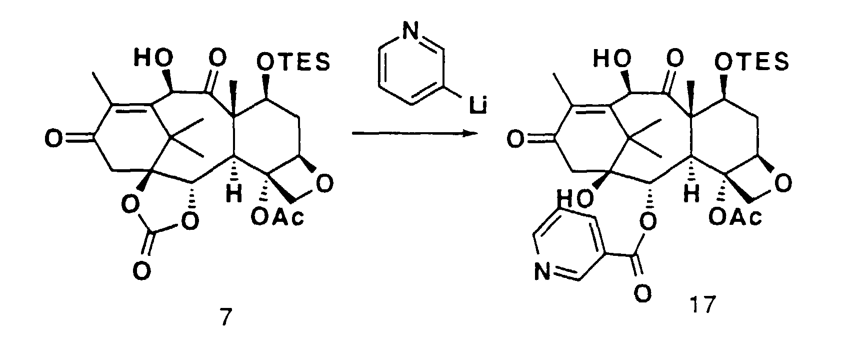

**Alcohol 17**. To a solution of 3-lithiopyridine (1.15 mmol) in tetrahydrofuran (7 mL), prepared from 3-bromopyridine (Aldrich Chemical Company Inc.) and *n*-Butyllithium (Aldrich Chemical Company Inc.) at -100 °C (methodology from Parham, W.E.; Piccirilli, R. M. *J. Org. Chem.* **1977**, *42*, 257), was added a solution of carbonate 7 (133.1 mg, 0.230 mmol) in tetrahydrofuran (2 mL) *via* cannula. The resulting solution was stirred for 1 h, allowed to warm to -78 °C, stirred for 1 h, and poured into a mixture of ethylacetate (10 mL) and aqueous NH₄Cl (10 mL). The organic layer was separated and the aqueous layer was extracted with ethylacetate (2 x 10 mL). The combined organic layer was washed with brine (10 mL), dried (MgSO₄), concentrated, and purified by flash chromatography (silica, 70 → 95% ethylacetate in petroleum ether) to give **7** (64.8 mg, 49%) and **17** (43.9 mg, 57% based on 51% conversion) as an amorphous solid.

**Physical Data for Alcohol 17**. R_{f} = 0.56 (silica, ethylacetate); IR (film) νₘₐₓ 3435, 2956, 2882, 1731, 1671, 1592, 1366, 1280, 1240, 1109, 991, 824, 739 cm⁻¹; ¹H NMR (500 MHz, CDCl₃) δ 9.24 (br s, 1H, pyridine), 8.81 (d, *J*= 1.0, 4.5 Hz, 1H, pyridine), 8.30 (ddd, *J*= 8.0, 2.0, 2.0 Hz, 1H, pyridine), 7.44 (dd, *J* = 8.0, 4.5 Hz, 1H, pyridine), 5.66 (d, *J* = 6.5 Hz, 1H, 2-H), 5.32 (s, 1H, 10-H), 4.92 (dd, *J* = 9.5, 2.0 Hz, 1H, 5-H), 4.38 (dd, *J* = 10.5, 6.5 Hz, 1H, 7-H), 4.32 (br s, 1H, OH), 4.30 (d, *J*= 8.5 Hz, 1H, 20-H), 4.13 (d, *J* = 8.5 Hz, 1H, 20-H), 3.96 (d, *J* = 6.5 Hz, 1H, 3-H), 2.92 (d, *J*= 19.5 Hz, 1H, 14-H), 2.66 (d, *J*= 19.5 Hz, 1H, 14-H), 2.48 (ddd, *J*= 15.5, 9.5, 6.5 Hz, 1H, 6-H), 2.18 (s, 3H, Me), 2.10 (s, 3H, Me), 2.03 (s, 1H, OH), 1.89 (ddd, *J*= 14.5, 10.5, 2.0 Hz, 1H, 6-H), 1.72 (s, 3H, Me), 1.23 (s, 3H, Me), 1.16 (s, 3H, Me), 0.92 (t, *J*= 8.0 Hz, 9H, OSi(CH₂CH₃)₃), 0.62-0.48 (band, 6H, OSi(CH₂CH₃)₃); FAB HRMS (NBA / CSI) *m/e* 790.2030, M + Cs⁺ calcd for C₃₄H₄₇O₁₀NSi 790.2024.

### Preparation of 4-N,N-dimethylaniline-C-2 ester derivative (Alcohol 18)

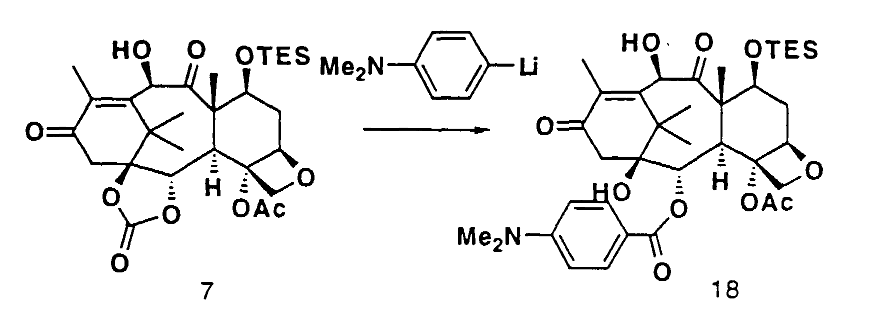

**Alcohol 18.** A solution of carbonate **7** (150 mg, 0.259 mmol) in tetrahydrofuran (20 mL) at -78 °C was treated with 4-lithio-*N,N*-dimethylaniline (6.5 mL of a 0.39 M solution in diethylether : pentane (3 : 1), 2.54 mmol, prepared from 4-bromo-*N,N*-dimethylaniline and t-Butyllithium; methodology from Jones, F.N.; Hauser, C.R. *J. Org. Chem.* **1962***, 27*, 4389) and stirred for 15 minutesutes The reaction mixture was poured into a mixture of CH₂Cl₂ (35 mL) and aqueous NH₄Cl (20 mL), the organic layer was separated, and the aqueous layer was extracted with CH₂Cl₂ (2 x 20 mL). The combined organic layer was washed with brine (20 mL), dried (MgSO₄), concentrated, and purified by flash chromatography (silica, 10 → 35% ethylacetate in petroleum ether) to give 18 (55.0 mg, 30%) as an amorphous solid.

**Physical Data for Alcohol 18**. R_{f} = 0.26 (silica, 35% ethylacetate in hexanes); IR (film) νₘₐₓ 3414, 2924, 1706, 1669, 1605, 1530, 1094; ¹H NMR (500 MHz, CDCl₃) δ 7.90 (d, *J*= 9.0 Hz, 2H, Ar), 6.64 (d, *J* = 9.0 Hz, 2H, Ar), 5.60 (br d, *J* = 7.0 Hz, 1H, 2-H), 5.29 (d, *J* = 2.5 Hz, 1H, 10-H), 4.89 (br d, *J* = 9.5 Hz, 1H, 5-H), 4.37 (d, *J* = 8.5 Hz, 1H, 20-H), 4.36 (dd, *J* = 10.5, 6.5 Hz, 1H, 7-H), 4.31 (d, *J*= 2.5 Hz, 1H, 10-OH), 4.13 (br d, *J*= 8.5 Hz, 1H, 20-H), 3.90 (d, *J* = 7.0 Hz, 1H, 3-H), 3.05 (s, 6H, NMe₂), 2.93 (s, 1H, OH), 2.90 (d, *J* = 20.0 Hz, 1H, 14-H), 2.61 (br d, *J* = 20.0 Hz, 1H, 14-H), 2.49-2.40 (m, 1H, 6-H), 2.16 (s, 3H, Me), 2.08 (s, 3H, Me), 1.90-1.83 (m, 1H, 6-H), 1.69 (s, 3H, Me), 1.20 (s, 3H, Me), 1.13 (s, 3H, Me), 0.90 (t, *J* = 8.0 Hz, 9H, OSi(CH₂CH₃)₃), 0.56-0.49 (band, 6H, OSi(CH₂CH₃)₃); FAB HRMS (NBA / NaI) *m/e* 722.3354, M + Na⁺ calcd for C₃₇H₅₃O₁₀NSi 722.3336.

### Preparation of 1-naphthalene-C-2 ester derivative (Alcohol 19)

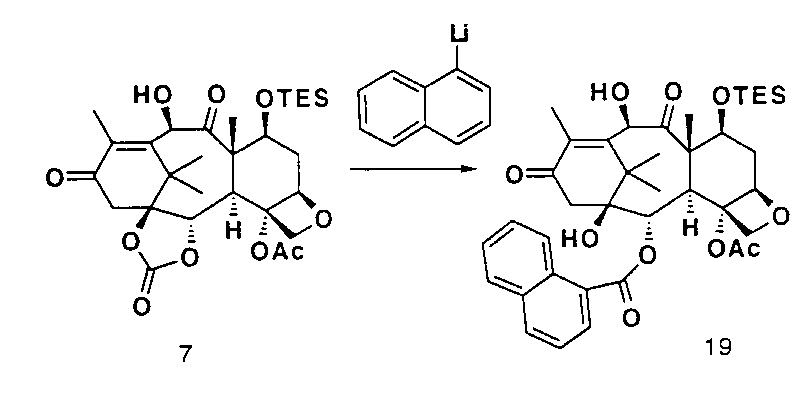

**Alcohol 19**. A solution of carbonate **7** (47 mg, 0.0812 mmol) in tetrahydrofuran (2 mL) at -78 °C was treated with 1-lithionaphthalene (6.3 mL of a 0.32 M solution in diethylether, 2.03 mmol, prepared from 1-bromonaphthalene from Aldrich Chemical Company Inc. and *t*Butyllithium; methodology from Gilman, H.; Moore, F.W. *J. Am. Chem. Soc.* **1940**, *62*, 1843) and stirred for 5 minutesutes The reaction mixture was poured into a mixture of CH₂Cl₂ (15 mL) and aqueous NH₄Cl (20 mL), the organic layer was separated, and the aqueous layer was extracted with CH₂Cl₂ (2 x 10 mL). The combined organic layer was washed with brine (10 mL), dried (MgSO₄) and concentrated to give alcohol 19 which was taken into the next step without further purification.

**Physical Data for Alcohol 19.** R_{f} = 0.27 (20% ethylacetate in petroleum ether); IR (film) νₘₐₓ 3442, 2954, 2882, 1724, 1671, 1461, 1362, 1279, 1228, 1195, 1092, 987, 826, 736 cm⁻¹; ¹H NMR (500 MHz, CDCl₃) δ 8.66 (s, 1H, naphthalene), 8.07 (dd, *J*= 9.0, 2.0 Hz, 1H, naphthalene), 7.97-7.89 (m, 3H, naphthalene), 7.68-7.57 (m, 2H, naphthalene), 5.71 (br d,*J*= 6.5 Hz, 1H, 2-H), 5.35 (d, *J*= 2.5 Hz, 1H, 10-H), 4.94 (br d, *J*= 8.0 Hz, 1H, 5-H), 4.41 (dd, *J*= 11.0, 7.0 Hz, 1H, 7-H), 4.37 (d, *J*= 8.5 Hz, 1H, 20-H), 4.35 (d, *J*= 2.0 Hz, 1H, 10-OH), 4.18 (d, *J* = 8.5 Hz, 1H, 20-H), 4.00 (d, *J*= 6.5 Hz, 1H, 3-H), 3.02 (d, *J* = 19.5 Hz, 1H, 14-H), 2.69 (d, *J* = 19.5 Hz, 1H, 14-H), 2.54-2.45 (m, 1H, 6-H), 2.27 (s, 3H, Me), 2.13 (s, 3H, Me), 1.94-1.87 (m, 1H, 6-H), 1.86 (s, 1H, OH), 1.75 (s, 3H, Me), 1.25 (s, 3H, Me), 1.20 (s, 3H, Me), 0.94 (t, *J* = 8.0 Hz, 9H, OSi(CH₂CH₃)₃), 0.63-0.49 (band, 6H, OSi(CH₂CH₃)₃); FAB HRMS (NBA) *m/e* 707.3270, M + H⁺ calcd for C₃₉H₅₀O₁₀Si 707.3252.

### Preparation of phenylacetylide-C-2 ester derivative (Alcohol 20)

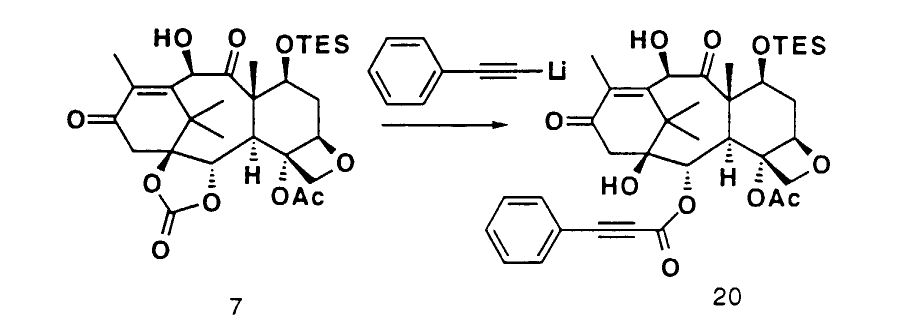

**Alcohol 20**. A solution of carbonate **7** (5.0 mg, 0.00864 mmol) in tetrahydrofuran (0.5 mL) at -78 °C was treated with lithium phenylacetylide from Aldrich Chemical Company Inc. (0.13 mL of a 1.0 M solution in tetrahydrofuran, 0.13 mmol) and stirred for 0.5 hour The reaction mixture was treated with aqueous NH₄Cl (0.5 mL), allowed to warm to 25 °C, and diluted with H₂O (5 mL) and diethylether (5 mL). The organic layer was separated, dried, and concentrated to give a 9 : 1 mixture of carbonate **7** and alcohol **20** (5.0 mg, 95%) as a film.

**Physical Data for Alcohol 20**. R_{f} = 0.59 (50% ethylacetate in hexanes); ¹H NMR (300 MHz, CDCl₃) δ 7.63-7.57 (m, 2 H, Ar), 7.53-7.27 (m, 3 H, Ar), 5.43 (d, *J* = 6.5 Hz, 1H, 2-H), 5.28 (d, *J* = 2.5 Hz, 1H, 10-H), 4.90 (br d, *J* = 7.5 Hz, 1H, 5-H), 4.67 (d, *J* = 8.5 Hz, 1H, 20-H), 4.44 (d, *J* = 8.5 Hz, 1H, 20-H), 4.37-4.30 (m, 1 H, 7-H), 4.28 (d, *J*= 2.5 Hz, 1H, 10-OH), 3.88 (d, *J* = 6.5 Hz, 1H, 3-H), 2.85 (d, *J* = 20.2 Hz, 1H, 14-H), 2.63 (d, *J* = 20.2 Hz, 1H, 14-H), 2.55-2.47 (m, 1 H, 6-H), 2.11 (s, 3 H, OAc), 2.08 (s, 3 H, 18-Me), 1.94-1.85 (m, 1 H, 6-H), 1.67 (s, 3 H, Me), 1.41 (s, 3 H, Me), 1.21 (s, 3 H, Me), 0.91 (t, *J* = 8.0 Hz, 9H, OSi(CH₂CH₃)₃), 0.59-0.42 (band, 6H, OSi(CH₂CH₃)₃).

### Preparation of Hydroxycarbamate-C-2 ester drivative (Alcohol 21)

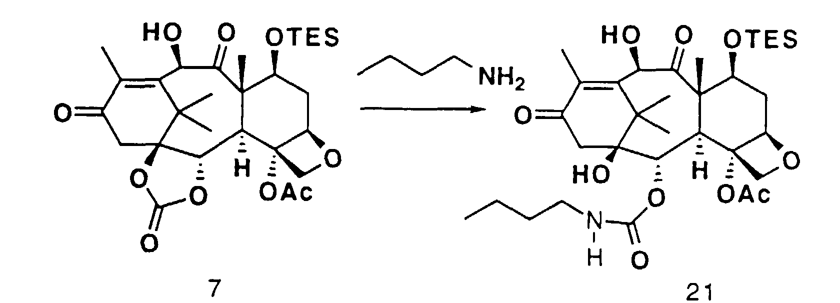

**Alcohol 21**. A solution of carbonate **7** (5 .0 mg, 0.00864 mmol) in MeOH (0.5 mL) at 25 °C was treated with *n*-Butyl-NH₂ from Aldrich Chemical Company Inc. (0.05 mL, 0.506 mmol) and stirred for 10 minutes. The reaction mixture was concentrated and purified by flash chromatography (silica, 30 → 50% ethylacetate in petroleum ether) to give 21 (5.2 mg, 92%) as an amorphous solid.

**Physical Data for Alcohol 21**. R_{f} = 0.13 (silica, 30% ethylacetate in petroleum ether); IR (film) νₘₐₓ 3434, 2957, 2881, 1711, 1671, 1368, 1243, 1108, 987, 829 cm⁻¹; ¹H NMR (500 MHz, CDCl₃) δ 5.27 (d, *J*= 2.0 Hz, 1H, 10-H), 5.23 (d, *J*= 6.5 Hz, 1H, 2-H), 4.91 (br d, *J* = 8.0 Hz, 1H, 5-H), 4.79 (t, *J* = 6.0 Hz, 1H, NH), 4.47 (d, *J* = 8.5 Hz, 1H, 20-H), 4.34 (dd, *J* = 11.0, 7.0 Hz, 1H, 7-H), 4.30 (d, *J* = 2.5 Hz, 1H, 10-OH), 4.28 (d, *J* = 8.5 Hz, 1H, 20-H), 3.78 (d, *J* = 6.5 Hz, 1H, 3-H), 3.29-3.12 (m, 2H, NHCH₂), 2.70 (d, *J*= 20.0 Hz, 1H, 14-H), 2.60 (d, *J*= 20.0 Hz, 1H, 14-H), 2.51-2.42 (m, 1H, 6-H), 2.24 (s, 1H, OH), 2.06 (s, 3H, Me), 2.05 (s, 3H, Me), 1.94-1.86 (m, 1H, 6-H), 1.69 (s, 3H, Me), 1.55-1.46 (m, 2H, NHCH₂CH₂), 1.40-1.30 (m, 2H, NHCH₂CH₂CH₂), 1.21 (s, 3H, Me), 1.09 (s, 3H, Me), 0.95-0.80 (m, 12H, CH₃ of Bu, OSi(CH₂CH₃)₃), 0.61-0.47 (band, 6H, OSi(CH₂CH₃)₃); FAB HRMS (NBA / NaI) *m/e* 674.3336, M + Na⁺ calcd for C₃₃H₅₃O₁₀NSi 674.3336.

### Preparation of NN-methyl-phenyl-hydroxycarbamate-C-2 ester derivative (Alcohol 22)

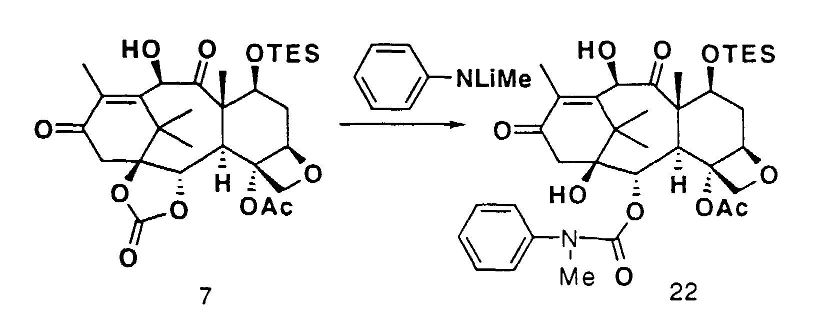

**Alcohol 22**. A solution of carbonate **7** (5.0 mg, 0.00864 mmol) in tetrahydrofuran (0.5 mL) at -78 °C was treated with UNMePh (0.2 mL of a 0.47 M solution in diethylether, 0.094 mmol, prepared from N-methylaniline (Aldrich) and *n*-Butyllithium) and stirred for 1.25 hour The reaction mixture was poured into a mixture of diethylether (5 mL) and aqueous NH₄Cl (5 mL), the organic layer was separated, and the aqueous layer was extracted with diethylether (2 x 5 mL). The combined organic layer was washed with brine (5 mL), dried (MgSO₄), concentrated, and purified by flash chromatography (silica, 15 → 35% ethylacetate in hexanes) to give **7** (2.5 mg, 50%) and 22 (2.8 mg, 93% based on 50% conversion) as an amorphous solid.

**Physical Data for Alcohol 22**. R_{f} = 0.22 (silica 35% ethylacetate in petroleum ether); ¹H NMR (500 MHz, CDCl₃) δ 7.45-7.18 (band, 5H), 5.25 (br d, *J*= 6.5 Hz, 1H, 2-H), 5.20 (d, *J* = 2.5 Hz, 1H, 10-H), 4.70 (br d, *J*= 8.0 Hz, 1H, 5-H), 4.26 (d, *J*= 2.5 Hz, 1H, 10-OH), 4.22 (dd, *J*= 10.5, 6.5 Hz, 1H, 7-H), 4.19 (d, *J* = 8.5 Hz, 1H, 20-H), 4.16 (d, *J* = 8.5 Hz, 1H, 20-H), 3.58 (d, *J*= 7.0 Hz, 1H, 3-H), 3.27 (s, 3H, MeN), 2.52 (d, *J*= 20.0 Hz, 1H, 14-H), 2.35 (d, *J* = 20.0 Hz, 1H, 14-H), 2.40-2.31 (m, 1H, 6-H), 2.03 (s, 1H, OH), 1.97 (s, 3H, Me), 1.85-1.76 (m, 1H, 6-H), 1.66 (s, 3H, Me), 1.57 (s, 3H, Me), 1.18 (s, 3H, Me), 1.08 (s, 3H, Me), 0.87 (t, *J* = 8.0 Hz, 9H, OSi(CH₂CH₃)₃), 0.55-0.43 (band, 6H, OSi(CH₂CH₃)₃); FAB HRMS (NBA) *m/e* 686.3358, M + H⁺ calcd for C₃₆H₅₁O₁₀NSi 686.3361.

### Preparation of Thioether-C-2 ester derivative (Alcohol 23)

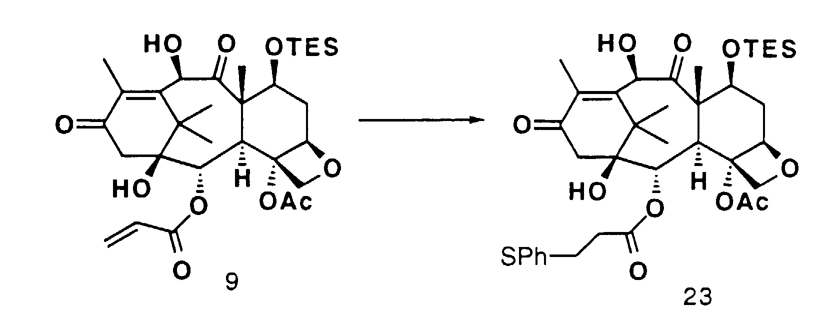

**Alcohol 23.** A solution of vinyl ester **9** (55.6 mg, 0.0916 mmol) and 4-dimethylaminutesopyridine from Aldrich Chemical Company Inc. (DMAP, 1.8 mg, 0.0147 mmol) in CH₂Cl₂ (4.3 mL) at 25 °C was treated with PhSH from Aldrich Chemical Company Inc. (0.030 mL, 0.292 mmol) and stirred for 1.5 hour The reaction mixture was concentrated and purified by flash chromatography (silica, 30% ethylacetate in petroleum ether) to give 23 (58.1, 88%) as a white solid.

**Physical Data for Alcohol 23**. R_{f} = 0.37 (silica, 30% ethylacetate in hexanes), 0.34 (10% ethylacetate in PhH, 2 elutions); IR (film) νₘₐₓ 3441, 3057, 2956, 2883, 1732, 1672, 1600, 1367, 1238, 1111, 988, 825, 739 cm⁻¹ ; ¹H NMR (500 MHz, CDCl₃) δ 7.39-7.24 (band, 5H), 5.44 (d, *J*= 6.5 Hz, 1H, 2-H), 5.28 (d, *J*= 2.5 Hz, 1H, 10-H), 4.90 (dd, *J*= 9.5, 2.0 Hz, 1H, 5-H), 4.38 (d, *J*= 8.0 Hz, 1H, 20-H), 4.33 (dd, *J*= 10.5, 6.5Hz, 1H, 7-H), 4.29 (d, *J*= 2.5 Hz, 1H, 10-OH), 4.18 (d, *J* = 8.0 Hz, 1H, 20-H), 3.83 (d, *J*= 6.5 Hz, 1H, 3-H), 3.24-3.13 (m, 2H, CH₂SPh), 2.76 (d, *J* = 19.5 Hz, 1H, 14-H), 2.72-2.58 (m, 3H, 14-H, CH₂CH₂SPh), 2.47 (ddd, *J*= 14.5, 9.5, 6.5 Hz, 1H, 6-H), 2.39 (s, 1H, OH), 2.07 (s, 3H, Me), 2.05 (s, 3H, Me), 1.89 (ddd, *J*= 14.5, 10.5, 2.0 Hz, 1H, 6-H), 1.68 (s, 3H, Me), 1.23 (s, 3H, Me), 1.12 (s, 3H, Me), 0.92 (t, *J* = 8.0 Hz, 9H, OSi(CH₂CH₃)₃), 0.61-0.47 (band, 6H, OSi(CH₂CH₃)₃); FAB HRMS (NBA / CsI) *m/e* 849.2085, M + Cs⁺ calcd for C₃7H₅₂O₁₀SSi 849.2105.

### Preparation of intermediates 25-27 and 2-furanyl-C-2-taxoid (28)

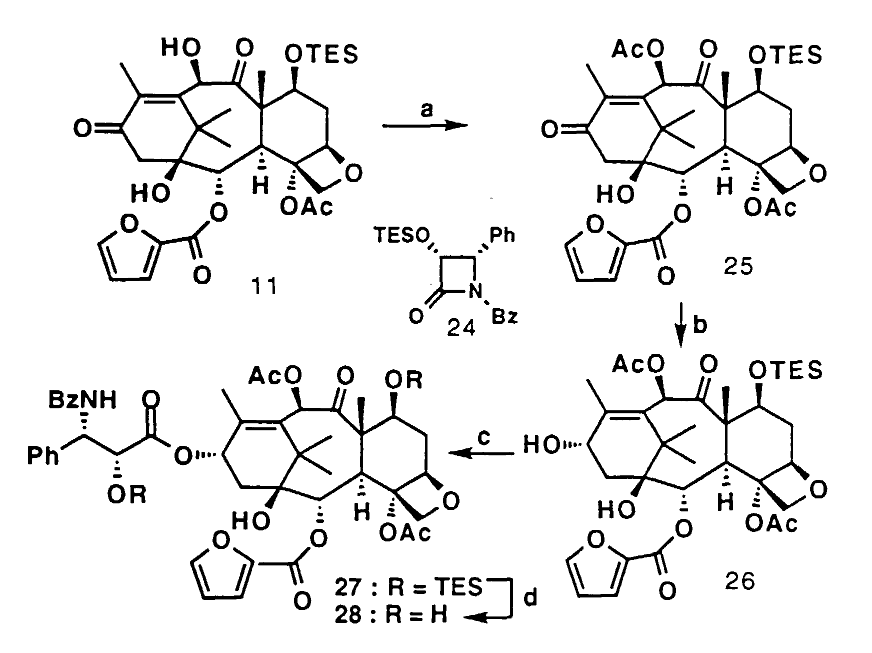

**Acetate 25**. A solution of alcohol **11** and 4-dimethylaminopyridine (DMAP, 100 mg, 0.819 mmol) in CH₂Cl₂ (3 mL) at 25 °C was treated with acetic anhydride (0.50 mL, 5.30 mmol) and stirred for 3 h. The reaction mixture was diluted with CH₂Cl₂ (5 mL), treated with aqueous NaHCO₃ (7 mL), and stirred vigorously for 25 min. The organic layer was separated and the aqueous layer was extracted with CH₂Cl₂ (2 x 10 mL). The combined organic layer was washed with brine (5 mL), dried (MgSO₄), concentrated, and purified by preparative TLC (silica, 10% ethylacetate in benzene, 3 elutions) to give 25 (36 mg, 66% from carbonate 7) as a white foam.

**Physical Data for Acetate 25**. Rf = 0.38 (20% ethylacetate in petroleum ether); IR (film) νₘₐₓ 3509, 2956, 2881, 1727, 1674, 1469, 1371, 1299, 1227, 1108, 746 cm⁻¹; ¹H NMR (500 MHz, CDCl₃) δ 7.65 (br s, 1H, furan), 7.24 ( br d, *J*= 3.0 Hz, 1H, furan), 6.58-6.54 (m, 2H, 10-H, furan), 5.59 (d, *J*= 6.5 Hz, 1H, 2-H), 4.92 (br d, *J* = 7.5 Hz, 1H, 5-H), 4.46 (dd, *J* = 10.5, 6.5 Hz, 1H, 7-H), 4.42 (d, *J* = 8.5 Hz, 1H, 20-H), 4.16 (d, *J* = 8.5 Hz, 1H, 20-H), 3.87 (d, *J*= 6.5 Hz, 1H, 3-H), 2.89 (d, *J*= 20.0 Hz, 1H, 14-H), 2.63 (d, *J*= 20.0 Hz, 1H, 14-H), 2.59-2.48 (m, 1H, 6-H), 2.22 (s, 3H, Me), 2.17 (s, 3H, Me), 2.14 (s, 3H, Me), 1.90-1.83 (m, 1H, 6-H), 1.65 (s, 3H, Me), 1.25 (s, 3H, Me), 1.18 (s, 3H, Me), 0.91 (t, *J*= 8.0 Hz, 9H, OSi(CH₂CH₃)₃), 0.64-0.52 (band, 6H, OSi(CH₂CH₃)₃); FAB HRMS (NBA / CsI) *m/e* 821.1966, M + Cs⁺ calcd for C₃₅H₄₈O₁₂Si 821.1969.

**Alcohol 26**. A solution of enone **25** (36 mg, 0.0523 mmol) in MeOH (3 mL) containing two drops of CH₃COOH at 0 °C was treated with NaBH₄ (200 mg, 5.29 mmol, added by portions) and stirred for 6 h. The reaction mixture was diluted with CH₂Cl₂ (10 mL), treated with aqueous NH₄Cl (5 mL), and stirred for 10 min. The organic layer was separated and the aqueous layer was extracted with CH₂Cl₂ (2 x 10 mL). The combined organic layer was washed with brine (5 mL), dried (MgSO₄), concentrated, and purified by preparative TLC (silica, 50% ethylacetate in petroleum ether) to give **26** (30 mg, 83% ) as an amorphous solid.

**Physical Data for Alcohol 26**. R_{f} = 0.42 (silica, 50% ethylacetate in petroleum ether); ¹H NMR (300 MHz, CDCl₃) δ 7.62 (br s, 1H, furan), 7.25 (d, *J*= 3.5 Hz, 1H, furan), 6.58 (d, *J*= 3.5 Hz, 1H, furan), 6.43 (s, 1 H, 10-H), 5.51 (d, *J*= 7.0 Hz, 1H, 2-H), 4.96 (d, *J*= 7.5 Hz, 1H, 5-H), 4.85-4.79 (m, 1 H, 13-H), 4.48 (dd, *J* = 10.5, 7.5 Hz, 1H, 7-H), 4.38 (d, *J* = 8.0 Hz, 1H, 20-H), 4.15 (d, *J*= 8.0 Hz, 1H, 20-H), 3.82 (d, *J*= 7.0 Hz, 1H, 3-H), 2.61-2.48 (m, 2 H, 6-H and 14-H), 2.28 (s, 3 H, OAc), 2.20-2.10 (m, 1 H, 14-H), 2.18 (s, 6 H, OAc and 18-Me), 1.98-1.80 (m, 1 H, 6-H), 1.18 (s, 3 H, 16-Me), 1.04 (s, 3 H, 17-Me), 0.90 (t, *J*= 8.0 Hz, 9H, OSi(CH₂CH₃)₃), 0.65-0.50 (band, 6H, OSi(CH₂CH₃)₃).

**DiTES taxoid 2**7. To a solution of alcohol **26** (30.0 mg, 0.0434 mmol, previously azeotroped twice with benzene) and β-lactam **24** (28.0 mg, 0.0734 mmol, previously azeotroped twice with benzene) in THF (2 mL), prepared from the Ojima-Holton protocol (Holton, R.A. *Chem Abstr.* **1990**, *114,* 164568q; Ojima, I.; Habus, I.; Zhao, M.; Georg, G. I.; Jayasinghe, L. R. *J. Org. Chem.* **1991**, *56*, 1681-1683; Ojima, I.; Habus, I.; Zhao, M.; Zucco, M.; Park, Y.H.; Sun, C. M.; Brigaud, T. *Tetrahedron* **1992,** *48*, 6985-7012), at 0 °C was added NaN(SiMe₃)₂ (0.130 mL of a 1.0 M solution in THF, 0.130mmol) dropwise. The resulting solution was stirred for 5 min and poured into a mixture of CH₂Cl₂ (10 mL) and aqueous NH₄Cl (5 mL). The organic layer was separated and the aqueous layer was extracted with CH₂Cl₂ (2 x 5 mL). The combined organic layer was washed with brine (5 mL), dried (MgSO₄), concentrated, and purified by preparative TLC (silica, 60% ethylacetate in petroleum ether) to give 27 (12 mg, 26%) as an amorphous solid which was taken directly into the next step.

**Taxoid 28**. A solution of silyl ether **27** (6 mg, 0.00560 mmol) in THF (1 mL) at 25 °C was treated with HF•pyridine (1 mL) and stirred for 1 h. The reaction mixture was poured into a mixture of ethylacetate (10 mL) and aqueous NaHCO₃ (10 mL) and the resulting mixture was stirred for 10 min. The organic layer was separated and the aqueous layer was extracted with ethylacetate (2 x 10 mL). The combined organic layer was washed with brine (5 mL), dried (MgSO₄), concentrated, and purified by preparative TLC (silica, 60% ethylacetate in petroleum ether) to give **28** (3 mg, 64%) as a colorless film.

**Physical Data for Taxoid 28**. R_{f} = 0.1 (50% ethylacetate in petroleum ether); IR (film) νₘₐₓ 3383, 2933, 2898, 1729, 1649, 1519, 1242, 1110, 1071 cm⁻¹; ¹H NMR (500 MHz, CDCl₃) δ 7.77-7.73 (m, 2H), 7.68-7.66 (m, 1H, furan), 7.55-7.33 (band, 9H), 6.98 (d, *J*= 9.0 Hz, 1H, NH), 6.58 (dd, *J*= 3.5, 1.5 Hz, 1H, furan), 6.27-6.21 (m, 2H, 10-H, 13-H), 5.80 (dd, *J*= 9.0, 2.0 Hz, 1H, 3'-H), 5.57 (d, *J*= 7.0 Hz, 1H, 2-H), 4.96 (dd, *J*= 10.0, 2.0 Hz, 1H, 5-H), 4.80 (d, *J*= 2.0 Hz, 1H, 2'-H), 4.43-4.37 (m, 2H, 7-H, 20-H), 4.24 (d, *J*= 8.5 Hz, 1H, 20-H), 3.77 (d, *J*= 7.0 Hz,1 H, 3-H), 2.60-2.52 (m, 1H, 6-H), 2.47 (d, *J*= 4.0 Hz, 1H, OH), 2.38 (s, 3H, Me), 2.35-2.21 (m, 2H, 14-CH₂), 2.25 (s, 3H, Me), 1.94-1.86 (m, 1H, 6-H), 1.81 (br s, 3 H, Me), 1.76 (s, 1H, OH), 1.68 (s, 3H, Me), 1.25 (s, 3H, Me), 1.13 (s, 3H, Me).

### Preparation of 2-thiophenyl-C-2 taxol (32)

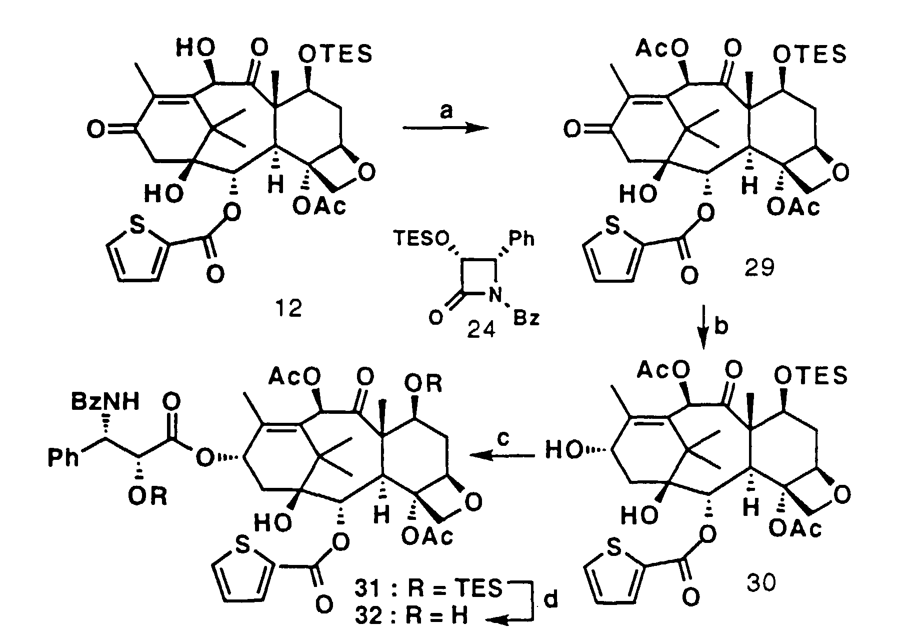

**Acetate 29.** A solution of alcohol **12** (36.0 mg, 0.0543 mmol) and 4-dimethylaminopyridine (DMAP, 33.0 mg, 0.270 mmol) in CH₂Cl₂ (3.0 mL) at 25 °C was treated with acetic anhydride (0.50 mL, 5.30 mmol) and stirred for 1 h. The reaction mixture was diluted with CH₂Cl₂ (10 mL), treated with aqueous NaHCO₃ (7 mL), and stirred vigorously for 0.5 h. The organic layer was separated and the aqueous layer was extracted with CH₂Cl₂ (2 x 10 mL). The combined organic layer was washed with brine (5 mL), dried (MgSO₄), concentrated, and purified by flash chromatography (silica, 10 → 35% ethylacetate in hexanes) to give 29 (29.5 mg, 77%) as an amorphous solid.

**Physical Data for Acetate 29**. R_{f} = 0.56 (silica, 50% ethylacetate in petroleum ether); IR (film) νₘₐₓ 3457, 2956, 1712, 1669, 1525, 1413, 1376, 1264, 1227, 1073; ¹H NMR (500 MHz, CDCl₃) δ 7.84 (dd, *J*= 4.0, 1.5 Hz, 1H, thiophene), 7.63 (dd, *J* = 5.0, 1.5 Hz, 1H, thiophene), 7.13 (dd, *J* = 5.0, 4.0 Hz, 1H, thiophene), 6.56 (s, 1H, 10-H), 5.58 (br d, *J*= 6.5 Hz, 1H, 2-H), 4.90 (br d, *J*= 8.0 Hz, 1H, 5-H), 4.44 (dd, *J*= 10.5, 7.0 Hz, 1H, 7-H), 4.42 (d, *J*= 8.5 Hz, 1H, 20-H), 4.18 (d, *J*= 8.5 Hz, 1H, 20-H), 3.85 (d, *J*= 6.5 Hz, 1H, 3-H), 2.91 (d, *J*= 19.5 Hz, 1H, 14-H), 2.64 (dd, *J*= 19.5, 1.0 Hz, 1H, 14-H), 2.55-2.48 (m, 1H, 6-H), 2.20 (s, 3H, Me), 2.15 (s, 3H, Me), 2.14 (s, 3H, Me), 1.89-1.82 (m, 1H, 6-H), 1.65 (s, 3H, Me), 1.23 (s, 3H, Me), 1.16 (s, 3H, Me), 0.88 (t, *J* = 8.0 Hz, 9H, OSi(CH₂CH₃)₃), 0.59-0.53 (band, 6H, OSi(CH₂CH₃)₃); FAB HRMS (NBA / CsI) *m/e* 837.1736, M + Cs⁺ calcd for C₃₅H₄₈O₁₁SSi 837.1741.

**Alcohol 30**. A solution of enone **29** (29.0 mg, 0.0411 mmol) in MeOH (5 mL) at 0 °C was treated with NaBH₄ (30.2 mg, 0.80 mmol, added by portions) and stirred for 2.5 h. The reaction mixture was diluted with CH₂Cl₂ (15 mL), treated with aqueous NH₄Cl (5 mL), and stirred for 10 min. The organic layer was separated and the aqueous layer was extracted with CH₂Cl₂ (2 x 10 mL). The combined organic layer was washed with brine (5 mL), dried (MgSO₄), concentrated, and purified by flash chromatography (silica, 25 → 50% ethylacetate in petroleum ether) to give 29 (4.0 mg, 14%) and 30 (14.7 mg, 59% based on 86% conversion) as an amorphous solid.

**Physical Data for Alcohol 30.** R_{f} = 0.34 (silica, 50% ethylacetate in petroleum ether); IR (film) νₘₐₓ 3478, 2946, 2892, 1717, 1520, 1365, 1238, 1083; ¹H NMR (500 MHz, CDCl₃) δ 7.85 (dd, *J*= 3.5, 1.5 Hz, 1H, thiophene), 7.61 (dd, *J*= 5.0, 1.5 Hz, 1H, thiophene), 7.12 (dd, *J*= 5.0, 3.5 Hz, 1H, thiophene), 6.43 (s, 1H, 10-H), 5.51 (d, *J*= 7.0 Hz, 1H, 2-H), 4.94 (br d, *J*= 7.5 Hz, 1H, 5-H), 4.83-4.77 (m, 1H, 13-H), 4.45 (dd, *J*= 10.5, 7.5 Hz, 1H, 7-H), 4.41 (d, *J* = 8.0 Hz, 1H, 20-H), 4.19 (br d, *J* = 8.0 Hz, 1H, 20-H), 3.82 (d, *J*= 7.0 Hz, 1H, 3-H), 2.55-2.48 (m, 1H, 6-H), 2.24 (s, 3H, Me), 2.26-2.21. (m, 2H, 14-CH₂), 2.16 (d, *J* = 1.0 Hz, 3H, 18-Me), 2.15 (s, 3H, Me), 2.00 (d, *J*= 5.0 Hz, 1H, OH), 1.90-1.82 (m, 1H, 6-H), 1.66 (s, 3H, Me), 1.58 (s, 1H, OH), 1.15 (s, 3H, Me), 1.02 (s, 3H, Me), 0.90 (t, *J*= 8.0 Hz, 9H, OSiCH₂CH₃)₃), 0.59-0.55 (band, 6H, OSi(CH₂CH₃)₃); FAB HRMS ( NBA / CsI) *m/e* 839.1893, M + Cs⁺ calcd for C₃₅H₅₀O₁₁SSi 839.1897.

**DiTES taxoid 31**. To a solution of alcohol **30** (14.5 mg, 0.0205 mmol, previously azeotroped twice with benzene) and β-lactam **24** (16.0 mg, 0.0420 mmol, previously azeotroped twice with benzene) in THF (1.0 mL), prepared from the Ojima-Holton protocol (Holton, R.A. *Chem Abstr.* **1990**, *114,* 164568q; Ojima, I.; Habus, I.; Zhao, M.; Georg, G. I.; Jayasinghe, L. R. *J. Org. Chem.* **1991**, *56,* 1681-1683; Ojima, I.; Habus, I.; Zhao, M.; Zucco, M.; Park, Y.H.; Sun, C. M.; Brigaud, T. *Tetrahedron* **1992**, *48*, 6985-7012), at -78 °C was added NaN(SiMe₃)₂ (0.051 mL of a 1.0 M solution in THF, 0.051 mmol) dropwise. The resulting solution was stirred for 0.5 h and poured into a mixture of diethylether (10 mL) and aqueous NH₄Cl (5 mL). The organic layer was separated and the aqueous layer was extracted with diethylether (2 x 5 mL). The combined organic layer was washed with brine (5 mL), dried (MgSO₄), concentrated, and purified by flash chromatography (silica, 10 → 35% ethylacetate in hexanes) followed by preparative TLC (silica, 15% ethylacetate in benzene) to give 30 (3.0 mg, 21%) and 31 (7.6 mg, 43% based on 79% conversion) as a white solid.

**Physical Data for DiTES taxoid 31**. R_{f} = 0.48 (silica, 50% ethylacetate in hexanes); IR (film) νₘₐₓ 3382, 2913, 2850, 1722, 1653,1461,1243,1083, 1014; ¹H NMR (500 MHz, CDCl₃) δ 7.90 (br d, *J* = 4.0 Hz, 1H, thiophene), 7.74 (d, *J*= 8.0 Hz, 2H, NBz), 7.62 (br d, *J* =5.0 Hz, 1H, thiophene), 7.48 (t, *J*= 7.0 Hz, 1H, Ar), 7.42-7.28 (band, 7H, Ar), 7.14 (dd, *J*= 5.0, 4.0 Hz, 1H, thiophene), 7.10 (d, *J*= 9.0 Hz, 1H, NH), 6.42 (s, 1H, 10-H), 6.20 (br t, *J*= 9.0 Hz, 1H, 13-H), 5.65 (br d, *J*= 9.0 Hz, 1H, 3'-H), 5.57 (d, *J*=7.0 Hz, 1H, 2-H), 4.94 (br d, *J* = 8.5 Hz, 1H, 5-H), 4.67 (d, *J* = 1.5 Hz, 1H, 2'-H), 4.44 (dd, *J*= 11.0, 6.5 Hz, 1H, 7-H), 4.43 (d, *J* = 8.5 Hz, 1H, 20-H), 4.26 (d, *J* = 8.5 Hz, 1H, 20-H), 3.77 (d, *J* = 7.0 Hz, 1H, 3-H), 2.51 (s, 3H, Me), 2.54-2.47 (m, 1H, 6-H), 2.34 (dd, *J* = 15.0, 9.5 Hz, 1H, 14-H), 2.15 (s, 3H, Me), 2.10 (dd, *J* = 15.0, 9.0, 1H, 14-H), 1.99 (s, 3H, Me), 1.93-1.86 (m, 1H, 6-H), 1.72 (s, 1H, OH), 1.68 (s, 3H, Me), 1.18 (s, 3H, Me), 1.16 (s, 3H, Me), 0.90 (t, *J* = 8.0 Hz, 9H, Si(CH₂CH₃)₃), 0.79 (t, *J*= 8.0 Hz, 9H, Si(CH₂CH₃)₃), 0.57-0.55 (band, 6H, Si(CH₂CH₃)₃), 0.45-0.40 (band, 6H, Si(CH₂CH₃)₃); FAB HRMS (NBA / CsI) *m/e* 1220.3685 M + Cs⁺ calcd for C₅₇H₇₇O₁₄NSSi₂ 1220.3658.

**Taxoid 32.** A solution of silyl ether **31** (7.5 mg, 0.00689 mmol) in THF (0.8 mL) at 25 °C was treated with HF•pyridine (0.150 mL) and stirred for 1 h. The reaction mixture was poured into a mixture of ethylacetate (10 mL) and aqueous NaHCO₃ (5 mL) and the resulting mixture was stirred for 10 min. The organic layer was separated and the aqueous layer was extracted with ethylacetate (2 x 10 mL). The combined organic layer was washed with brine (5 mL), dried (MgSO₄), concentrated, and purified by flash chromatography (silica, 50 → 100% ethylacetate in petroleum ether) to give 32 (4.2 mg, 71%) as a colorless film.

**Physical Data for Taxoid 32.** R_{f} = 0.44 (silica, 75% ethylacetate in petroleum ether); IR (film) νₘₐₓ 3417, 2929, 1716, 1649, 1521, 1460, 1417, 1368, 1247, 1076; ¹H NMR (500 MHz, CDCl₃) δ 7.90 (dd, *J*= 4.0, 1.0 Hz, 1H, thiophene), 7.73 (d, *J* = 7.0 Hz, 2H, NBz), 7.63 (dd, *J* = 5.0, 1.0 Hz, 1H, thiophene), 7.51-7.32 (band, 8H, Ar), 7.14 (dd, *J* = 5.0, 4.0 Hz, 1H, thiophene), 6.96 (d, *J*= 9.0 Hz, 1H, NH), 6.24 (s, 1H, 10-H), 6.19 (br t, *J* = 9.0 Hz, 1H, 13-H), 5.75 (dd, *J* = 9.0, 2.5 Hz, 1H, 3'-H), 5.55 (d, *J* = 7.0 Hz, 1H, 2-H), 4.94 (br d, *J* = 8.0 Hz, 1H, 5-H), 4.76 (dd, *J* = 5.0, 2.5 Hz, 1H, 2'-H), 4.41 (d, *J* = 8.5 Hz, 1H, 20-H), 4.40-4.33 (m, 1H, 7-H), 4.24 (d, *J* = 8.5 Hz, 1H, 20-H), 3.73 (d, *J* = 7.0 Hz, 1H, 3-H), 3.52 (d, *J* = 5.0 Hz, 1H, 2'-OH), 2.58-2.49 (m, 1H, 6-H), 2.44 (d, *J* = 4.0 Hz, 1H, 7-OH), 2.35 (s, 3H, Me), 2.29 (d, *J* = 9.0 Hz, 2H, 14-CH₂), 2.22 (s, 3H, Me), 1.91-1.83 (m, 1H, 6-H), 1.76 (s, 3H, Me), 1.66 (s, 3H, Me), 1.23 (s, 3H, Me), 1.10 (s, 3H, Me); FAB HRMS (NBA / CsI) *m/e* 992.1252, M + Cs⁺ calcd for C₄₅H₄₉NO₁₄S 992.1928.

### Preparation of 3-thiophenyl-C-2 taxol (36)

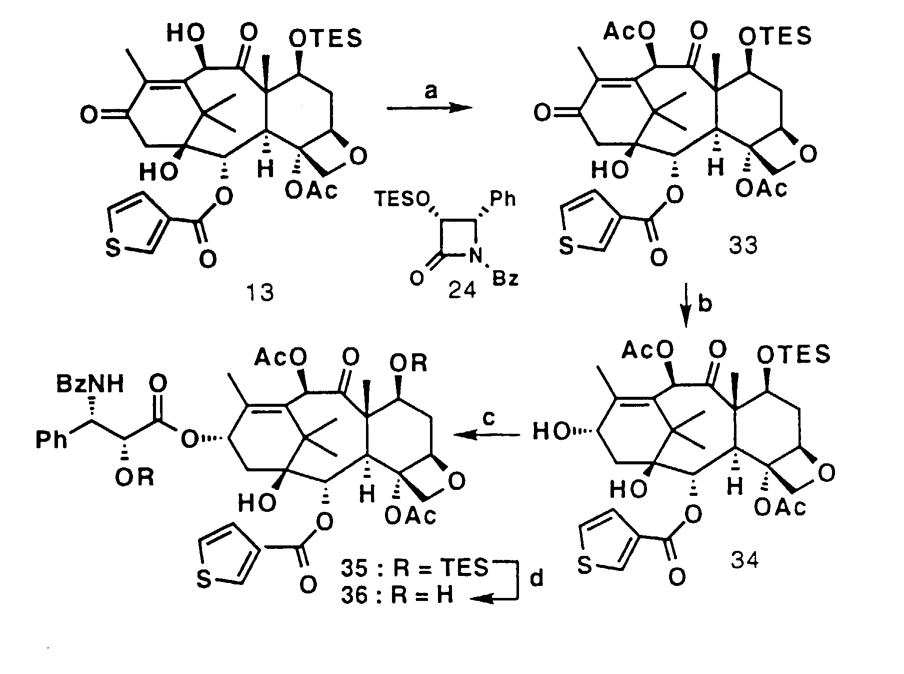

**Acetate 33**. A solution of alcohol **13** (68.4 mg, 0.103 mmol) and 4-dimethylaminopyridine (DMAP, 37.8 mg, 0.309 mmol) in CH₂Cl₂ (4.4 mL) at 25 °C was treated with acetic anhydride (0.370 mL, 3.92 mmol) and stirred for 2 h. The reaction mixture was diluted with CH₂Cl₂ (5 mL), treated with aqueous NaHCO₃ (7 mL), and stirred vigorously for 25 min. The organic layer was separated and the aqueous layer was extracted with CH₂Cl₂ (2 x 10 mL). The combined organic layer was washed with brine (5 mL), dried (MgSO₄), concentrated, and purified by flash chromatography (silica, 30% ethylacetate in hexanes) to give 33 (66.0 mg, 91%) as an amorphous solid.

**Physical Data for Acetate 33**. R_{f} = 0.48 (silica, 10% ethylacetate in benzene, 3 elutions); IR (film) νₘₐₓ 3518, 2956, 2881, 1727, 1676, 1520, 1460, 1371, 1236, 1098, 985, 824, 744 cm⁻¹ ; ¹H NMR (500 MHz, CDCl₃) δ 8.19 (dd, *J*= 3.0, 1.1 Hz, 1H, thiophene), 7.55 (dd, *J*= 5.0, 1.1 Hz, 1H, thiophene), 7.38 (dd, *J* = 5.0, 3.0 Hz, 1H, thiophene), 6.58 (s, 1H, 10-H), 5.61 (dd, *J* = 6.5, 0.7 Hz, 1H, 2-H), 4.92 (dd, *J* = 9.5, 2.0 Hz, 1H, 5-H), 4.47 (dd, *J* = 10.5, 6.5 Hz, 1H, 7-H), 4.38 (d, *J*= 8.5 Hz, 1H, 20-H), 4.14 (d, *J*= 8.5 Hz, 1H, 20-H), 3.88 (d, *J*= 6.5 Hz, 1H, 3-H), 2.89 (d, *J* = 20 Hz, 1H, 14-H), 2.64 (br d, *J* = 20 Hz, 1H, 14-H), 2.54 (ddd, *J* = 14.5, 9.5, 6.5 Hz, 1H, 6-H), 2.23 (s, 3H, Me), 2.18 (s, 3H, Me), 2.17 (s, 3H, Me), 1.87 (ddd, *J*= 14.5, 10.5, 2.0, 1H, 6-H), 1.85 (s, 1H, OH), 1.66 (s, 3H, Me), 1.26 (s, 3H, Me), 1.19 (s, 3H, Me), 0.92 (t, *J* = 8.0 Hz, 9H, OSi(CH₂CH₃)₃), 0.65-0.54 (band, 6H, OSi(CH₂CH₃)₃); FAB HRMS (NBA / CsI) *m/e* 837.1760, M+Cs⁺ calcd for C₃₅H₄₈O₁₁SSi 837.1741.

**Alcohol 34.** A solution of enone **33** (57.3 mg, 0.0813 mmol) in MeOH-THF (5 : 1, 4.1 mL) at 0 °C was treated with NaBH4 (69.1 mg, 1.83 mmol, added by portions) and stirred for 2.5 h. The reaction mixture was diluted with CH₂Cl₂ (10 mL), treated with aqueous NH₄Cl (5 mL), and stirred for 10 min. The organic layer was separated and the aqueous layer was extracted with CH₂Cl₂ (2 x 10 mL). The combined organic layer was washed with brine (5 mL), dried (MgSO₄), concentrated, and purified by flash chromatography (silica, 30% ethylacetate in hexanes) to give 33 (6.8 mg, 12%) and 34 (45.2 mg, 89% based on 88% conversion) as an amorphous solid.

**Physical Data for Alcohol 34**. R_{f} = 0.48 (silica, 50% ethylacetate in hexanes); IR (film) νₘₐₓ 3520, 2953, 2881, 1719, 1520, 1370, 1238, 1100, 979, 823, 746 cm⁻¹; ¹H NMR (500 MHz, CDCl₃) δ 8.20 (dd, *J*= 3.0, 1.0 Hz, 1H, thiophene), 7.57 (dd, *J*= 5.0, 1.0 Hz, 1H, thiophene), 7.35 (dd, *J* = 5.0, 3.0 Hz, 1H, thiophene), 6.45 (s, 1H, 10-H), 5.54 (d, *J*= 7.0 Hz, 1H, 2-H), 4.96 (br d, *J*= 8.5 Hz, 1H, 5-H), 4.82 (br dd, *J*= 12.0, 8.0 Hz, 1H, 13-H), 4.48 (dd, *J* = 10.5, 6.5 Hz, 1H, 7-H), 4.36 (d, *J* = 8.5 Hz, 1H, 20-H), 4.15 (d, *J* = 8.5 Hz, 1H, 20-H), 3.85 (d, *J* = 7.0 Hz, 1H, 3-H), 2.53 (ddd, *J*= 14.5, 9.5, 6.5, 1H, 6-H), 2.27 (s, 3H, Me), 2.28-2.21 (m, 2H, 14-CH₂), 2.18 (s, 6H, Me, Me), 2.03 (s, 1H, OH), 1.87 (ddd, *J*= 14.5, 10.5, 2.0 Hz, 1H, 6-H), 1.67 (s, 3H, Me), 1.65 (s, 1H, OH), 1.18 (s, 3H, Me), 1.04 (s, 3H, Me), 0.92 (t, *J*= 8.0 Hz, 9H, OSi(CH₂CH₃)₃), 0.64-0.50 (band, 6H, OSi(CH₂CH₃)₃); FAB HRMS ( NBA / CsI) *m/e* 839.1908 M + Cs⁺ calcd for C₃₅H₅₀O₁₁SSi 839.1897.

**DiTES taxoid 35.** To a solution of alcohol **34** (19.5 mg, 0.0276 mmol, previously azeotroped twice with benzene) and β-lactam **24** (27.5 mg, 0.0721 mmol, previously azeotroped twice with benzene) in THF (1.4 mL), prepared from the Ojima-Holton protocol (Holton, R.A. *Chem Abstr.* **1990**, *114,* 164568q; Ojima, I.; Habus, I.; Zhao, M.; Georg, G. I.; Jayasinghe, L. R. *J. Org. Chem.* **1991**, *56,* 1681-1683; Ojima, I.; Habus, I.; Zhao, M.; Zucco, M.; Park, Y.H.; Sun, C. M.; Brigaud, T. *Tetrahedron* **1992***, 48*, 6985-7012), at 0 °C was added NaN(SiMe₃)₂ (0.066 mL of a 1.0 M solution in THF, 0.066 mmol) dropwise. The resulting solution was stirred for 0.5 h and poured into a mixture of CH₂Cl₂ (10 mL) and aqueous NH₄Cl (5 mL). The organic layer was separated and the aqueous layer was extracted with CH₂Cl₂ (2 x 5 mL). The combined organic layer was washed with brine (5 mL), dried (MgSO₄), concentrated, and purified by flash chromatography (silica, 20 → 30 % ethylacetate in hexanes) to give 34 (1.1 mg, 6%) and 35 (17.3 mg, 61% based on 94% conversion) as a white solid.

**Physical Data for DiTES taxoid 35**. R_{f} = 0.86 (silica, 50% ethylacetate in hexanes); IR (film) νₘₐₓ 3519, 3437, 2953, 2879, 1726, 1666, 1515, 1483, 1369, 1240, 1100, 979, 825, 746 cm⁻¹; ¹H NMR (500 MHz, CDCl₃) δ 8.32 (dd, *J*= 3.0, 1.2 Hz, 1H, thiophene), 7.76-7.73 (m, 2H), 7.60 (dd, *J* = 5.0, 1.2 Hz, 1H, thiophene), 7.52-7.29 (band, 9H), 7.10 (d, *J*= 9.0 Hz, 1H, NH), 6.44 (s, 1H, 10-H), 6.26 (br t, *J*= 9.0 Hz, 1H, 13-H), 5.72 (dd, *J*= 9.0, 2.0 Hz, 1H,3'-H),5.61 (d, *J* = 7.0 Hz, 1H, 2-H), 4.95 (dd, *J* = 9.5, 2.0 Hz, 1H, 5-H), 4.70 (d, *J* = 2.0 Hz, 1H, 2'-H), 4.48 (dd, *J* = 10.5, 6.5 Hz, 1H, 7-H), 4.37 (d, *J* = 8.5 Hz, 1H, 20-H), 4.23 (d, *J* = 8.5 Hz, 1H, 20-H), 3.81 (d, *J*= 7.0 Hz, 1H, 3-H), 2.56-2.49 (m, 1H, 6-H), 2.54 (s, 3H, Me), 2.35 (dd, *J*= 15.5, 9.0 Hz, 1H, 14-H), 2.17 (s, 3H, Me), 2.07 (dd, *J*= 15.5, 9.0 Hz, 1H, 14-H), 2.03 (d, *J*= 1.0 Hz, 3H, 18-Me), 1.94-1.87 (m, 1H, 6-H), 1.69 (s, 3H, Me), 1.68 (s, 1H, OH), 1.20 (s, 3H, Me), 1.18 (s, 3H, Me), 0.93 (t, *J* = 8.0 Hz, 9H, OSi(CH₂CH₃)₃), 0.81 (t, *J*= 8.0 Hz, 9H, OSi(CH₂CH₃)₃), 0.63-0.53 (band, 6H, OSi(CH₂CH₃)₃), 0.52-0.36 (band, 6H, OSi(CH₂CH₃)₃); FAB HRMS (NBA / CsI) *m/e* 1220.3675, M + Cs⁺ calcd for C₅₇H₇₇O₁₄SSi₂N 1220.3658.

**Taxoid 36.** A solution of silyl ether **35** (17.3 mg, 0.0159 mmol) in THF (0.6 mL) at 25 °C was treated with HF•pyridine (0.150 mL) and stirred for 2 h. The reaction mixture was poured into a mixture of ethylacetate (10 mL) and aqueous NaHCO₃ (5 mL) and the resulting mixture was stirred for 10 min. The organic layer was separated and the aqueous layer was extracted with ethylacetate (2 x 10 mL). The combined organic layer was washed with brine (5 mL), dried (MgSO₄), concentrated, and purified by preparative TLC (silica, 25% ethylacetate in petroleum ether) to give 36 (7.7 mg, 56%) as a colorless film.

**Preparation of Taxoid 36**. R_{f} = 0.11 (silica, 50% ethylacetate in hexanes); IR (film) νₘₐₓ 3496, 3434, 2940, 1723, 1648, 1519, 1370, 1243, 1071, 975 cm⁻¹; ¹H NMR (500 MHz, CDCl₃) δ 8.32 (dd, *J* = 3.0, 1.0 Hz, 1H, thiophene), 7.75-7.72 (m, 2H), 7.60 (dd, *J* = 5.0, 1.0 Hz, 1H, thiophene), 7.53-7.33 (band, 9H), 6.95 (d, *J*= 9.0 Hz, 1H, NH), 6.28-6.23 (m, 2H, 10-H, 13-H), 5.81 (dd, *J* = 9.0, 2.0 Hz, 1H, 3'-H), 5.58 (d, *J* = 7.0 Hz, 1H, 2-H), 4.95 (dd, *J* = 9.5, 2.0 Hz, 1H, 5-H), 4.80 (dd, *J* = 4.5, 2.0 Hz, 1H, 2'-H), 4.41 (br t, *J* = 7.5 Hz, 1H, 7-H), 4.36 (d, *J* = 8.5 Hz, 1H, 20-H), 4.22 (d, *J* = 8.5 Hz, 1H, 20-H), 3.78 (d, *J* = 7.0 Hz, 1H, 3-H), 3.49 (d, *J* = 4.5 Hz, 1H, 2'-OH), 2.55 (ddd, *J* = 14.5, 9.5, 6.5 Hz, 1H, 6-H), 2.45 (br s, 1H, OH), 2.40 (s, 3H, Me), 2.34 (dd, *J* = 15.5, 9.0 Hz, 1H, 14-H), 2.25 (dd, *J* = 15.5, 9.0 Hz, 1H, 14-H), 2.24 (s, 3H, Me), 1.89 (ddd, *J* = 14.5, 11.0, 2.0 Hz, 1H, 6-H), 1.81 (d, *J* = 2.0 Hz, 3H, 18-Me), 1.74 (br s, 1H, OH), 1.67 (s, 3H, Me), 1.24 (s, 3H, Me), 1.13 (s, 3H, Me); FAB HRMS (NBA / CsI) *m/e* 992.1940, M + Cs⁺ calcd for C₄₅H₄₉O₁₄NS 992.1928.

### Preparation of 2-pyridinyl-C-2 taxol (40)

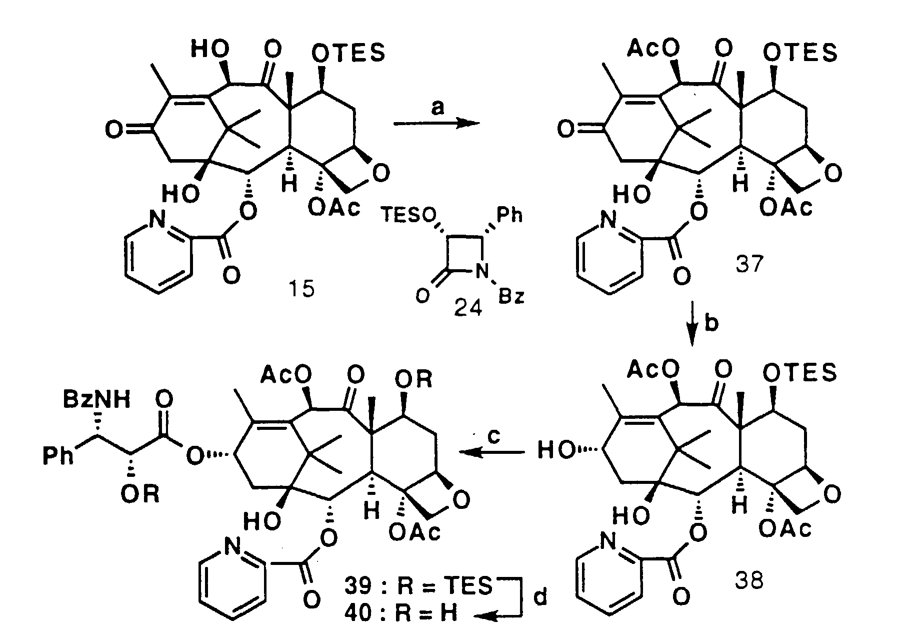

**Acetate 37**. A solution of alcohol **15** (23.2 mg, 0.0353 mmol) and 4-dimethylaminopyridine (DMAP, 12.9 mg, 0.106 mmol) in CH₂Cl₂ (1.5 mL) at 25 °C was treated with acetic anhydride (0.126 mL, 1.34 mmol) and stirred for 2 h. The reaction mixture was diluted with ethylacetate (5 mL), treated with aqueous NaHCO₃ (7 mL), and stirred vigorously for 25 min. The organic layer was separated and the aqueous layer was extracted with ethylacetate (2 x 10 mL). The combined organic layer was washed with brine (5 mL), dried (MgSO₄), concentrated, and purified by flash chromatography (silica, 70 → 100% ethylacetate in petroleum ether) to give **37** (19.0 mg, 77%) as an amorphous solid.

**Preparation of Acetate 37.** R_{f} = 0.58 (silica, ethylacetate); IR (film) νₘₐₓ 3482, 2954, 2881, 1730, 1675, 1370, 1304, 1231, 1118, 987, 823, 739 cm⁻¹; ¹H NMR (500 MHz, CDCl₃) δ 8.77 (ddd, *J* = 4.5, 1.7, 1.0 Hz, 1H, pyridine), 8.05 (ddd, *J* = 8.0, 1.0, 1.0 Hz, 1H, pyridine), 7.89 (ddd, *J* = 8.0, 8.0, 1.7 Hz, 1H, pyridine), 7.53 (ddd, *J* = 8.0, 4.5, 1.0 Hz, 1H, pyridine), 6.59 (s, 1H, 10-H), 5.65 (dd, *J* = 6.6, 1.0 Hz, 1H, 2-H), 4.92 (dd, *J* = 9.5, 2.0 Hz, 1H, 5-H), 4.48 (dd, *J* = 10.5, 7.0 Hz, 1H, 7-H), 4.35 (d, *J* = 8.5 Hz, 1H, 20-H), 4.26 (dd, *J* = 8.5, 1.0 Hz, 1H, 20-H), 3.91 (d, *J* = 6.5 Hz, 1H, 3-H), 3.00 (d, *J* = 20.0 Hz, 1H, 14-H), 2.71 (dd, *J* = 20.0, 1.0 Hz, 1H, 14-H), 2.54 (ddd, *J* = 14.5, 9.5, 7.0 Hz, 1H, 6-H), 2.53 (s, 1H, OH), 2.23 (s, 3H, Me), 2.18 (s, 3H, Me), 2.14 (s, 3H, Me), 1.88 (ddd, *J* = 14.5, 10.5, 2.0 Hz, 1H, 6-H), 1.70 (s, 3H, Me), 1.27 (s, 3H, Me), 1.20 (s, 3H, Me), 0.92 (t, 9H, *J* = 8.0 Hz, OSi(CH₂CH₃)₃), 0.64-0.52 (band, 6H, OSi(CH₂CH₃)₃); FAB HRMS (NBA / CsI) *m/e* 832.2139, M + Cs⁺ calcd for C₃₆H₄₉O₁₁NSi 832.2129.

**Alcohol 38**. A solution of enone **37** (47.6 mg, 0.0680 mmol) in MeOH-THF (5 : 1, 3.8 mL) at 0 °C was treated with NaBH₄ (46.0 mg, 1.22 mmol, added by portions) and stirred for 1.5 h. The reaction mixture was diluted with ethylacetate (10 mL), treated with aqueous NH₄Cl (5 mL), and stirred for 10 min. The organic layer was separated and the aqueous layer was extracted with ethylacetate (2 x 10 mL). The combined organic layer was washed with brine (5 mL), dried (MgSO₄), concentrated, and purified by flash chromatography (basic alumina, ethylacetate → 10% MeOH in ethylacetate) to give **27f** (28.0 mg, 59%) as an amorphous solid.

**Physical Data for Alcohol 38.** R_{f} = 0.36 (silica, ethylacetate); IR (film) νₘₐₓ 3487, 2951, 2880, 1736, 1583, 1369, 1307, 1236, 1132, 983, 824, 739 cm⁻¹; ¹H NMR (500 MHz, CDCl₃) δ 8.79 (dm, *J* = 4.5 Hz, 1H, pyridine), 8.13 (brd, *J* = 7.5 Hz, 1H, pyridine), 7.88 (ddd, *J* = 7.5, 7.5, 1.7 Hz, 1H, pyridine), 7.51 (ddd, *J* = 7.5,4.5, 1.0 Hz, 1H, pyridine), 6.46 (s, 1H, 10-H), 5.64 (d, *J* = 7.0 Hz, 1H, 2-H), 4.96 (dd, *J* = 9.5, 2.0 Hz, 1H, 5-H), 4.85 (br t, *J* = 8.0 Hz, 1H, 13-H), 4.49 (dd, *J* = 10.5, 6.5 Hz, 1H, 7-H), 4.31 (d, *J* = 8.0 Hz, 1H, 20-H), 4.25 (d, *J* = 8.0 Hz, 1H, 20-H), 3.89 (d, *J* = 7.0 Hz, 1H, 3-H), 2.53 (ddd, *J* = 14.5, 9.5, 6.5 Hz, 1H, 6-H), 2.36-2.11 (m, 2H, 14-CH₂), 2.25 (s, 3H, Me), 2.19 (d, *J* = 1.0 Hz, 3H, 18-Me), 2.18 (s, 3H, Me), 1.88 (ddd, *J* = 14.0, 10.5, 2.5 Hz, 1H, 6-H), 1.70 (s, 3H, Me), 1.20 (s, 3H, Me), 1.05 (s, 3H, Me), 0.92 (t, *J* = 8.0 Hz, 9H, OSi(CH₂CH₃)₃), 0.65-0.51 (band, 6H, OSi(CH₂CH₃)₃); FAB HRMS (NBA / CsI) *m/e* 834.2311, M + Cs⁺ calcd for C₃₆H₅₁O₁₁NSi 834.2286.

**DiTES taxoid 39**. To a solution of alcohol **38** (10.3 mg, 0.0147 mmol, previously azeotroped twice with benzene) and β-lactam **24** (17.0 mg, 0.0446 mmol, previously azeotroped twice with benzene) in THF (0.75 mL) at 0 °C, prepared from the Ojima-Holton protocol (Holton, R.A. *Chem Abstr.* **1990**, *114,* 164568q; Ojima, I.; Habus, I.; Zhao, M.; Georg, G. I.; Jayasinghe, L. R. *J. Org. Chem.* **1991**, *56* , 1681-1683; Ojima, I.; Habus, I.; Zhao, M.; Zucco, M.; Park, Y.H.; Sun, C. M.; Brigaud, T. *Tetrahedron* **1992**, *48,* 6985-7012), was added NaN(SiMe₃)₂ (0.038 mL of a 1.0 M solution in THF, 0.038 mmol) dropwise. The resulting solution was stirred for 20 min and poured into a mixture of ethylacetate (10 mL) and aqueous NH₄Cl (5 mL). The organic layer was separated and the aqueous layer was extracted with ethylacetate (2 x 5 mL). The combined organic layer was washed with brine (5 mL), dried (MgSO₄), concentrated, and purified by preparative TLC (silica, 60% ethylacetate in petroleum ether) to give 39 (2.7 mg, 17%) as a film.

**Physical Data for DiTES taxoid 39**. R_{f} = 0.28 (silica, 50% ethylacetate in hexane); IR (film) νₘₐₓ 3429, 2952, 2927, 2878, 1728, 1662, 1585, 1369, 1236, 1124, 1016, 984, 742 cm⁻¹; ¹H NMR (500 MHz, CDCl₃) δ 8.78 (br d, *J* = 4.5 Hz, 1H, pyridine), 8.21 (d, *J* = 8.0 Hz, 1H, pyridine), 7.95 (ddd, *J* = 8.0, 8.0, 1.7 Hz, 1H, pyridine), 7.75-7.70 (m, 2H), 7.54-7.22 (band, 9H), 7.12 (d, *J* = 9.0 Hz, 1H, NH), 6.45 (s, 1H, 10-H), 6.27 (br t, *J* = 9.0 Hz, 1H, 13-H), 5.73-5.67 (m, 2H, 2-H, 3'-H), 4.95 (dd, *J* = 9.5, 2.0 Hz, 1H, 5-H), 4.70 (d, *J* = 2.0 Hz, 1H, 2'-H), 4.48 (dd, *J* = 10.5, 6.5, 1H, 7-H), 4.32 (br s, 2H, 20-CH₂), 3.85 (d, *J*= 7.0 Hz, 1H, 3-H), 2.56-2.48 (m, 1H, 6-H), 2.52 (s, 3H, Me), 2.40 (dd, *J* = 15.0 Hz, 9.5 Hz, 1H, 14-H), 2.20-2.12 (m, 2H, 14-H, OH), 2.18 (s, 3H, Me), 2.04 (s, 3H, Me), 1.92 (ddd, *J* = 14.5, 10.5, 2.0 Hz, 1H, 6-H), 1.72 (s, 3H, Me), 1.22 (s, 3H, Me), 1.19 (s, 3H, Me), 0.93 (t, *J* = 8.0 Hz, 9H, OSi(CH₂CH₃)₃), 0.81 (t, *J* = 8.0 Hz, 9H, OSi(CH₂CH₃)₃), 0.64-0.34 (band, 12H, OSi(CH₂CH₃)₃); FAB HRMS (NBA / CsI) *m/e* 1215.4065, M + Cs⁺ calcd for C₅₈H₇₈O₁₄N₂Si₂ 1215.4046.

**Taxoid 40**. A solution of silyl ether **39** (2.7 mg, 0.00249 mmol) in THF (0.4 mL) at 25 °C was treated with HF•pyridine (0.170 mL) and stirred for 3 h. The reaction mixture was poured into a mixture of ethylacetate (10 mL) and aqueous NaHCO₃ (5 mL) and the resulting mixture was stirred for 10 min. The organic layer was separated and the aqueous layer was extracted with ethylacetate (2 x 10 mL). The combined organic layer was washed with brine (5 mL), dried (MgSO₄), concentrated, and purified by preparative TLC (silica, ethylacetate) to give 40 (0.8 mg, 38%) as a colorless film.

**Physical Data for Taxoid 40**. Rf = 0.54 (silica, ethylacetate); ¹H NMR (500 MHz, CDCl₃) δ 8.80 (br d, *J* = 4.5 Hz, 1H, pyridine), 8.22 (d, *J* = 7.5 Hz, 1H, pyridine), 7.93 (ddd, *J* = 7.5, 7.5, 1.5 Hz, 1H, pyridine), 7.75-7.71 (m, 2H), 7.54-7.30 (band, 9H), 6.98 (d, *J* = 9.0 Hz, 1H, NH), 6.30-6.24 (m, 2H, 10-H, 13-H), 5.82 (dd, *J* = 9.0, 2.5 Hz, 1H, 3'-H), 5.67 (d, *J* = 7.0 Hz, 1H, 2-H), 4.95 (dd, *J* = 10.0, 2.0 Hz, 1H, 5-H), 4.81 (dd, *J* = 4.5, 2.5 Hz, 1H, 2'-H), 4.41 (ddd, *J* = 11.0, 7.0, 4.5 Hz, 1H, 7-H), 4.31 (s, 2H, 20-CH₂), 3.81 (d, *J* = 7.0 Hz, 1H, 3-H), 3.52 (br s, 1H, OH), 3.50 (d, *J* = 4.5 Hz, 1H, 2'-OH), 2.56 (ddd, *J* = 14.5, 9.5, 7.0 Hz, 1H, 6-H), 2.46 (d, *J* = 4.0 Hz, 1H, 7-OH), 2.43-2.30 (m, 2H, 14-CH₂), 2.38 (s, 3H, OAc), 2.25 (s, 3H, OAc), 1.90 (ddd, *J* = 14.5, 11.0, 2.0 Hz, 1H, 6-H), 1.81 (s, 3H, Me), 1.71 (s, 3H, Me), 1.26 (s, 3H, Me), 1.15 (s, 3H, Me).

### Prepartion of 3-pyridinyl-C-2-taxol (44)

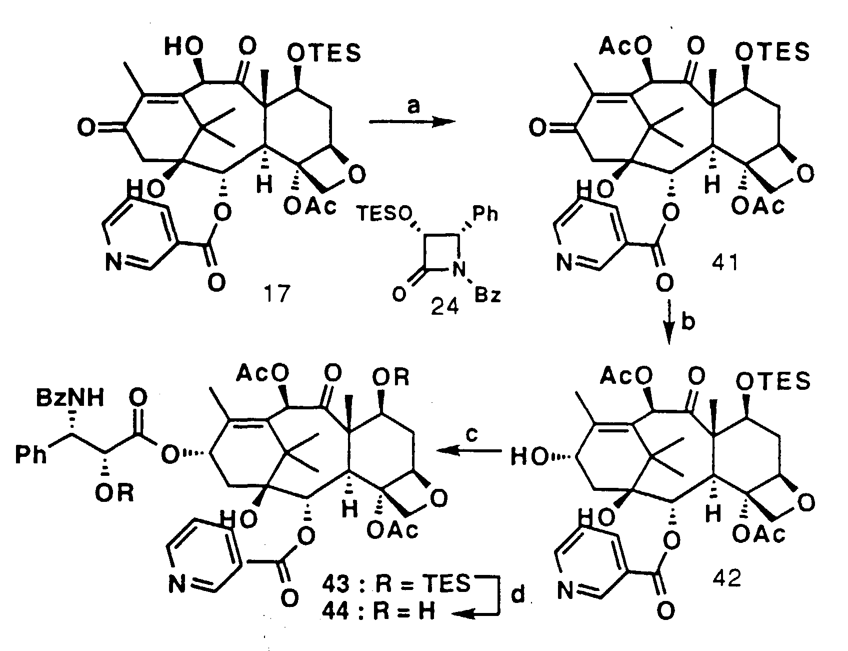

**Acetate 41**. A solution of alcohol **17** (42.9 mg, 0.0652 mmol) and 4-dimethylaminopyridine (DMAP, 23.9 mg, 0.196 mmol) in CH₂Cl₂ (2.8 mL) at 25 °C was treated with acetic anhydride (0.235 mL, 2.49 mmol) and stirred for 2 h. The reaction mixture was diluted with ethylacetate (5 mL), treated with aqueous NaHCO₃ (7 mL), and stirred vigorously for 25 min. The organic layer was separated and the aqueous layer was extracted with ethylacetate (2 x 10 mL). The combined organic layer was washed with brine (5 mL), dried (MgSO₄), concentrated, and purified by flash chromatography (silica, ethylacetate) to give 41 (43.5 mg, 95%) as a white solid.

**Physical Data for Acetate 41**. R_{f} = 0.61 (silica, ethylacetate); IR (film) νₘₐₓ 3470, 3327, 2955, 2881, 1731, 1675, 1592, 1370, 1279, 1229, 1108, 822, 738 cm⁻¹; ¹H NMR (500 MHz, CDCl₃) δ 9.23 (br s, 1H, pyridine), 8.79 (br s, 1H, pyridine), 8.30 (ddd, *J* = 8.0, 2.0, 2.0 Hz, 1H, pyridine), 7.43 (dd, *J* = 8.0, 5.0 Hz, 1H, pyridine), 6.58 (s, 1H, 10-H), 5.70 (dd, *J* = 6.5, 1.0 Hz, 1H, 2-H), 4.91 (dd, *J*= 9.5, 2.0 Hz, 1H, 5-H), 4.47 (dd, *J* = 10.5, 7.0 Hz, 1H, 7-H), 4.28 (d, *J* = 8.0 Hz, 1H, 20-H), 4.11 (d, *J* = 8.0 Hz, 1H, 20-H), 3.91 (d, *J* = 6.5 Hz, 1H, 3-H), 2.93 (d, *J* = 20.0 Hz, 1H, 14-H), 2.68 (dd, *J*= 20.0, 1.0 Hz, 1H, 14-H), 2.53 (ddd, *J* = 14.5, 9.5, 7.0 Hz, 1H, 6-H), 2.24 (br s, 1H, OH), 2.22 (s, 3H, Me), 2.18 (s, 3H, Me), 2.17 (s, 3H, Me), 1.85 (ddd, *J* = 14.5, 10.5, 2.0 Hz, 1H, 6-H), 1.66 (s, 3H, Me), 1.26 (s, 3H, Me), 1.18 (s, 3H, Me), 0.90 (t, *J* = 8.0 Hz, 9H, OSi(CH₂CH₃)₃), 0.63-0.51 (band, 6H, OSi(CH₂CH₃)₃); FAB HRMS (NBA / CsI) *m/e* 832.2145, M + Cs⁺ calcd for C₃₆H₄O₁₁NSi 832.2129.

**Alcohol 42**. A solution of enone **41** (39.8 mg, 0.0569 mmol) in MeOH-THF (5 : 1, 3.1 mL) at 0 °C was treated with NaBH4 (65.0 mg, 1.72 mmol, added by portions) and stirred for 1.5 h. The reaction mixture was diluted with ethylacetate (10 mL), treated with aqueous NH₄Cl (5 mL), and stirred for 10 min. The organic layer was separated and the aqueous layer was extracted with ethylacetate (2 x 10 mL). The combined organic layer was washed with brine (5 mL), dried (MgSO₄), concentrated, and purified by flash chromatography (silica, ethylacetate) to give **41** (3.7 mg, 9%) and **42** (24.3 mg, 67% based on 91% conversion) as an amorphous solid.

**Physical Data for Alcohol 42.** R_{f} = 0.42 (silica, ethylacetate); IR (film) νₘₐₓ 3490, 2953, 2881, 1727, 1592, 1369, 1235, 1110, 822, 740 cm⁻¹; ¹H NMR (500 MHz, CDCl₃) δ 9.30 (d, *J* = 2.0 Hz, 1H, pyridine), 8.81 (dd, *J* = 5.0, 2.0 Hz, 1H, pyridine), 8.35 (ddd, *J* = 8.0, 2.0, 2.0 Hz, 1H, pyridine), 7.44 (dd, *J* = 8.0, 5.0 Hz, 1H, pyridine), 6.46 (s, 1H, 10-H), 5.64 (d, *J* = 7.0 Hz, 1H, 2-H), 4.96 (dd, *J* = 9.5, 1.5 Hz, 1H, 5-H), 4.83 (br dd, *J* = 12.5, 7.5 Hz, 1H, 13-H), 4.49 (dd, *J* = 10.5, 6.5 Hz, 1H, 7-H), 4.28 (d, *J* = 8.0 Hz, 1H, 20-H), 4.15 (d, *J* = 8.0 Hz, 1H, 20-H), 3.89 (d, *J* = 7.0 Hz, 1H, 3-H), 2.53 (ddd, *J* = 14.5, 9.5, 6.5 Hz, 1H, 6-H), 2.30-2.20 (m, 2H, 14-CH₂), 2.28 (s, 3H, Me), 2.19 (d, *J* = 1.0 Hz, 3H, 18-Me), 2.18 (s, 3H, Me), 1.87 (ddd, *J* = 14.5, 10.5, 2.0 Hz, 1H, 6-H), 1.68 (s, 3H, Me), 1.63 (br s, 2H, OH, OH), 1.19 (s, 3H, Me), 1.04 (s, 3H, Me), 0.92 (t, *J* = 8.0 Hz, 9H, OSi(CH₂CH₃)₃), 0.64-0.51 (band, 6H, OSi(CH₂CH₃)₃); FAB HRMS (NBA / CsI) *m/e* 834.2270, M + Cs⁺ calcd for C₃₆H₅₁O₁₁NSi 834.2286.

**DiTES taxoid 43**. To a solution of alcohol **42** (12.6 mg, 0.018 mmol, previously azeotroped twice with benzene) and β-lactam **24** (17.0 mg, 0.0446 mmol, previously azeotroped twice with benzene) in THF (0.97 mL) at 0 °C, prepared from the Ojima-Holton protocol (Holton, R.A. *Chem Abstr.* **1990**, *114,* 164568q; Ojima, I.; Habus, I.; Zhao, M.; Georg, G. I.; Jayasinghe, L. R. *J. Org. Chem.* **1991**, *56,* 1681-1683; Ojima, I.; Habus, I.; Zhao, M.; Zucco, M.; Park, Y.H.; Sun, C. M.; Brigaud, T. *Tetrahedron* **1992**, *48,* 6985-7012), was added NaN(SiMe₃)₂ (0.054 mL of a 1.0 M solution in THF, 0.054 mmol) dropwise. The resulting solution was stirred for 0.5 h and poured into a mixture of ethylacetate (10 mL) and aqueous NH₄Cl (5 mL). The organic layer was separated and the aqueous layer was extracted with ethylacetate (2 x 5 mL). The combined organic layer was washed with brine (5 mL), dried (MgSO₄), concentrated, and purified by flash chromatography (silica, 50 → 95% ethylacetate in hexanes) to give 42 (1.0 mg, 8%) and 43 (8.6 mg, 48% based on 92% conversion) as a white solid.

**Physical Data for DiTES taxoid 43.** R_{f} = 0.40 (silica, 50% ethylacetate in hexanes); IR (film) νₘₐₓ 3433, 2955, 2880, 1730, 1662, 1370, 1238, 1112, 1018, 985, 824, 740 cm⁻¹; ¹H NMR (500 MHz, CDCl₃) δ 9.34 (d, *J* = 2.0 Hz, 1H, pyridine), 8.82 (dd, *J* = 5.0, 2.0 Hz, 1H, pyridine), 8.42 (ddd, *J* = 8.0, 2.0, 2.0 Hz, 1H, pyridine), 7.74-7.69 (m, 2H), 7.51-7.20 (band, 9H), 7.08 (d, *J* = 9.0 Hz, 1H, NH), 6.46 (s, 1H, 10-H), 6.22 (br t, *J* = 9.0 Hz, 1H, 13-H), 5.74-5.66 (m, 2H, 2-H, 3'-H), 4.95 (dd,*J* = 9.5, 2.0 Hz, 1H, 5-H), 4.70 (d, *J* = 2.0 Hz, 1H, 2'-H), 4.48 (dd, *J* = 10.5,6.5 Hz, 1H, 7-H), 4.30 (d, *J* = 8.0 Hz, 1H, 20-H), 4.21 (d, *J* = 8.0 Hz, 1H, 20-H), 3.86 (d, *J* = 7.0 Hz, 1H, 3-H), 2.58-2.48 (m, 1H, 6-H), 2.54 (s, 3H, Me), 2.40 (dd, *J* = 15.5, 9.0 Hz, 1H, 14-H), 2.17 (s, 3H, Me), 2.14 (dd, *J* = 15.5, 9.0 Hz, 1H, 14-H), 2.03 (br s, 3H, Me), 1.95-1.86 (m, 1H, 6-H), 1.73 (s, 4H, Me, OH), 1.22 (s, 3H, Me), 1.18 (s, 3H, Me), 0.93 (t, *J* = 8.0 Hz, 9H, OSi(CH₂CH₃)₃), 0.82 (t, *J* = 8.0 Hz, 9H, OSi(CH₂CH₃)₃), 0.65-0.37 (band, 12H, OSi(CH₂CH₃)₃, OSi(CH₂CH₃)₃); FAB HRMS (NBA / CsI) *m/e* 1215.4066, M + Cs⁺ calcd for C₅₈H₇₈O₁₄N₂Si₂ 1215.4046.

**Taxoid 44.** A solution of silyl ether **43** (6.4 mg, 0.0059 mmol) in THF (0.4 mL) at 25 °C was treated with HF•pyridine (0.160 mL) and stirred for 1.25 h. The reaction mixture was poured into a mixture of ethylacetate (10 mL) and aqueous NaHCO₃ (5 mL) and the resulting mixture was stirred for 10 min. The organic layer was separated and the aqueous layer was extracted with ethylacetate (2 x 10 mL). The combined organic layer was washed with brine (5 mL), dried (MgSO₄), concentrated, and purified by preparative TLC (silica, ethylacetate) to give 44 (3.8 mg, 75%) as a colorless film.

**Physical Data for Taxoid 44**. R_{f} = 0.59 (silica, ethylacetate); IR (film) νₘₐₓ 3396, 2928, 1728, 1644, 1371, 1273, 1241, 1111, 1071 cm⁻¹; ¹H NMR (500 MHz, CDCl₃) δ 9.34 (br s, 1H, pyridine), 8.83 (br d, *J* = 3.5 Hz, 1H, pyridine), 8.41 (br d,*J* = 8.0 Hz, 1H, pyridine), 7.75-7.68 (m, 2H), 7.53-7.34 (band, 9H), 6.91 (d, *J* = 9.0 Hz, 1H, NH), 6.27 (s, 1H, 10-H), 6.23 (br t, *J* = 9.0 Hz, 1H, 13-H), 5.78 (dd, *J* = 9.0, 2.5 Hz, 1H, 3'-H), 5.69 (d, *J* = 7.0 Hz, 1H, 2-H), 4.95 (dd, *J* = 9.5, 2.0 Hz, 1H, 5-H), 4.79 (dd, *J* = 5.5, 2.5 Hz, 1H, 2'-H), 4.41 (ddd, *J* = 11.0, 6.5, 4.0 Hz, 1H, 7-H), 4.29 (d, *J* = 8.5 Hz, 1H, 20-H), 4.20 (d, *J* = 8.5 Hz, 1H, 20-H), 3.82 (d, *J* = 7,0 Hz, 1H, 3-H), 3.54 (d, *J* = 5.5 Hz, 1H, 2'-OH), 2.56 (ddd, *J* = 14.5, 9.5, 6.5 Hz, 1H, 6-H), 2.49 (d, *J* = 4.0 Hz, 1H, 7-OH), 2.43-2.26 (m, 2H, 14-CH₂), 2.38 (s, 3H, Me), 2.24 (s, 3H, Me), 1.89 (ddd, *J* = 14.5, 11.0, 2.0 Hz, 1H, 6-H), 1.83 (s, 1H, OH), 1.82 (s, 3H, Me), 1.69 (s, 3H, Me), 1.25 (s, 3H, Me), 1.14 (s, 3H, Me); FAB HRMS (NBA / CsI) *m/e* 987.2325, M + Cs⁺ calcd for C₄₆H₅₀O₁₄N₂ 987.2316.

### Preparation of 4-N,N-dbnethylaniline-C-2 taxol (48)

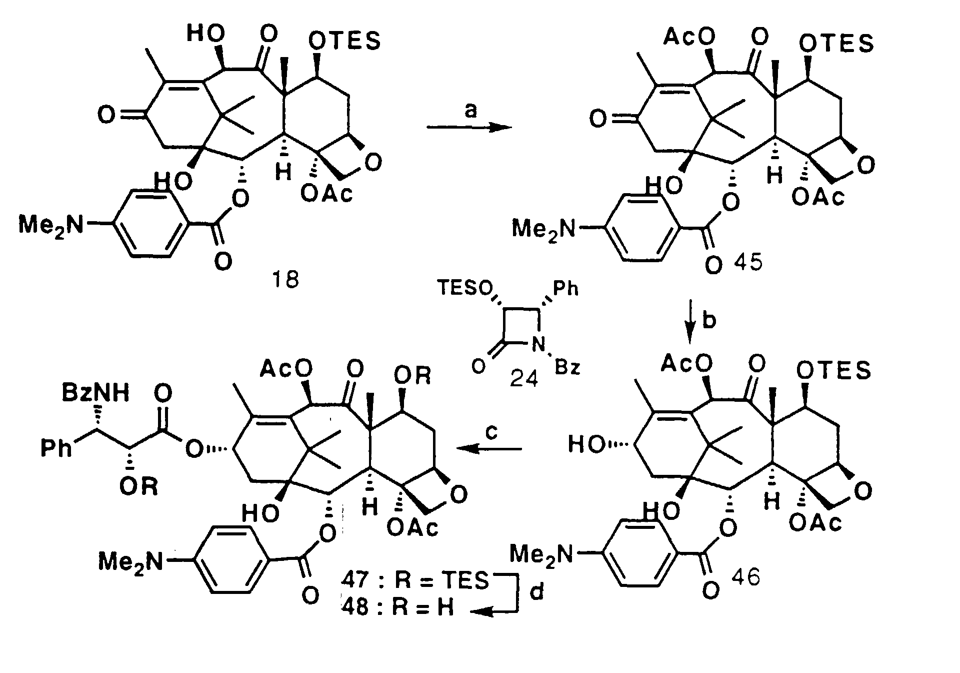

**Acetate 45.** A solution of alcohol **18** (50.0 mg, 0.0714 mmol) and 4-dimethylaminopyridine (DMAP, 26.0 mg, 0.213 mmol) in CH₂Cl₂ (3.0 mL) at 25 °C was treated with acetic anhydride (0.250 mL, 2.65 mmol) and stirred for 2.5 h. The reaction mixture was diluted with CH₂Cl₂ (10 mL), treated with aqueous NaHCO₃ (7 mL), and stirred vigorously for 25 min. The organic layer was separated and the aqueous layer was extracted with CH₂Cl₂ (2 x 10 mL). The combined organic layer was washed with brine (5 mL), dried (MgSO₄), concentrated, and purified by flash chromatography (silica, 10% ethylacetate in benzene) to give 45 (41.0 mg, 77%) as an amorphous solid.

**Physical Data for Acetate 45.** R_{f} = 0.27 (silica, 35% ethylacetate in hexanes); IR (film) νₘₐₓ 3425, 2945, 1722, 1674, 1605, 1365, 1275, 1232, 1179, 1094; ¹H NMR (500 MHz, CDCl₃) δ 7.89 (d, *J* = 9.0 Hz, 2H, Ar), 6.64 (d, *J* = 9.0 Hz, 2H, Ar), 6.56 (s, 1H, 10-H), 5.64 (d,*J* = 6.5 Hz, 1H, 2-H), 4.90 (brd, *J* = 8.0 Hz, 1H, 5-H), 4.45 (dd, *J* = 10.5, 7.0 Hz, 1H, 7-H), 4.36 (d, *J* = 9.0 Hz, 1H, 20-H), 4.11 (d, *J* = 9.0 Hz, 1H, 20-H), 3.85 (d, *J* = 6.5 Hz, 1H, 3-H), 3.05 (s, 6H, NMe₂), 2.90 (d, *J*= 20.0 Hz, 1H, 14-H), 2.62 (d, *J*= 20.0 Hz, 1H, 14-H), 2.51 (ddd, *J*= 14.0, 8.0, 7.0, 1H, 6-H), 2.20 (s, 3H, Me), 2.16 (s, 3H, Me), 2.15 (s, 3H, Me), 2.04 (s, 1H, OH), 1.84 (ddd, *J*= 14.0, 10.5, 2.0 Hz, 1H, 6-H), 1.63 (s, 3H, Me), 1.23 (s, 3H, Me), 1.16 (s, 3H, Me), 0.89 (t, *J*= 8.0 Hz, 9H, OSi(CH₂CH₃)₃), 0.58-0.53 (band, 6H, OSi(CH₂CH₃)₃); FAB HRMS (NBA / CsI) *m/e* 874.8589, M + Cs⁺ calcd for C₃₉H₅₅O₁₁NSi 874.8594.

**Alcohol 46**. A solution of enone **45** (40.0 mg, 0.0539 mmol) in MeOH-THF (5.8 : 1, 4.1 mL) at 0 °C was treated with NaBH₄ (30.2 mg, 0.80 mmol, added by portions), stirred for 1 h, allowed to warm to 25 °C and stirred for 1.5 h. The reaction mixture was diluted with CH₂Cl₂ (15 mL), treated with aqueous NH₄Cl (5 mL), and stirred for 10 min. The organic layer was separated and the aqueous layer was extracted with CH₂Cl₂ (2 x 10 mL). The combined organic layer was washed with brine (5 mL), dried (MgSO₄), concentrated, and purified by flash chromatography (silica, 25 → 50% ethylacetate in petroleum ether) to give 45 (6.0 mg, 15%) and 46 (30.0 mg, 88% based on 85% conversion) as an amorphous solid.

**Physical Data for Alcohol 46**. R_{f} = 0.30 (silica, 50% ethylacetate in petroleum ether); ¹H NMR (500 MHz, CDCl₃) δ 7.93 (d, *J* = 9.0 Hz, 2H, Ar), 6.64 (d, *J*= 9.0 Hz, 2H, Ar), 6.42 (s, 1H, 10-H), 5.57 (d, *J* = 7.0 Hz, 1H, 2-H), 4.94 (br d, *J* = 8.0 Hz, 1H, 5-H), 4.83-4.75 (m, 1H, 13-H), 4.46 (dd, *J*= 10.5, 6.5 Hz, 1H, 7-H), 4.34 (d, *J*= 8.5 Hz, 1H, 20-H), 4.13 (d, *J*= 8.5 Hz, 1H, 20-H), 3.82 (d, *J* = 7.0 Hz, 1H, 3-H), 3.04 (s, 6H, Me₂N), 2.54-2.44 (m, 1H, 6-H), 2.26 (s, 3H, Me), 2.23 (d,*J*= 7.5 Hz, 2H, 14-CH₂), 2.16 (s, 6H, Me, Me), 2.08 (d, *J*= 4.5 Hz, 1H, OH), 1.89-1.80 (m, 2H, 6-H, OH), 1.64 (s, 3H, Me), 1.16 (s, 3H, Me), 1.01 (s, 3H, Me), 0.89 (t, *J* = 8.5 Hz, 9H, OSi(CH₂CH₃)₃), 0.62-0.48 (band, 6H, OSi(CH₂CH₃)₃).

**DiTES taxoid 47**. To a solution of alcohol **46** (14.0 mg, 0.0188 mmol, previously azeotroped twice with benzene) and β-lactam 24 (25.0 mg, 0.0656 mmol, previously azeotroped twice with benzene) in THF (0.75 mL) at 0 °C, prepared from the Ojima-Holton protocol (Holton, R.A. *Chem Abstr.* **1990**, *114,* 164568q; Ojima, I.; Habus, I.; Zhao, M.; Georg, G. I.; Jayasinghe, L. R. *J. Org. Chem.* **1991**, *56,* 1681-1683; Ojima, I.; Habus, I.; Zhao, M.; Zucco, M.; Park, Y.H.; Sun, C. M.; Brigaud, T. *Tetrahedron* **1992**, *48,* 6985-7012), was added NaN(SiMe₃)₂ (0.056 mL of a 1.0 M solution in THF, 0.056 mmol) dropwise. The resulting solution was stirred for 20 min and poured into a mixture of CH₂Cl₂ (10 mL) and aqueous NH₄Cl (5 mL). The organic layer was separated and the aqueous layer was extracted with CH₂Cl₂ (2 x 5 mL). The combined organic layer was washed with brine (5 mL), dried (MgSO₄), concentrated, and purified by flash chromatography (silica, 10 → 15% ethylacetate in benzene, then 50% ethylacetate in petroleum ether) to give 47 (12.0 mg, 57%) as a white solid.

**Physical Data for DiTES taxoid 47**. R_{f} = 0.26 (silica, 15% ethylacetate in PhH); IR (film) νₘₐₓ 3425, 2946, 2882, 1722, 1669, 1600, 1365, 1275, 1238, 1179, 1094 cm⁻¹; ¹H NMR (500 MHz, CDCl₃) δ 7.96 (d, *J* = 9.0 Hz, 2H, Ar), 7.77-7.72 (m, 2H), 7.54-7.26 (band, 8H), 7.12 (d, *J*= 8.5 Hz, 1H, NH), 6.69 (d, *J* = 9.0 Hz, 2H), 6.43 (s, 1H, 10-H), 6.23 (br t, *J*= 9.0 Hz, 1H, 13-H), 5.68-5.63 (m, 2H, 2-H, 3'-H), 4.93 (br d, *J* = 8.0 Hz, 1H, 5-H), 4.67 (d, *J*= 2.0 Hz, 1H, 2'-H), 4.45 (dd, *J*= 10.5 Hz, 6.5 Hz, 1H, 7-H), 4.36 (d, *J*= 8.5 Hz, 1H, 20-H), 4.20 (d, *J*= 8.5 Hz, 1H, 20-H), 3.78 (d, *J*= 7.0 Hz, 1H, 3-H), 3.04 (s, 6H, Me₂N), 2.55-2.46 (m, 1H, 6-H), 2.53 (s, 3H, OAc), 2.36 (dd, *J* = 15.5, 9.0 Hz, 1H, 14-H), 2.15 (s, 3H, Me), 2.09 (dd, *J*= 15.5, 9.0 Hz, 1H, 14-H), 2.00 (d, *J* = 1.0 Hz, 3H, Me), 1.92-1.84 (m, 2H, 6-H, OH), 1.67 (s, 3H, Me), 1.20 (s, 3H, Me), 1.16 (s, 3H, Me), 0.90 (t, *J* = 8.0 Hz, 9H, OSi(CH₂CH₃)₃), 0.79 (t, *J*= 8.0 Hz, 9H, OSi(CH₂CH₃)₃), 0.63-0.35 (band, 12 H, OSi(CH₂CH₃)₃); FAB HRMS (NBA / CsI) *m/e* 1257.4503, M + Cs⁺ calcd for C₆₁H₈₄O₁₄N₂Si₂ 1257.4515.

**Taxoid 48**. A solution of silyl ether **47** (12.0 mg, 0.0107 mmol) in THF (1.0 mL) at 25 °C was treated with HF•pyridine (0.05 mL) and stirred for 1.5 h. The reaction mixture was poured into a mixture of ethylacetate (10 mL) and aqueous NaHCO₃ (5 mL) and the resulting mixture was stirred for 10 min. The organic layer was separated and the aqueous layer was extracted with ethylacetate (2 x 10 mL). The combined organic layer was washed with brine (5 mL), dried (MgSO₄), concentrated, and purified by flash chromatography (silica, 50 → 75% ethylacetate in petroleum ether) to give 48 (8.0 mg, 84%) as a colorless film.

**Physical Data for Taxoid 48.** R_{f} = 0.44 (silica, 75% ethylacetate in petroleum ether); IR (film) νₘₐₓ 3414, 2914, 2850, 1722, 1664, 1660, 1371, 1275, 1243, 1179 cm⁻¹; ¹H NMR (500 MHz, CDCl₃) δ 7.95 (d, *J*= 9.0 Hz, 2H), 7.77-7.72 (m, 2H), 7.55-7.30 (band, 8H), 7.03 (d, *J*= 9.0 Hz, 1H, NH), 6.67 (d, *J*= 9.0 Hz, 2H), 6.24 (s, 1H, 10-H), 6.20 (br t, *J*= 9.0 Hz, 1H, 13-H), 5.76 (dd, *J*= 9.0, 2.5 Hz, 1H, 3'-H), 5.62 (d, *J*= 7.0 Hz, 1H, 2-H), 4.93 (br d, *J*= 7.5 Hz, 1H, 5-H), 4.76 (dd, *J*= 5.0, 2.5 Hz, 1H, 2'-H), 4.37 (ddd, *J*= 11.5, 6.5, 4.0 Hz, 1H, 7-H), 4.34 (d, *J*= 8.5 Hz, 1H, 20-H), 4.18 (d, *J* = 8.5 Hz, 1H, 20-H), 3.73 (d, *J* = 7.0 Hz, 1H, 3-H), 3.57 (d, *J*= 5.0 Hz, 1H, 2'-OH), 3.04 (s, 6H, Me₂N), 2.58-2.48 (m, 1H, 6-H), 2.44 (d, *J* = 4.0 Hz, 1H, 7-OH), 2.37 (s, 3H, Me), 2.30-2.25 (m, 2H, 14-CH₂), 2.22 (s, 3H, Me), 1.95 (s, 1H, OH), 1.88-1.81 (m, 1H, 6-H), 1.74 (d, *J* = 1.0 Hz, 3H, Me), 1.65 (s, 3H, Me), 1.21 (s, 3H, Me), 1.11 (s, 3H, Me); FAB HRMS (NBA / CsI) *m/e* 1029.2760, M + Cs⁺ calcd for C₄₉H₅₆N₂O₁₄ 1029.2786.

### Preparation of 1-naphthalene-C-2-taxol (52)

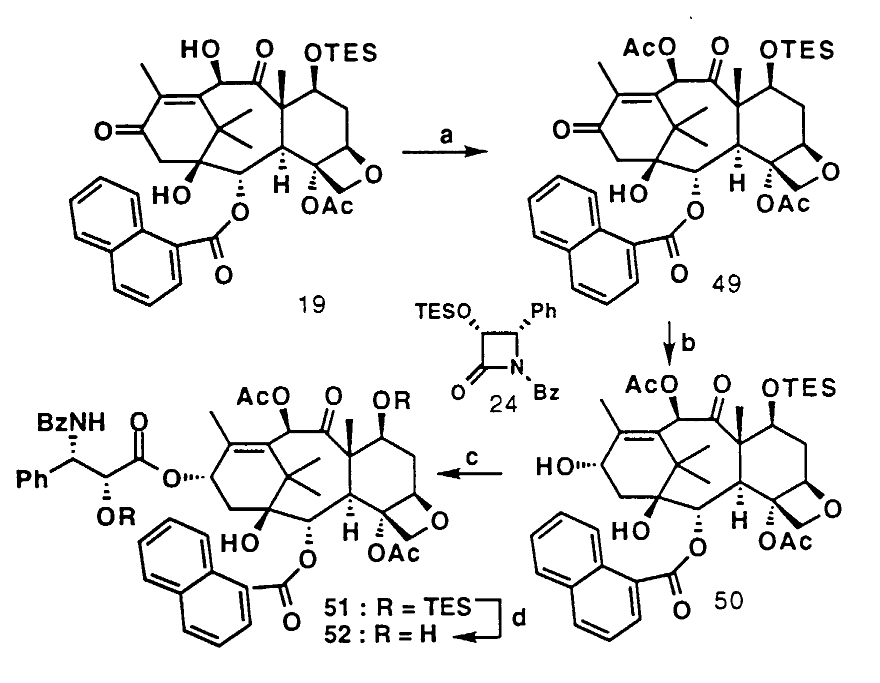

**Acetate 49.** A solution of previous alcohol **19** and 4-dimethylaminopyridine (DMAP, 100 mg, 0.819 mmol) in CH₂Cl₂ (3 mL) at 25 °C was treated with acetic anhydride (0.50 mL, 5.30 mmol) and stirred for 3 h. The reaction mixture was diluted with CH₂Cl₂ (5 mL), treated with aqueous NaHCO₃ (7 mL), and stirred vigorously for 25 min. The organic layer was separated and the aqueous layer was extracted with CH₂Cl₂ (2 x 10 mL). The combined organic layer was washed with brine (5 mL), dried (MgSO₄), concentrated, and purified by preparative TLC (silica, 10% ethylacetate in benzene) to give 49 (54.1 mg, 89% from carbonate **7**) as an amorphous solid.

**Physical Data for Acetate 49**. R_{f} = 0.27 (20% ethylacetate in petroleum ether); IR (film) νₘₐₓ 3416, 2953, 2879, 1726, 1676, 1370, 1224, 1089 cm⁻¹; ¹H NMR (500 MHz, CDCl₃) 8 8.66 (s, 1H, naphthalene), 8.06 (dd, 1H, *J*= 9.0, 2.0 Hz, naphthalene), 7.98-7.89 (m, 3H, naphthalene), 7.68-7.55 (m, 2H, naphthalene), 6.61 (s, 1H, 10-H), 5.75 (d, *J*= 7.0 Hz, 1H, 2-H), 4.95 (br d, *J* = 8.0 Hz, 1H, 5-H), 4.50 (dd, *J* = 10.5, 7.0 Hz, 1H, 7-H), 4.35 (d, *J*= 8.5 Hz, 1H, 20-H), 4.16 (d, *J*= 8.5 Hz, 1H, 20-H), 3.96 (d, *J*= 8.5 Hz, 1H, 20-H), 3.96 (d, *J*= 7.0 Hz, 1H, 3-H), 3.03 (d, *J* = 20.0 Hz, 1H, 14-H), 2.70 (d, *J*= 20.0 Hz, 1H, 14-H), 2.61-2.50 (m, 2H, 6-H, OH), 2.27 (s, 3H, Me), 2.24 (s, 3H, Me), 2.21 (s, 3H, Me), 1.91-1.83 (m, 1H, 6-H), 1.70 (s, 3H, Me), 1.30 (s, 3H, Me), 1.20 (s, 3H, Me), 0.93 (t, *J* = 8.0 Hz, 9H, OSi(CH₂CH₃)₃), 0.66-0.57 (band, 6H, OSi(CH₂CH₃)₃); FAB HRMS (NBA / CsI) m / e 881.2326, M + Cs⁺ calcd for C₄₁H₅₂O₁₁Si 881.2333.

**Alcohol 50**. A solution of enone **49** (54.1 mg, 0.0722 mmol) in MeOH (10 mL) at 25 °C was treated with NaBH₄ (54.5 mg, 1.44 mmol, added by portions) and stirred for 2.0 h. The reaction mixture was diluted with CH₂Cl₂ (10 mL), treated with aqueous NH₄Cl (5 mL), and stirred for 10 min. The organic layer was separated and the aqueous layer was extracted with CH₂Cl₂ (2 x 10 mL). The combined organic layer was washed with brine (5 mL), dried (MgSO₄), concentrated, and purified by preparative TLC (silica, 20% ethylacetate in petroleum ether) to give **50** (26 mg, 48%) as an amorphous solid.

**Physical Data for Alcohol 50**. R_{f} = 0.12 (20% ethylacetate in petroleum ether); IR (film) νₘₐₓ 3524, 2953, 1719, 1369, 1231, 1093, 829 cm⁻¹ ; ¹H NMR (500 MHz, CDCl₃) δ 8.70 (s, 1H, naphthalene), 8.11 (dd, *J* = 8.5, 1.5 Hz, 1H, naphthalene), 7.96-7.86 (m, 3H, naphthalene), 7.65-7.54 (m, 2H, naphthalene), 6.45 (s, 1H, 10-H), 5.68 (d, *J*= 7.0 Hz, 1H, 2-H), 4.98 (br d, *J* = 8.0 Hz, 1H, 5-H), 4.88-4.81 (m, 1H, 13-H), 4.51 (dd, *J*= 10.5, 7.0 Hz, 1H, 7-H), 4.34 (d, *J*= 8.5 Hz, 1H, 20-H), 4.19 (d, *J*= 8.5 Hz, 1H, 20-H), 3.93 (d, *J*= 7.0 Hz, 1H, 3-H), 2.58-2.50 (m, 1H, 6-H), 2.41-2.14 (m, 3H, 14-CH₂, 13-OH), 2.37 (s, 3H, Me), 2.21 (br s, 3H, Me), 2.19 (s, 3H, Me), 1.92-1.84 (m, 1H, 6-H), 1.72 (s, 1H, OH) 1.71 (s, 3H, Me), 1.22 (s, 3H, Me), 1.05 (s, 3H, Me), 0.93 (t, *J*= 8.0 Hz, 9H, OSi(CH₂CH₃)₃), 0.65-0.51 (band, 6H, OSi(CH₂CH₃)₃); FAB HRMS (NBA / CsI) *m/e* 883.2484, M + Cs⁺ calcd for C₄₁H₅₄O₁₁Si 883.2490.

**DiTES taxoid 5**1. To a solution of alcohol **50** (20.0 mg, 0.0266 mmol, previously azeotroped twice with benzene) and β-lactam 24 (20.0 mg, 0.0525 mmol, previously azeotroped twice with benzene) in THF (1.1 mL) at -78 °C, prepared from the Ojima-Holton protocol (Holton, R.A. *Chem Abstr.* **1990**, *114,* 164568q; Ojima, I.; Habus, I.; Zhao, M.; Georg, G. I.; Jayasinghe, L. *R. J. Org. Chem.* **1991**, *56*, 1681-1683; Ojima, I.; Habus, I.; Zhao, M.; Zucco, M.; Park, Y.H.; Sun, C. M.; Brigaud, T. *Tetrahedron* **1992**, *48*, 6985-7012), was added NaN(SiMe₃)₂ (0.065 mL of a 1.0 M solution in THF, 0.065mmol) dropwise. The resulting solution was stirred for 10 min and poured into a mixture of CH₂Cl₂ (10 mL) and aqueous NH₄Cl (5 mL). The organic layer was separated and the aqueous layer was extracted with CH₂Cl₂ (2 x 5 mL). The combined organic layer was washed with brine (5 mL), dried (MgSO₄), concentrated, and purified by preparative TLC (silica, 20% ethylacetate in petroleum ether) to give 51 (18.7 mg, 62%) as a white solid.

**Taxoid 52**. A solution of silyl ether **51** (18.7 mg, 0.0165 mmol) in THF (2 mL) at 25 °C was treated with HF•pyridine (1 mL) and stirred for 1 h. The reaction mixture was poured into a mixture of ethylacetate (10 mL) and aqueous NaHCO₃ (5 mL) and the resulting mixture was stirred for 10 min. The organic layer was separated and the aqueous layer was extracted with ethylacetate (2 x 10 mL). The combined organic layer was washed with brine (5 mL), dried (MgSO₄), concentrated, and purified by preparative TLC (silica, 50% ethylacetate in petroleum ether) to give **52** (12.8 mg, 86%) as a colorless film.

**Physical Data for Taxoid 52**. R_{f} = 0.16 (silica, 50% ethylacetate in petroleum ether); IR (film) νₘₐₓ 3420, 2967, 2896, 1721, 1652, 1519, 1370, 1233, 1073, 776 cm⁻¹; ¹H NMR (500 MHz, CDCl₃) δ 8.67 (s, 1H, naphthalene), 8.04 (dd, *J*= 8.5, 1.5 Hz, 1H, naphthalene), 7.95 (br d, *J* = 8.5 Hz, 1H, naphthalene), 7.87 (br d, *J* = 9.0 Hz, 1H), 7.81 (br d, *J* = 8.5 Hz, 1H), 7.65-7.61 (m, 2H), 7.56-7.51 (m, 1H), 7.49-7.22 (band, 9H), 6.94 (d, *J*= 9.0 Hz, 1H, NH), 6.23-6.16 (m, 2H, 10-H, 13-H), 5.78 (dd, *J*= 9.0, 2.0 Hz, 1H, 3'-H), 5.64 (br d, *J* = 7.0 Hz, 1H, 2-H), 4.87 (br d, *J* = 8.0 Hz, 1H, 5-H), 4.78-4.72 (m, 1H, 2'-H), 4.38-4.31 (m, 1H, 7-H), 4.24 (d, *J* = 8.5 Hz, 1H, 20-H), 4.16 (d, *J* = 8.5 Hz, 1H, 20-H), 3.76 (d, *J* = 7.0 Hz, 1H, 3-H), 3.53 (br s, 1H, OH), 2.52-2.43 (m, 1H, 6-H), 2.42 (d, *J*= 4.0 Hz, 1H, OH), 2.40 (s, 3H, Me), 2.36 (dd, *J* = 15.5, 9.0 Hz, 1H, 14-H), 2.25 (dd, *J* = 15.5, 9.0 Hz, 1H, 14-H), 2.17 (s, 3H, Me), 1.85-1.77 (m, 2H, 6-H, OH), 1.74 (br s, 3H, Me), 1.63 (s, 3H, Me), 1.17 (s, 3H, Me), 1.09 (s, 3H, Me); FAB HRMS (NBA / CsI) *m/e* 1036.2505, M + Cs⁺ calcd for C₅₁H₅₃NO₁₄ 1036.2520

### Preparation of thioether-C-2 taxol (56)

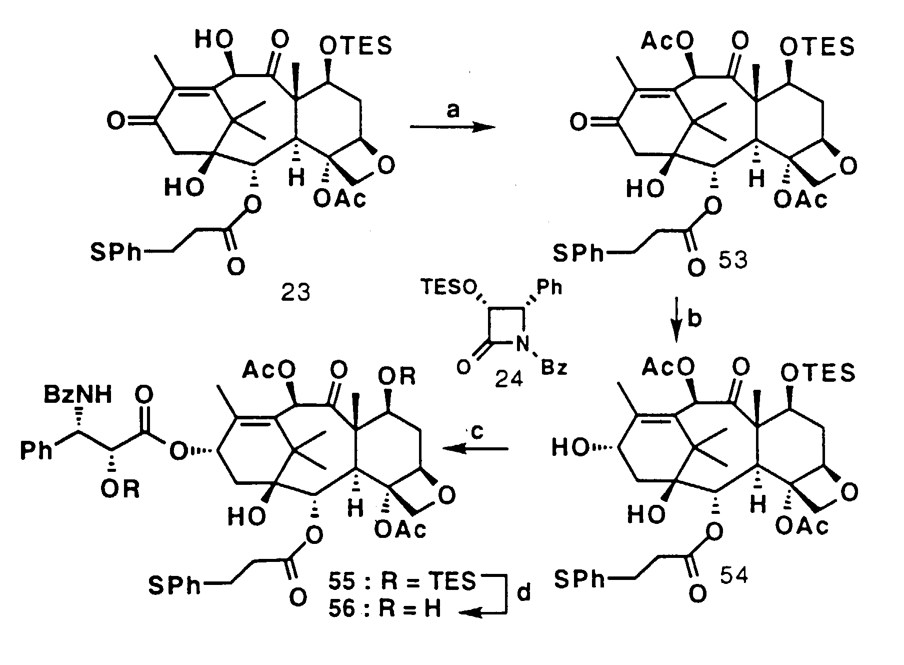

**Acetate 53**. A solution of alcohol **23** (25.2 mg, 0.0351 mmol) and 4-dimethylaminopyridine (DMAP, 12.2 mg, 0.0999 mmol) in CH₂Cl₂ (1.5 mL) at 25 °C was treated with acetic anhydride (0.120 mL, 1.27 mmol) and stirred for 1.5 h. The reaction mixture was diluted with CH₂Cl₂ (5 mL), treated with aqueous NaHCO₃ (7 mL), and stirred vigorously for 25 min. The organic layer was separated and the aqueous layer was extracted with CH₂Cl₂ (2 x 10 mL). The combined organic layer was washed with brine (5 mL), dried (MgSO₄), concentrated, and purified by flash chromatography (silica, 30% ethylacetate in petroleum ether) to give 53 (25.3 mg, 95%) as a colorless oil.

**Physical Data for Acetate 53**. R_{f} = 0.41 (silica, 10% ethylacetate in benzene, 2 elutions); IR (film) νₘₐₓ 3471, 2954, 2881, 1729, 1675, 1370, 1226, 986, 824, 738 cm⁻¹; ¹H NMR (500 MHz, CDCl₃) δ 7.38-7.25 (band, 5H, SPh), 6.54 (s, 1H, 10-H), 5.49 (br d, *J*= 6.5 Hz, 1H, 2-H), 4.90 (dd, *J*= 9.5, 2.0 Hz, 1H, 5-H), 4.42 (dd, *J* = 10.5, 6.5 Hz, 1H, 7-H), 4.37 (d, *J* = 8.0 Hz, 1H, 20-H), 4.17 (d, *J*= 8.0 Hz, 1H, 20-H), 3.78 (d, *J*= 6.5 Hz, 1H, 3-H), 3.23-3.13 (m, 2H, CH₂SPh), 2.78 (d, *J* = 20.0 Hz, 1H, 14-H), 2.72-2.58 (m, 3H, CH₂CH₂SPh, 14-H), 2.52 (ddd, *J* = 14.5, 9.5, 6.5, 1H, 6-H), 2.45 (s, 1H, OH), 2.21 (s, 3H, Me), 2.15 (s, 3H, Me), 2.04 (s, 3H, Me), 1.86 (ddd, *J*= 14.5, 10.5, 2.0 Hz, 1H, 6-H), 1.62 (s, 3H, Me), 1.23 (s, 3H, Me), 1.19 (s, 3H, Me), 0.91 (t, *J*= 8.0 Hz, 9H, OSi(CH₂CH₃)₃), 0.64-0 52 (band, 6H, OSi(CH₂CH₃)₃); FAB HRMS (NBA / CsI) *m/e* 891.2225, M + Cs⁺ calcd for C₃₉H₅₄O₁₁SSi 891.2210

**Alcohol 54.** A solution of enone **53** (24.4 mg, 0.032 mmol) in MeOH-THF (5 : 1, 1.9 mL) at 0 °C was treated with NaBH₄ (18.1 mg, 0.48 mmol, added by portions) and stirred for 1.25 h. The reaction mixture was diluted with CH₂Cl₂ (5 mL), treated with aqueous NH₄Cl (5 mL), and stirred for 10 min. The organic layer was separated and the aqueous layer was extracted with CH₂Cl₂ (2 x 5 mL). The combined organic layer was washed with brine (5 mL), dried (MgSO₄), concentrated, and purified by flash chromatography (silica, 30% ethylacetate in hexanes) to give 54 (14.6 mg, 60%) as an amorphous solid.

**Physical Data for Alcohol 54**. R_{f} = 0.11 (silica, 30% ethylacetate in hexanes); IR (film) νₘₐₓ 3487, 2938, 2880, 1729, 1586, 1369, 1234, 977, 738 cm⁻¹; ¹H NMR (500 MHz, CDCl₃) δ 7.40-7.23 (band, 5H, SPh), 6.42 (s, 1H, 10-H), 5.43 (d, *J* = 7.0 Hz, 1H, 2-H), 4.94 (dd, *J*= 9.5, 2.0 Hz, 1H, 5-H), 4.85-4.78 (m, 1H, 13-H), 4.43 (dd, *J*= 10.5, 6.5 Hz, 1H, 7-H), 4.37 (d, *J*= 8.0 Hz, 1H, 20-H), 4.18 (d, *J* = 8.0 Hz, 1H, 20-H), 3.74 (d, *J* = 7.0 Hz, 1H, 3-H), 3.25-3.15 (m, 2H, CH₂SPh), 2.71-2.57 (m, 2H, CH₂CH₂SPh), 2.51 (ddd, *J* = 14.5, 9.5, 6.5 Hz, 1H, 6-H), 2.25 (dd, *J* = 15.5, 9.5 Hz, 1H, 14-H), 2.16 (s, 3H, Me), 2.15 (d, *J*= 1.0 Hz, 3H, 18-Me), 2.15 (s, 3H, Me), 2.09 (dd, *J*= 15.5, 7.0 Hz, 1H, 14-H), 2.05 (br s, 1H, OH), 1.99-1.96 (m, 1H, OH), 1.86 (ddd, *J* = 14.5, 10.5, 2.0 Hz, 1H, 6-H), 1.63 (s, 3H, Me), 1.15 (s, 3H, Me), 1.04 (s, 3H, Me), 0.91 (t, *J*= 8.0 Hz, 9H, OSi(CH₂CH₃)₃), 0.64-0.50 (band, 6H, Si(CH₂CH₃)₃); FAB HRMS (NBA / CsI) *m/e* 893.2350, M + Cs⁺ calcd for C₃₉H₅₆O₁₁SSi 893.2367.

**DiTES taxoid 55**. To a solution of alcohol **54** (21.8 mg, 0.0286 mmol, previously azeotroped twice with benzene) and β-lactam **24** (33.0 mg, 0.0866 mmol, previously azeotroped twice with benzene) in THF (1.1 mL) at 0 °C, prepared from the Ojima-Holton protocol (Holton, R.A. *Chem Abstr.* **1990***, 114,* 164568q; Ojima, I.; Habus, I.; Zhao, M.; Georg, G. I.; Jayasinghe, L. R. *J. Org. Chem.* **1991**, *56,* 1681-1683; Ojima, I.; Habus, I.; Zhao, M.; Zucco, M.; Park, Y.H.; Sun, C. M.; Brigaud, T. *Tetrahedron* **1992***, 48,* 6985-7012), was added NaN(SiMe₃)₂ (0.086 mL of a 1.0 M solution in THF, 0.086 mmol) dropwise. The resulting solution was stirred for 20 min and poured into a mixture of CH₂Cl₂ (10 mL) and aqueous NH₄Cl (5 mL). The organic layer was separated and the aqueous layer was extracted with CH₂Cl₂ (2 x 5 mL). The combined organic layer was washed with brine (5 mL), dried (MgSO₄), concentrated, and purified by flash chromatography (silica, 15 → 30 → 50% ethylacetate in petroleum ether) to give 55 (13.8 mg, 42%) as an amorphous solid.

**Physical Data for DiTES taxoid 55**. R_{f} = 0.40 (silica, 30% ethylacetate in hexanes); IR (film) νₘₐₓ 3437, 2952, 2879, 1735, 1662, 1482, 1369, 1236, 1128, 981, 740 cm⁻¹; ¹H NMR (500 MHz, CDCl₃) δ 7.82-7.76 (m, 2H), 7.54-7.16 (band, 13H), 7.11 (d, *J*= 9.0 Hz, 1H, NH), 6.41 (s, 1H, 10-H), 6.18 ( br t, *J*= 9.0 Hz, 1H, 13-H), 5.62 (dd, *J*= 9.0, 2.0 Hz, 1H, 3'-H), 5.49 (d, *J*= 7.0 Hz, 1H, 2-H), 4.93 (dd, *J* = 9.5, 2.0 Hz, 1H, 5-H), 4.64 (d, *J* = 2.0 Hz, 1H,2'-H),4.42 (dd, *J*= 10.5,6.5 Hz, 1H, 7-H), 4.40 (d, *J* = 8.0 Hz, 1H, 20-H), 4.21 (d, *J* = 8.0 Hz, 1H, 20-H), 3.70 (d, *J* = 7.0 Hz, 1H, 3-H), 3.23-3.17 (m, 2H, CH₂SPh), 2.78-2.69 (m, 1H, HCHCH₂SPh), 2.67-2.57 (m, 1H, HCHCH₂SPh), 2.55-2.46 (m, 2H, 6-H, OH), 2.38 (s, 3H, Me), 2.27-2.10 (m, 2H, 14-CH₂), 2.16 (s, 3H, Me), 1.98 (d, *J*= 1.0 Hz, 3H, Me), 1.89 (ddd, *J*= 14.0, 11.0, 2.0 Hz, 1H, 6-H), 1.64 (s, 3H, Me), 1.18 (s, 3H, Me), 1.17 (s, 3H., Me), 0.91 (t, *J*= 8.0 Hz, 9H, OSi(CH₂CH₃)₃), 0.81 (t, *J*= 8.0 Hz, 9H, OSi(CH₂CH₃)₃), 0.64-0.36 (band, 12 H, OSi(CH₂CH₃)₃); FAB HRMS (NBA / CsI) *m/e* M + Cs⁺ 1274.4125 calcd for C₆₁H₈₃O₁₄SSi₂ 1274.4127.

**Taxoid 56**. A solution of silyl ether **55** (8.1 mg, 0.0071 mmol) in THF (0.5 mL) at 25 °C was treated with HF•pyridine (0.150 mL) and stirred for 3.75 h. The reaction mixture was poured into a mixture of ethylacetate (10 mL) and aqueous NaHCO₃ (5 mL) and the resulting mixture was stirred for 10 min. The organic layer was separated and the aqueous layer was extracted with ethylacetate (2 x 10 mL). The combined organic layer was washed with brine (5 mL), dried (MgSO₄), concentrated, and purified by preparative TLC (silica, 60% ethylacetate in petroleum ether) to give 56 (3.2 mg, 49%) as a colorless film.

**Physical Data for Taxoid 56.** R_{f} = 0.39 (silica, 60% ethylacetate in petroleum ether); IR (film) νₘₐₓ 3426, 2928, 1731, 1642, 1371, 1238, 1070, 739, 709 cm⁻¹; ¹H NMR (500 MHz, CDCl₃) δ 7.80-7.75 (m, 2H), 7.55-7.18 (band, 13 H), 6.94 (d, *J*= 9.0 Hz, 1H, NH), 6.23 (s, 1H, 10-H), 6.19 (br t, *J*= 9.0 Hz, 1H, 13-H), 5.74 (dd, *J*= 9.0, 2.5 Hz, 1H, 3'-H), 5.47 (d, *J*= 7.0 Hz, 1H, 2-H), 4.93 (dd, *J*= 9.5, 2.0 Hz, 1H, 5-H), 4.74 (dd, *J*= 5.0, 2.5 Hz, 1H, 2'-H), 4.38 (d, *J* = 8.0 Hz, 1H, 20-H), 4.35 (ddd, *J* = 11.0, 6.5 Hz, 4.5 Hz, 1H, 7-H), 4.21 (d, *J*= 8.0 Hz, 1H, 20-H), 3.67 (d, *J* = 7.0 Hz, 1H, 3-H), 3.51 (d, *J*= 5.0 Hz, 1H, 2'-OH), 3.28-3.14 (m, 2H, CH₂SPh), 2.77-2.68 (m, 1H, HCHCH₂SPh), 2.67-2.59 (m, 1H, HCHCH₂SPh), 2.54 (ddd, *J* = 14.5, 9.5, 6.5 Hz, 1H, 6-H), 2.44 (d, *J* = 4.5 Hz, 1H, 7-OH), 2.36 (dd, *J* = 15.5, 9.0 Hz, 1H, 14-H), 2.26 (br s, 1H, OH), 2.23 (s, 3H, Me), 2.21 (s, 3H, Me), 2.18 (dd, *J* = 15.5, 9.0 Hz, 1H, 14-H), 1.88 (ddd, *J* = 14.5, 11.0, 2.0 Hz, 1H, 6-H), 1.75 (d, *J* = 1.0 Hz, 3H, Me), 1.63 (s, 3H, Me), 1.24 (s, 3H, Me), 1.10 (s, 3H, Me); FAB HRMS (NBA / CsI) *m/e* 1046.2410, M + Cs⁺ calcd for C₄₉H₅₅O1₄NS 1046.2398.

### Preparation of MPA taxoid 57

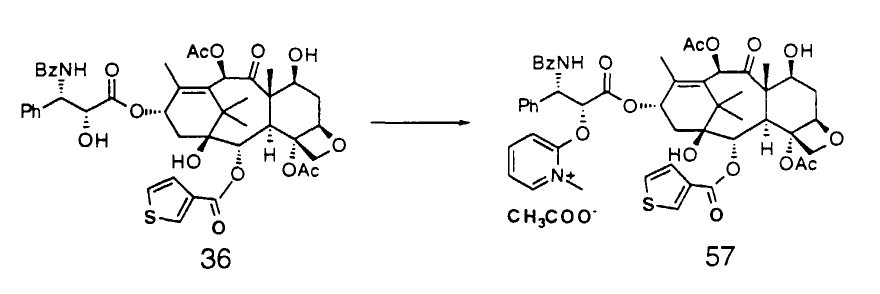

**MPA taxoid 57**. A solution of taxoid **36** (4.3 mg, 0.005 mmol) and triethylamine (0.0033 mL, 0.0237 mmol) in CH₂Cl₂ (0.2 mL) at 25 °C was treated with 2-fluoro-1-methylpyridinium p-toluenesulfonate (2.1 mg, 0.0075 mmol) and stirred for 35 min. The clear colorless solution rapidly turned to a clear pale yellow. The course of the reaction was monitored through thin layer chromatography (TLC)(E. Merck RP- 18 silica, 65 tetrahydrofuran: 35 water, UV/phosphomolybidic acid) and after thirty minutes of stirring at ambient temperature, judged complete as no taxol remained and only one compound was apparent by TLC. The reaction mixture was directly purified by HPLC (Vydak RP-18, 22.5 x 3 mm, A → B 0.5 h linear, A: 20% MeOH in 20 mM NH₄OAc, B: 100% MeOH, 9 mL / min, RT = 26.12) to give 36 (0.8 mg, 19%) and 57 (4.1 mg, 100% based on 81% conversion) as a colorless film.

**Physical Data for taxoid 57** ¹H NMR (500 MHz, CDCl₃) δ 10.5 (d, *J* = 7.5 Hz, 1H), 8.44 (ddd, *J* = 9.0, 7.5, 2.0 Hz, 1H), 8.33-8.29 (m, 2H), 8.15 (dd, *J* = 3.0, 1.0 Hz, 1H, thiophene), 8.12 (br d, *J*= 6.0 Hz, 1H), 7.84 (br d, *J*= 8.5 Hz, 1H), 7.74-7.69 (m, 2H), 7.53 (dd, *J*= 5.0, 1.0 Hz, 1H, thiophene), 7.48-7.34 (band, 7H), 7.16-7.12 (m, 1H), 6.53-6.43 (m, 1H, 2'-H), 6.21 (s, 1H, 10-H), 6.03 (dd, *J*= 10.5, 8.0 Hz, 1H, 3'-H), 5.82 (br t, *J*= 9.0 Hz, 1H, 13-H), 5.44 (d, *J*= 7.0 Hz, 1H, 2-H), 4.90 (dd, *J*= 9.5, 2.0 Hz, 1H, 5-H), 4.33 (dd, *J*= 11.0, 6.5 Hz, 1H, 7-H), 4.30 (d, *J*= 8.0 Hz, 1H, 20-H), 4.15 (d, *J*= 8.0 Hz, 1H, 20-H), 4.08 (s, 3H, N⁺Me), 3.68 (d, *J*= 7.0 Hz, 1H, 3-H), 2.58-2.49 (m, 1H, 6-H), 2.52 (s, 3H, OAc), 2.21 (s, 3H, OAc), 2.04 (s, 3H, OAc), 2.02 (br s, 2H, OH, OH), 1.88 (ddd, *J* = 14.5, 11.5, 2.0 Hz, 1H, 6-H), 1.78 (br s, 3H, 18-Me), 1.64 (s, 3H, Me), 1.61 (dd, *J*= 16.0, 7.0 Hz, 1H, 14-H), 1.18 (dd, *J* = 16.0, 9.0 Hz, 1H, 14-H), 1.13 (s, 3 H, Me), 1.08 (s, 3 H, Me).

### Preparation of MPA taxoid 58

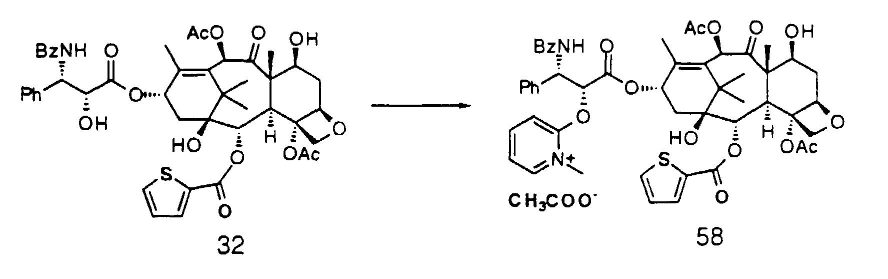

**MPA taxoid 58.** A solution of taxoid **32** (1.0 equiv.) and triethylamine (4.7 equiv.) in CH₂Cl₂ (0.025 M) at 25 °C is treated with 2-fluoro-1-methylpyridinium *p*-toluenesulfonate from Aldrich Chemical company inc. (1.5 equiv.) and stirred for 35 minutes. The course of the reaction was monitored through thin layer chromatography (TLC)(E. Merck RP- 18 silica, 65 tetrahydrofuran: 35 water, UV/phospho-molybidic acid) and after thirty minutes of stirring at ambient temperature, judged complete as no taxol remained and only one compound was apparent by TLC. The reaction mixture is then directly purified by HPLC (Vydak RP-18, 22.5 x 3 mm, A → B 0.5 h linear, A: 20% MeOH in 20 mM NH₄OAc, B: 100% MeOH, 9 mL / min, RT = 26.12) to give **58** as a colorless film.

### Preparation of MPA taxoid 59

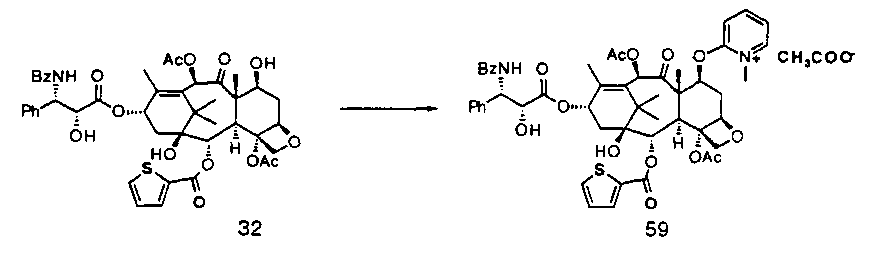

**MPA taxoid 59.** The synthesis of the taxoid-7-MPA **59** differs only slightly from the synthesis of taxoid-2'-MPA **58**. The C-2 taxoid **32** is dissolved in methylene chloride (.006 M) and treated sequentially with triethylamine (40 equivalents) and 2-fluoro-1-methyl-pyridinium tosylate (10 equivalents) Aldrich Chemicals, and allowed to stir at ambient temperature for 5 minutes. The reaction mixture is then directly purified by HPLC (Vydak RP-18, 22.5 x 3 mm, A → B 0.5 h linear, A: 20% MeOH in 20 mM NH₄OAc, B: 100% MeOH, 9 mL / min, RT = 26.12) to give **59** as a colorless film.

### Preparation of MPA taxoid 60

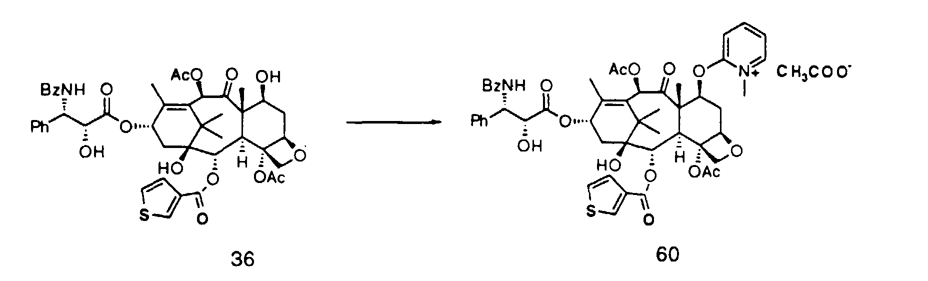

**MPA taxoid 60**. The synthesis of the taxoid-7-MPA **60** differs only slightly from the synthesis of taxoid-2'-MPA **57.** The C-2 taxoid **36** is dissolved in methylene chloride (.006 M) and treated sequentially with triethylamine (40 equivalents) and 2-fluoro-1-methyl-pyridinium tosylate (10 equivalents) Aldrich Chemicals, and allowed to stir at ambient temperature for 5 minutes. The reaction mixture is then directly purified by HPLC (Vydak RP-18, 22.5 x 3 mm, A → B 0.5 h linear, A: 20% MeOH in 20 mM NH₄OAc, B: 100% MeOH, 9 mL / min, RT = 26.12) to give 60 as a colorless film.

### Preparation of C-2-taxoid-2'-methyl-pyridinium salts

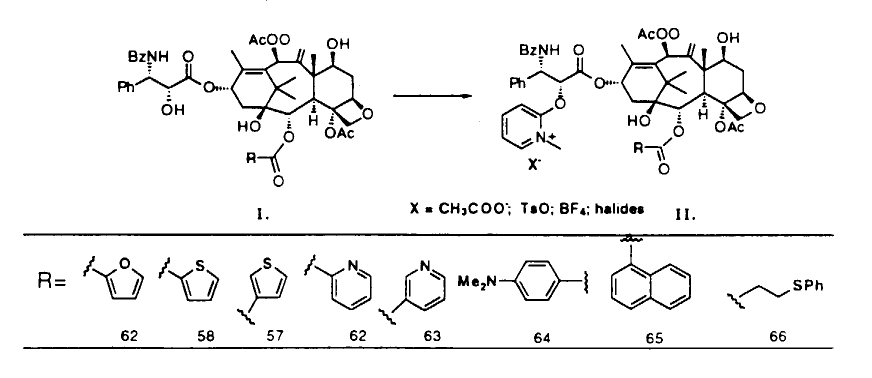

**C-2-taxoid-2'-onium salts 62-66.** A solution of taxoid (**62-66**) I. (1.0 equiv.) and triethylamine (4.7 equiv.) in CH₂Cl₂ (0.025 M) at 25 °C is treated with 2-fluoro-1-methylpyridinium p-toluenesulfonate from Aldrich Chemical company inc. (1.5 equiv.) and stirred for 35 minutes. The course of the reaction was monitored through thin layer chromatography (TLC)(E. Merck RP- 18 silica, 65 tetrahydrofuran: 35 water, UV/phospho-molybidic acid) and after thirty minutes of stirring at ambient temperature, judged complete as no taxol remained and only one compound was apparent by TLC. The reaction mixture is then directly purified by HPLC (Vydak RP-18, 22.5 x 3 mm, A → B 0.5 h linear, A: 20% MeOH in 20 mM NH₄OAc, B: 100% MeOH, 9 mL / min, RT = 26.12) to give (**62-66**) **II**. as a colorless film.

### Preparation of C-2-taxoid-7-methyl-pyridinium salts

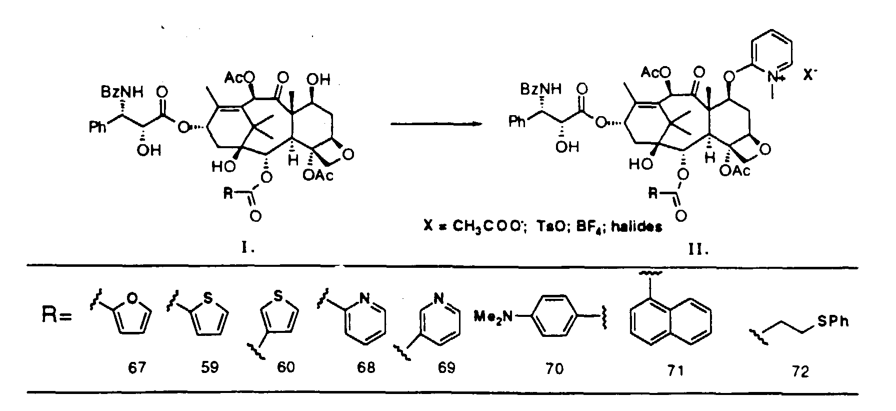

**C-2-taxoid-7-onium salts 67-72**. The synthesis of the taxoid-7-methyl-pyridinium salts (**67-72**) **II**, differs only slightly from the synthesis of taxoid-2'-methyl-pyridinium salts (**62-66**) **II**. The C-2 taxoid (**67-72**)**I** is dissolved in methylene chloride (.006 M) and treated sequentially with triethylamine (40 equivalents) and 2-fluoro-1-methylpyridinium tosylate (10 equivalents) Aldrich Chemicals, and allowed to stir at ambient temperature for 5 minutes. The reaction mixture is then directly purified by HPLC (Vydak RP-18, 22.5 x 3 mm, A → B 0.5 h linear, A: 20% MeOH in 20 mM NH40Ac, B: 100% MeOH, 9 mL / min, RT = 26.12) to give (67-72) II as a colorless film.

### Preparation of C-2-taxoid-bis-2',7-methyl-pyridinium salts

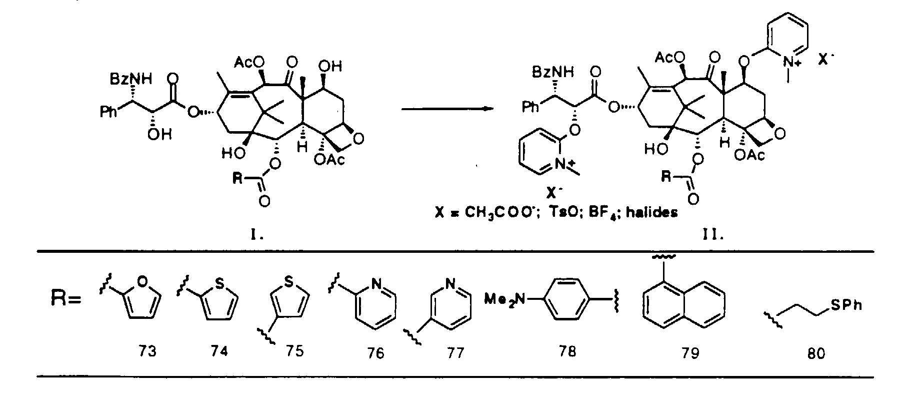

**C-2-taxoid-bis-2',7-onium salts 73-80.** The synthesis of C-2-taxoid-bis-2',7-methyl-pyridinium salts II (**73-80**), differs from the synthesis of taxoid-7-methylpyridinium salts (**67-72**) **II** only with respect to reaction time. The C-2 taxoid (**73-80**) **I** is dissolved in methylene chloride (.006 M) and treated sequentially with triethylamine (40 equivalents) and 2-fluoro-1-methyl-pyridinium tosylate (10 equivalents) Aldrich Chemicals, and allowed to stir at ambient temperature for 18 hours. The reaction mixture is then directly purified by HPLC (Vydak RP-18, 22.5 x 3 mm, A → B 0.5 h linear, A: 20% MeOH in 20 mM NH40Ac, B: 100% MeOH, 9 mL / min, RT = 26.12) to give (**73-80**) **II** as a colorless film.

### Preparation of C-2-taxoid-2'-benzothiazolium salts

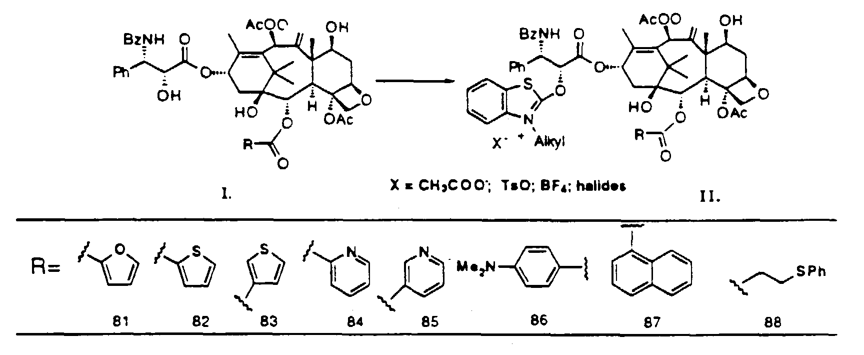

**C-2-taxoid-2'-benzothiazolium salts 81-88**. A solution of taxoid (**81-88**) **I** (1.0 equiv.) and triethylamine (4.7 equiv.) in CH₂Cl₂ (0.025 M) at 25 °C is treated with 2-fluoro-1methylpyridinium p-toluenesulfonate from Aldrich Chemical company inc. (1.5 equiv.) and stirred for 35 minutes. The course of the reaction was monitored through thin layer chromatography (TLC)(E. Merck RP- 18 silica, 65 tetrahydrofuran: 35 water, UV/phospho-molybidic acid) and after thirty minutes of stirring at ambient temperature, judged complete as no taxol remained and only one compound was apparent by TLC. The reaction mixture is directly purified by HPLC (Vydak RP-18, 22.5 x 3 mm, → B 0.5 h linear, A: 20% MeOH in 20 mM NH₄OAc, B: 100% MeOH, 9 mL / min, RT = 26.12) to give (**81-88**) **II**. as a colorless film.

### Preparation of C-2-taxoid-7-benzothiazolium salts

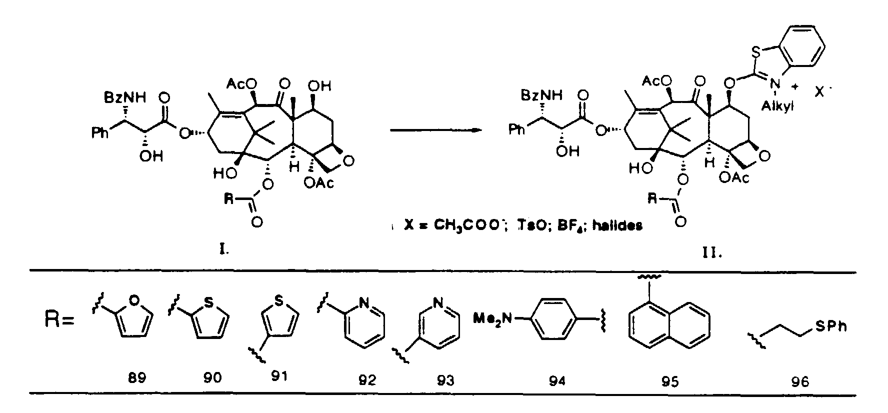

**C-2-taxoid-7-benzothiazolium salts (89-96)**. The synthesis of the taxoid-7-benzothiazolium salts **(89-96) II**, differs only slightly from the synthesis of taxoid-2'-benzothiazolium salts **(81-88) II**. The C-2 taxoid **(89-96 )I** is dissolved in methylene chloride (.006 M) and treated sequentially with triethylamine (40 equivalents) and 2-fluoro-3-ethylbenzothiazolium tetrafluoroborate (10 equivalents) Aldrich Chemicals, and allowed to stir at ambient temperature for 5 minutes. The reaction mixture is then directly purified by HPLC (Vydak RP-18, 22.5 x 3 mm, A → B 0.5 h linear, A: 20% MeOH in 20 mM NH₄OAc, B: 100% MeOH, 9 mL / min, RT = 26.12) to give **(89-96) II** as a colorless film.

### Preparation of C-2-taxoid-2'-benzoxazolium salts

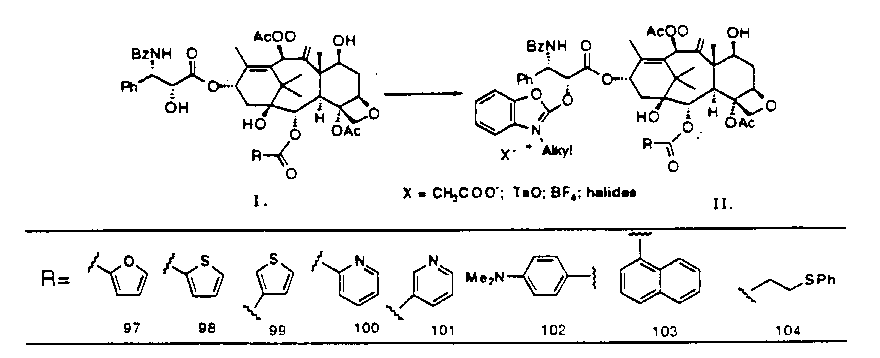

**C-2-taxoid-2'-benzoxazolium salts 97-104**. A solution of taxoid **97-1041 I**. (1.0 equiv.) and triethylamine (4.7 equiv.) in CH₂Cl₂ (0.025 M) at 25 °C is treated with 2-fluoro-1-methylpyridinium p-toluenesulfonate from Aldrich Chemical company inc. (1.5 equiv.) and stirred for 35 minutes. The course of the reaction was monitored through thin layer chromatography (TLC)(E. Merck RP- 18 silica, 65 tetrahydrofuran: 35 water, UV/phospho-molybidic acid) and after thirty minutes of stirring at ambient temperature, judged complete as no taxol remained and only one compound was apparent by TLC. The reaction mixture is then directly purified by HPLC (Vydak RP-18, 22.5 x 3 mm, A → B 0.5 h linear, A: 20% MeOH in 20 mM NH₄OAc, B: 100% MeOH, 9 mL / min, RT = 26.12) to give **97-104 II**. as a colorless film.

### Preparation of C-2-taxoid-7-benzoxazolium salts

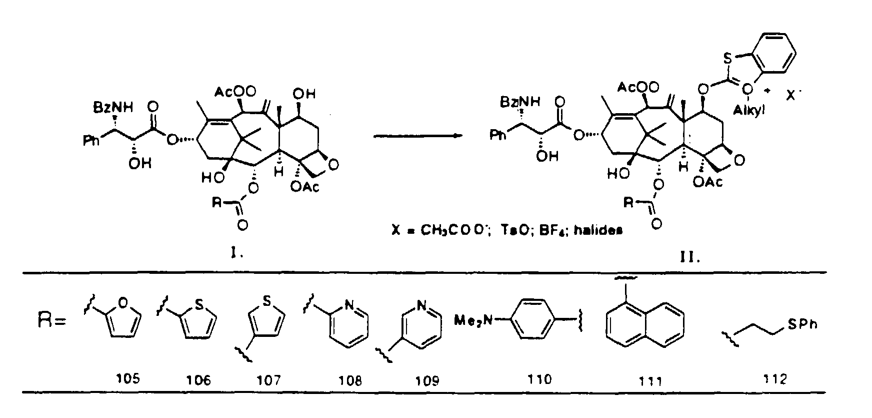

**C-2-taxoid-7-benzoxazolium salts (105-112)**. The synthesis of the taxoid-7-benzoxazolium salts **(105-112) II,** differs only slightly from the synthesis of taxoid-2'-benzoxazolium salts **(97-104) II**. The C-2 taxoid **(105-112) I** is dissolved in methylene chloride (.006 M) and treated sequentially with triethylamine (40 equivalents) and 2-chloro-3-ethylbenzoxazolium tetrafluoroborate from Aldrich Company (10 equivalents) Aldrich Chemicals, and allowed to stir at ambient temperature for 5 minutes. The reaction mixture is then directly purified by HPLC (Vydak RP-18, 22.5 x 3 mm, A → B 0.5 h linear, A: 20% MeOH in 20 mM NH₄OAc, B: 100% MeOH, 9 mL / min, RT = 26.12) to give **(105-112) II** as a colorless film.

### Preparation of C-2-taxoid-2'-pyrimidinium salts

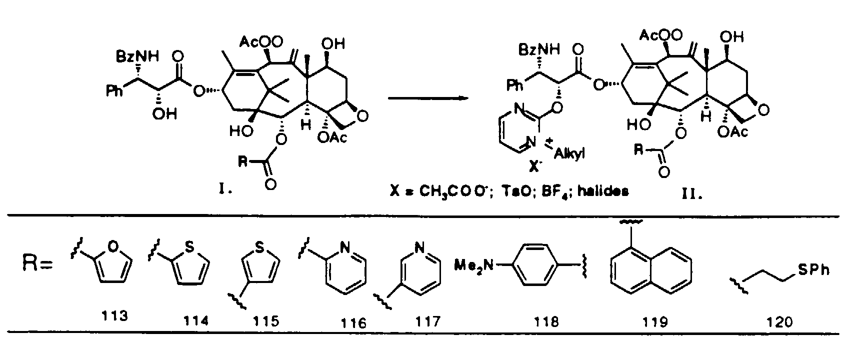

**C-2-taxoid-2'-pyrimidinium salts 113-120.** A solution of taxoid **(113-120) I**. (1.0 equiv.) and triethylamine (4.7 equiv.) in CH₂Cl₂ (0.025 M) at 25 °C is treated with 2-chloro-methyl-pyrimidinium fluoride from Aldrich Company (1.5 equiv.) and stirred for 35 minutes. The course of the reaction was monitored through thin layer chromatography (TLC)(E. Merck RP- 18 silica, 65 tetrahydrofuran: 35 water, UV/phospho-molybidic acid) and after thirty minutes of stirring at ambient temperature, judged complete as no taxol remained and only one compound was apparent by TLC. The reaction mixture is then directly purified by HPLC (Vydak RP-18, 22.5 x 3 mm, A → B 0.5 h linear, A: 20% MeOH in 20 mM NH₄OAc, B: 100% MeOH, 9 mL / min, RT = 26.12) to give (**113-120) II**. as a colorless film.

### Preparation of C-2-taxoid-7-pyrimidinium salts

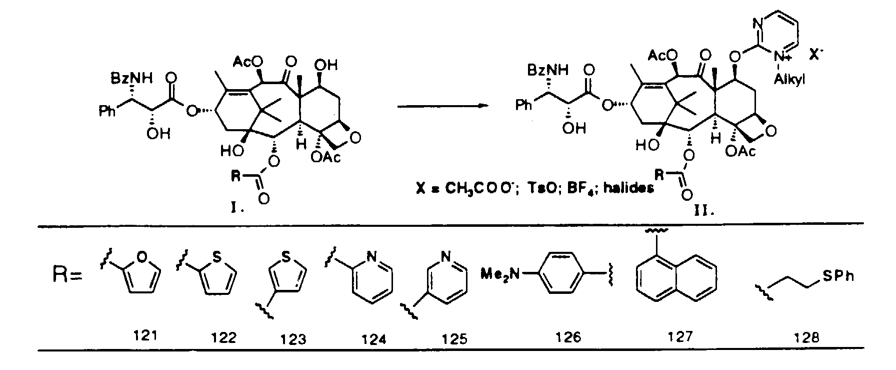

**C-2-taxoid-7-pyrimidinium salts (121-128)**. The synthesis of the taxoid-7-pyrimidinium salts **(121-128) II**, differs only slightly from the synthesis of taxoid-2'-pyrimidinium salts **(113-120) II**. The C-2 taxoid (121-128) I is dissolved in methylene chloride (.006 M) and treated sequentially with triethylamine (40 equivalents) and 2-chloro-methyl-pyrimidinium fluoride from Aldrich Company (10 equivalents), and allowed to stir at ambient temperature for 5 minutes. The reaction mixture is then directly purified by HPLC (Vydak RP-18, 22.5 x 3 mm, A → B 0.5 h linear, A: 20% MeOH in 20 mM NH₄OAc, B: 100% MeOH, 9 mL / min, RT = 26.12) to give **(121-128) II** as a colorless film.

## Claims

1. A taxo-diterpenoid represented by the following formula: 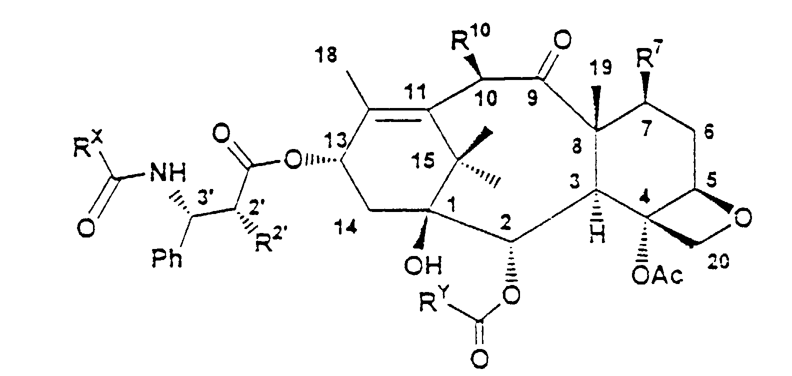 wherein:
R^{x} is selected from the group consisting of Ph and tBuO;
R¹⁰ is selected from the group consisting of OAc and OH;
R^{Y} is selected from the group consisting of phenyl and substituents represented by the following structures: 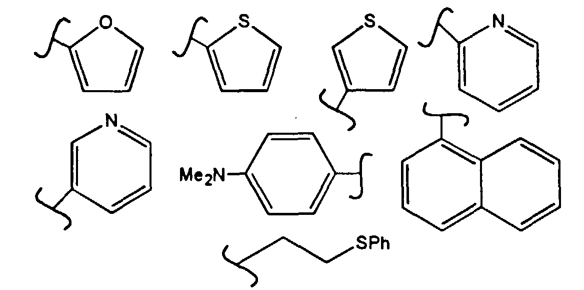 and
R^{2'} and R⁷ are each selected from the group consisting of OH and an onium salt of a 2-O-aza-arene, with the proviso that at least one of R^{2'} and R⁷ is said onium salt of the 2-O-aza-arene, said onium salt of the 2-O-aza-arene being selected from the group consisting of onium salt I and onium salt II represented by the following formulas: 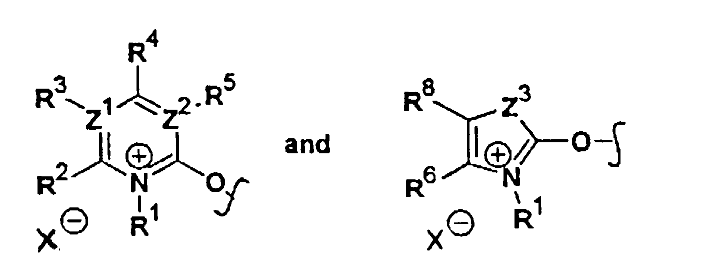
wherein:
Z¹ and Z² are each selected from the group consisting of C and N;
Z³ is selected from the group consisting of S and O;
R¹ is selected from the group consisting of C₁-C₆ alkyl and allyl;
R² is selected from the group consisting of H, C₁-C₆ alkyl and allyl;
R⁴ is selected from the group consisting of H, C₁-C₆ alkyl, allyl, C₁-C₄ O-alkyl, OH, and halogen;
if Z¹ is C, then R³ is selected from the group consisting of H, C₁-C₆ alkyl, allyl, C₁-C₆ O-alkyl, OH, and halogen;
if Z¹ is N, then R³ is absent;
R6 is selected from the group consisting of H, C₁-C₆ alkyl, and allyl, or together with R⁸ and the intervening vinylene group forms a fused aromatic six-membered ring system;
R⁸ is selected from the group consisting of H, C₁-C₆ alkyl, allyl, C₁-C₆ O-alkyl, OH, and halogen, or togther with R⁶ and the intervening vinylene group forms a fused aromatic six-membered ring system; and
if Z² is C, then R⁵ is selected from the group consisting of H, C₁-C₆ alkyl, allyl, C₁-C₆ O-alkyl, OH, and halogen;
if Z² is N, , then R⁵ is absent
X^{^{⊖}} is a counter ion.

2. A taxo-diterpenoid as described in claim 1 wherein:
X^{^{⊖}} is selected from the group consisting of counter ions consisting of Cl⁻, Br⁻, I⁻, CH₃COO⁻, BF₄⁻, ClO₄⁻, ArSO₃⁻, and C₁-C₆ AlkylSO₃⁻.

3. A taxo-diterpenoid as described in claim 1 wherein:
R^{2'} is said onium salt and
R⁷ is OH.

4. A taxo-diterpenoid as described in claim 1 wherein:
R^{2'} and R⁷ are both said onium salt.

5. A taxo-diterpenoid as described in claim 1 wherein:
R⁷ is said onium salt and
R^{2'} is OH.

6. A taxo-diterpenoid as described in any one of claims 3 to 5 wherein:
R^{x} is Ph;
R¹⁰ is AcO; and
said onium salt is onium salt I wherein:
Z¹ and Z² are both C;
R¹ is C₁-C₆ alkyl;
R², R³, R⁴, and R⁵ are each H; and
X^{^{⊖}} is selected from the group of counter ions consisting of Cl⁻, Br⁻, I⁻, CH₃COO⁻, BF₄⁻, ClO₄⁻, ArSO₃⁻, and C₁-C₆ AlkylSO₃⁻.

7. A taxo-diterpenoid as described in any one of claims 3 to 5 wherein:
R^{x} is *t*BuO;
R¹⁰ is OH; and
said onium salt is onium salt I wherein:
Z¹ and Z² are both C;
R¹ is C₁-C₆ alkyl;
R², R³, R⁴ and R⁵ are each H; and
X^{^{⊖}} is selected from the group of counter ions consisting of Cl⁻, Br⁻, I⁻, CH₃COO⁻, BF₄⁻, ClO₄⁻, ArSO₃⁻, and C₁-C₆ AlkylSO₃⁻.

8. A taxo-diterpenoid as described in any one of claims 6 and 7 wherein:
R¹ is methyl; and
X^{^{⊖}} is ArSO₃⁻ or CH₃COO⁻.

9. Taxo-diterpenoids as claimed in any one of claims 1 to 8 for use in therapy.

10. Use of taxo-diterpenoids as claimed in any one of claims 1 to 8 in the manufacture of a medicament for treating neoplasms.

## Patentansprüche

1. Taxo-Diterpenoid der folgenden Formel: 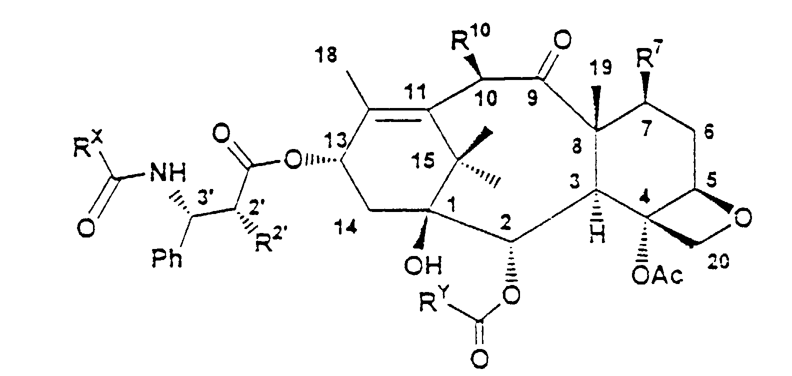 worin:
R^{x} aus der aus Ph und tBuO bestehenden Gruppe gewählt wird;
R¹⁰ aus der aus OAc und OH bestehenden Gruppe gewählt wird;
R⁷ aus der aus Phenyl und Substituenten der folgenden Strukturen bestehenden Gruppe gewählt wird: 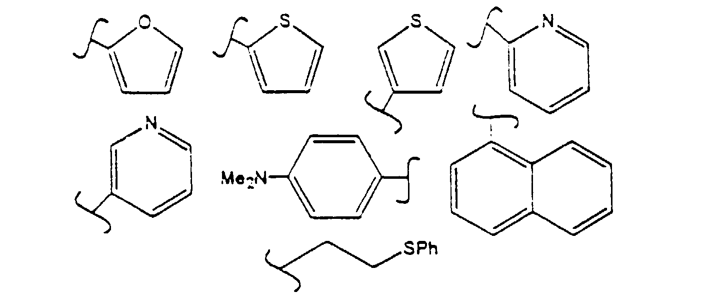 und
R^{2'} und R⁷ jeweils aus der Gruppe gewählt werden, die aus OH und einem Oniumsalz eines 2-O-Aza-Arens besteht, mit der Maßgabe, daß mindestens eines von R^{2'} und R⁷ das Oniumsalz des 2-O-Aza-Arens ist, wobei das Oniumsalz des 2-O-Aza-Arens aus der Gruppe gewählt wird, die aus Oniumsalz I und Oniumsalz II der folgenden Formeln besteht; 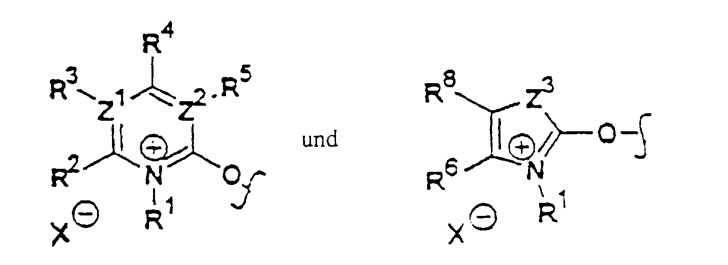
worin:
Z¹ und Z² jeweils aus der aus C und N bestehenden Gruppe gewählt werden:
Z³ aus der aus S und O bestehenden Gruppe gewählt wird;
R¹ aus der aus C₁-C₆-Alkyl und Allyl bestehenden Gruppe gewählt wird;
R^{2'} aus der aus H, C₁-C₆-Alkyl und Allyl bestehenden Gruppe gewählt wird;
R⁴ aus der aus H, C₁-C₆-Alkyl, Allyl, C₁-C₆-O-Alkyl, OH und Halogen bestehenden Gruppe gewählt wird;
wenn Z¹ C ist, dann R³ aus der aus H, C₁-C₆-Alkyl, Allyl, C₁-C₆-O-Alkyl, OH und Halogen bestehenden Gruppe gewählt wird;
wenn Z¹ N ist, dann R³ nicht vorhanden ist;
R⁶ gewählt wird aus der Gruppe, die besteht aus H, C₁-C₆-Alkyl und Allyl, oder zusammen mit R⁸ und der dazwischen liegenden Vinylengruppe ein verschmolzenes aromatisches sechsgliedriges Ringsystem bildet;
R⁸ aus der Gruppe gewählt wird, die aus H, C₁-C₆-Alkyl, Allyl, C₁-C₆-O-Alkyl, OH und Halogen besteht, oder zusammen mit R⁶ und der dazwischen liegenden Vinylengruppe ein verschmolzenes aromatisches sechsgliedriges Ringsystem bildet; und
wenn Z² C ist, dann R⁵ aus der Gruppe gewählt wird, die aus H, C₁-C₆-Alkyl, Allyl, C₁-C₆-O-Alkyl, OH und Halogen besteht;
wenn Z² N ist, dann R⁵ nicht vorhanden ist,
wobei X⁻ ein Gegenion ist.

2. Taxo-Diterpenoid, wie in Anspruch 1 beschrieben, und worin:
X⁻ aus der Gruppe gewählt wird, die aus Gegenionen besteht, bestehend aus Cl⁻, Br⁻, I⁻, CH₃COO⁻, BF₄⁻, ClO₄⁻, ArSO₃⁻ und C₁-C₆-Alkyl-SO₃⁻.

3. Taxo-Diterpenoid, wie in Anspruch 1 beschrieben, worin:
R^{2'} das Oniumsalz ist und
R⁷ OH ist.

4. Taxo-Diterpenoid, wie in Anspruch 1 beschrieben, worin:
R^{2'} und R⁷ beide das Oniumsaiz sind.

5. Taxo-Diterpenoid, wie in Anspruch 1 beschrieben, worin:
R⁷ das Oniumsalz ist und
R^{2'} OH ist.

6. Taxo-Diterpenoid, wie in mindestens einem der Ansprüche 3 bis 5 beschrieben, worin:
R^{x} Ph ist;
R¹⁰ AcO ist; und
das Oniumsalz das Oniumsalz I ist, worin:
Z¹ und Z² beide C sind;
R¹ C₁-C₆-Alkyl ist;
R², R³, R⁴ und R⁵ jeweils H sind; und
X⁻ aus der Gruppe gewählt ist, die aus Gegenionen besteht, bestehend aus Cl⁻, Br⁻, I⁻, CH₃COO⁻, BF₄⁻, ClO₄⁻, ArSO₃⁻ und C₁-C₆-Alkyl-SO₃⁻.

7. Taxo-Diterpenoid, wie nach mindestens einem der Ansprüche 3 bis 5 beschrieben, worin:
R^{x} tBuO ist;
R¹⁰ OH ist; und
das Oniumsalz das Oniumsalz I ist, worin:
Z¹ und Z² beide C sind;
R¹ C₁-C₆-Alkyl ist;
R², R³, R⁴ und R⁵ jeweils H sind; und
X⁻ aus der Gruppe von Gegenionen gewählt ist, bestehend aus Cl⁻, Br⁻, I⁻, CH₃COO⁻, BF₄⁻, ClO₄⁻, ArSO₃⁻ und C₁-C₆-Alkyl-SO₃⁻.

8. Taxo-Diterpenoid, wie in mindestens einem der Ansprüche 6 und 7 beschrieben. worin:
R¹ Methyl ist;
X⁻ ArSO₃⁻ oder CH₃COO⁻ ist.

9. Taxo-Diterpenoide, wie gemäß einem der Ansprüche 1 bis 8 beansprucht, zur Verwendung in der Therapie.

10. Verwendung von Taxo-Diterpenoiden, wie sie in mindestens einem der Ansprüche 1 bis 8 beansprucht sind, bei der Herstellung eines Medikamentes zur Behandlung von Neoplasmen.

## Revendications

1. Taxo-diterpènoïde représenté par la formule suivante : 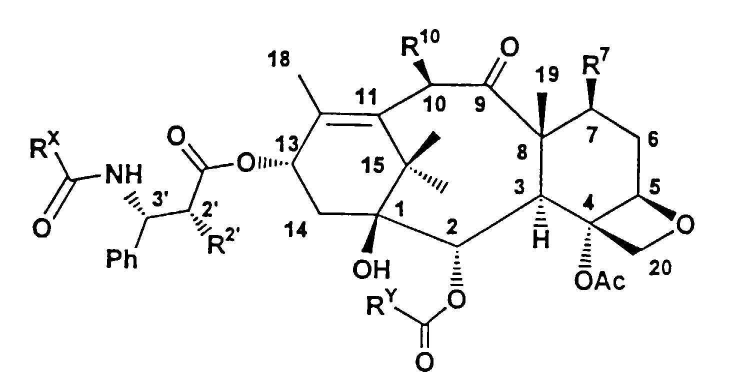 dans laquelle :
R^{x} est choisi dans le groupe consistant en Ph et tBuO ;
R¹⁰ est choisi dans le groupe consistant en OAc et OH ;
R^{y} est choisi dans le groupe consistant en phényle et les substituants représentés par les structures suivantes : 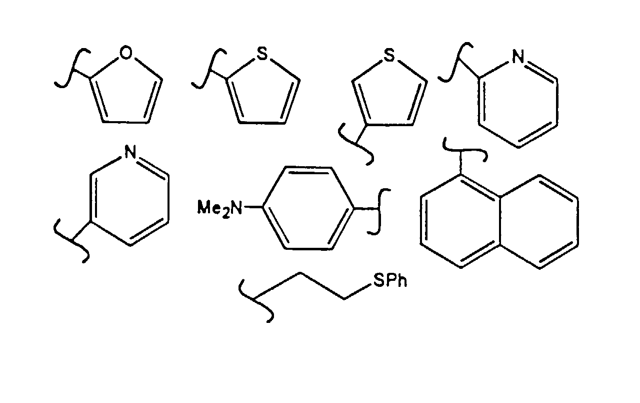 et
R^{2'} et R⁷ sont chacun choisis dans le groupe consistant en OH et un sel onium d'un 2-O-aza-arène, étant entendu que l'un au moins de R^{2'} et R⁷ est ledit sel onium dudit 2-O-aza-arène, ledit sel onium du 2-O-aza-arène étant choisi dans le groupe consistant en le sel onium I et le sel onium II représentés par les formules suivantes : 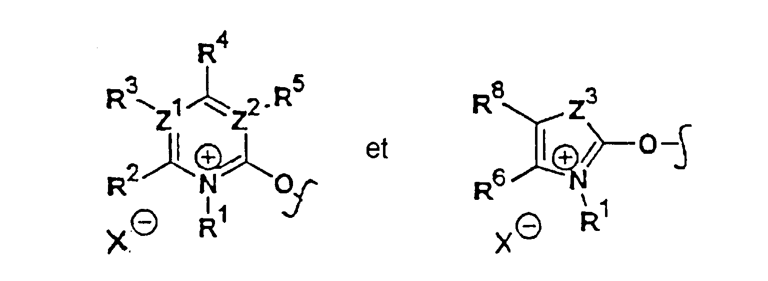
dans lesquelles :
Z¹ et Z² sont chacun choisis dans le groupe consistant en C et N ;
Z³ est choisi dans le groupe consistant en S et O ;
R¹ est choisi dans le groupe consistant en alkyle en C₁-C₆ et allyle ;
R^{2'} est choisi dans le groupe consistant en H, alkyle en C₁-C₆ et allyle ;
R⁴ est choisi dans le groupe consistant en H, alkyle en C₁-C₆, allyle, O-alkyle en C₁-C₆, OH et halogéno ;
si Z¹ représente C, dans ce cas R³ est choisi dans le groupe consistant en H, alkyle en C₁-C₆, allyle, O-alkyle en C₁-C₆, OH et halogéno ;
si Z¹ représente N, dans ce cas R³ est absent ;
R⁶ est choisi dans le groupe consistant en H, alkyle en C₁-C₆ et allyle, ou pris ensemble avec R⁸ et le groupe vinylidène intervenant, forment un système cyclique aromatique fusionné à six chaînons ;
R⁸ est choisi dans le groupe consistant en H, alkyle en C₁-C₆, allyle, O-alkyle en C₁-C₆, OH et halogéno, ou pris ensemble avec R⁶ et le groupe vinylidène intervenant, forment un système cyclique aromatique fusionné à six chaînons ; et
si Z² représente C, dans ce cas R⁵ est choisi dans le groupe consistant en H, alkyle en C₁-C₆, allyle, O-alkyle en C₁-C₆, OH et halogéno ;
si Z² représente N, dans ce cas R⁵ est absent et
X⁻ est un ion de charge complémentaire.

2. Taxo-diterpènoïde selon la revendication 1, dans lequel :
X⁻ est choisi dans le groupe consistant en ions de charge complémentaire consistant en Cl⁻, Br⁻, I⁻, CH₃COO⁻, BF₄⁻, ClO₄⁻, ArSO₃⁻, et (alkyle en C₁-C₆)SO₃⁻.

3. Taxo-diterpènoïde selon la revendication 1, dans lequel :
R^{2'} représente ledit sel onium et
R⁷ représente OH.

4. Taxo-diterpènoïde selon la revendication 1, dans lequel :
R^{2'} et R⁷ représentent tous deux ledit sel onium.

5. Taxo-diterpènoïde selon la revendication 1, dans lequel :
R⁷ représente ledit sel onium et
R^{2'} représente OH.

6. Taxo-diterpènoïde selon l'une quelconque des revendications 3 à 5, dans lequel :
R^{x} représente Ph ;
R¹⁰ représente AcO ; et
ledit sel onium est ledit sel onium I dans lequel :
Z¹ et Z² représentent tous deux C ;
R¹ représente alkyle en C₁-C₆ ;
R², R³, R⁴ et R⁵ représentent chacun H ; et
X⁻ est choisi dans le groupe des ions de charge complémentaire consistant en Cl⁻, Br⁻, I⁻, CH₃COO⁻, BF₄⁻, ClO₄⁻, ArSO₃⁻, et (alkyle en C₁-C₆)SO₃-.

7. Taxo-diterpènoïde selon l'une quelconque des revendications 3 à 5, dans lequel :
R^{x} représente tBuO ;
R¹⁰ représente OH ; et
ledit sel onium est le sel onium I dans lequel :
Z¹ et Z² représentent tous deux C ;
R¹ représente alkyle en C₁-C₆;
R², R³, R⁴ et R⁵ représentent chacun H ; et
X⁻ est choisi dans le groupe des ions de charge complémentaire consistant en Cl⁻, Br⁻, I⁻, CH₃COO⁻, BF₄⁻, ClO₄⁻, ArSO₃⁻, et (alkyle en C₁-C₆)SO₃⁻.

8. Taxo-diterpènoïde selon l'une quelconque des revendications 6 et 7, dans lequel :
R¹ représente méthyle ; et
X⁻ eprésente ArSO₃⁻ ou CH₃COO⁻.

9. Taxo-diterpènoïdes selon l'une quelconque des revendications 1 à 8 pour une utilisation en thérapeutique.

10. Utilisation des taxo-diterpènoïdes selon l'une quelconque des revendications 1 à 8 dans la fabrication d'un médicament pour traiter les néoplasmes.
